(19) Europäisches Patentamt / European Patent Office / Office européen des brevets

(11) **EP 1 519 956 B1**

(12) **EUROPEAN PATENT SPECIFICATION**

(45) Date of publication and mention of the grant of the patent: **21.09.2011 Bulletin 2011/38**

(21) Application number: **03750125.1**

(22) Date of filing: **12.05.2003**

(51) Int Cl.:
***C07K 16/28*** *(2006.01)* ***C07K 16/00*** *(2006.01)*
***A61K 39/395*** *(2006.01)* ***C12N 5/16*** *(2006.01)*

(86) International application number: **PCT/US2003/015044**

(87) International publication number: **WO 2003/094859 (20.11.2003 Gazette 2003/47)**

(54) **EPHA2 MONOCLONAL ANTIBODIES AND METHODS OF USE THEREOF**

EPHA2 MONOKLONALE ANTIKÖRPER UND DEREN ANWENDUNGSVERFAHREN

ANTICORPS MONOCLONAUX CONTRE EPHA2 ET LEURS METHODES D'UTILISATION

(84) Designated Contracting States:
**AT BE BG CH CY CZ DE DK EE ES FI FR GB GR HU IE IT LI LU MC NL PT RO SE SI SK TR**

(30) Priority: **10.05.2002 US 379322 P**
**14.10.2002 US 418213 P**
**03.04.2003 US 460507 P**

(43) Date of publication of application: **06.04.2005 Bulletin 2005/14**

(73) Proprietor: **MEDIMMUNE, INC.**
**Gaithersburg, MD 20878 (US)**

(72) Inventors:
- **KINCH, Michael, S.**
  **Laytonsville, MD 20882 (US)**
- **CARLES-KINCH, Kelly**
  **Laytonsville, MD 20882 (US)**
- **KIENER, Peter**
  **Potomac, MD 20854 (US)**
- **LANGERMANN, Solomon**
  **Baltimore, MD 21215 (US)**

(74) Representative: **Chapman, Paul Gilmour et al**
**Marks & Clerk LLP**
**Aurora**
**120 Bothwell Street**
**Glasgow G2 7JS (GB)**

(56) References cited:
**WO-A-01/12840** **WO-A-2004/014292**
**WO-A1-01/12172** **US-A1- 2001 031 252**

- **ZELINSKI D P ET AL: "EphA2 overexpression causes tumorigenesis of mammary epithelial cells." CANCER RESEARCH. 1 MAR 2001, vol. 61, no. 5, 1 March 2001 (2001-03-01), pages 2301-2306, XP001182247 ISSN: 0008-5472**
- **CARLES-KINCH K ET AL: 'Antibody Targeting of the EphA2 Tyrosine Kinase Inhibits Malignant Cell Behavior' CANCER RESEARCH vol. 62, no. 10, 15 May 2002, pages 2840 - 2847, XP002904266**
- **ZANTEK N D ET AL: 'E-cadherin regulates the functon of the EphA2 receptor tyrosine kinase' CELL GROWTH DIFFER vol. 10, no. 9, September 1999, pages 629 - 638, XP000978524**
- **LU M ET AL: 'EphA2 Overexpression Decreases Estrogen Dependence and Tamoxifen Sensitivity' CANCER RESEARCH vol. 63, 15 June 2003, pages 3425 - 3429, XP002904267**
- **WALKER-DANIELS J ET AL: 'c-Cbl-Dependent EphA2 Protein Degradation Is Induced by Ligand Binding' MOLECULAR CELLULAR RESEARCH vol. 1, November 2002, pages 79 - 87, XP002904268**
- **PRATT R L ET AL: 'Ligand binding up-regulates EphA2 mesenger RNA through the mitogen-activated protein/extracellular signal-regulated kinase pathway' MOLECULAR CELLULAR RESEARCH vol. 1, December 2003, pages 1070 - 1076, XP009032924**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 519 956 B1

SEQUENCE LISTING

<110> MedImmune, Inc.
Kinch, Michael
Carles-Kinch, Kelly
Kiener, Peter
Langermann, Solomon

<120> EphA2 Monoclonal Antibodies and Methods of Use Thereof

<130> 10271-097-228

<140>
<141>

<150> 60/379,322
<151> 2002-05-10

<150> 60/418,213
<151> 2002-10-14

<150> 60/418,213
<151> 2003-04-03

<160> 48

<170> PatentIn version 3.2

<210> 1
<211> 106
<212> PRT
<213> Homo sapiens

<400> 1

```
Gln Ile Val Leu Thr Gln Ser Pro Ala Leu Met Ser Ala Ser Pro Gly
1               5                   10                  15

Glu Lys Val Thr Met Thr Cys Ser Ala Ser Ser Ser Val Ser Tyr Met
            20                  25                  30

Tyr Trp Tyr Gln Gln Lys Pro Arg Ser Ser Pro Lys Pro Trp Ile Tyr
        35                  40                  45

Leu Thr Thr Asn Leu Ala Ser Gly Val Pro Ala Arg Phe Ser Gly Ser
    50                  55                  60

Gly Ser Gly Thr Ser Tyr Ser Leu Thr Ile Ser Ser Met Glu Ala Glu
65                  70                  75                  80

Asp Ala Ala Thr Tyr Tyr Cys Gln Gln Trp Ser Ser Asn Pro Phe Thr
                85                  90                  95

Phe Gly Ser Gly Thr Lys Leu Glu Ile Arg
            100                 105
```

<210> 2
<211> 10
<212> PRT
<213> Homo sapiens

<400> 2

```
Ser Ala Ser Ser Ser Val Ser Tyr Met Tyr
1               5                   10
```

<210> 3
<211> 7
<212> PRT
<213> Homo sapiens

<400> 3

Leu Thr Thr Asn Leu Ala Ser
1 5

<210> 4
<211> 9
<212> PRT
<213> Homo sapiens

<400> 4

Gln Gln Trp Ser Ser Asn Pro Phe Thr
1 5

<210> 5
<211> 118
<212> PRT
<213> Homo sapiens

<400> 5

Gln Val Gln Leu Gln Gln Pro Gly Ala Glu Leu Val Lys Pro Gly Ala
1 5 10 15

Ser Val Lys Leu Ser Cys Lys Ala Ser Gly Tyr Thr Phe Thr Ser Tyr
20 25 30

Trp Met His Trp Val Lys Gln Arg Pro Gly Gln Gly Leu Glu Trp Ile
35 40 45

Gly Met Ile His Pro Asn Ser Gly Ser Thr Asn Tyr Asn Glu Lys Phe
50 55 60

Lys Ser Lys Ala Thr Leu Thr Val Asp Lys Ser Ser Ser Thr Ala Tyr
65 70 75 80

Met Arg Leu Ser Ser Leu Thr Ser Glu Asp Ser Ala Val Tyr Tyr Cys
85 90 95

Ala Arg Gly Gly Asn Met Val Gly Gly Gly Tyr Trp Gly Gln Gly Thr
100 105 110

Thr Leu Thr Val Ser Ser
115

<210> 6
<211> 10
<212> PRT
<213> Homo sapiens

<400> 6

```
Gly Tyr Thr Phe Thr Ser Tyr Trp Met His
1               5                   10
```

<210> 7
<211> 17
<212> PRT
<213> Homo sapiens

<400> 7

```
Met Ile His Pro Asn Ser Gly Ser Thr Asn Tyr Asn Glu Lys Phe Lys
1               5                   10                  15
```

<210> 8
<211> 9
<212> PRT
<213> Homo sapiens

<400> 8

```
Gly Gly Asn Met Val Gly Gly Gly Tyr
1               5
```

<210> 9
<211> 318
<212> DNA
<213> Homo sapiens

<400> 9

```
caaattgttc tcacccagtc tccagcactc atgtctgcat ctccagggga gaaggtcacc     60
atgacctgca gtgccagctc aagtgtaagt tacatgtact ggtaccagca gaagccaaga    120
tcctccccca aaccctggat ttatctcaca accaacctgg cttctggagt ccctgctcgc    180
ttcagtggca gtgggtctgg gacctcttac tctctcacaa tcagcagcat ggaggctgaa    240
gatgctgcca cttattactg ccagcagtgg agtagtaacc cattcacgtt cggctcgggg    300
acaaagttgg aaataaga                                                  318
```

<210> 10
<211> 27
<212> DNA
<213> Homo sapiens

<400> 10
gccagctcaa gtgtaagtta catgtac 27

<210> 11
<211> 21
<212> DNA
<213> Homo sapiens

<400> 11
ctcacaacca acctggcttc t 21

<210> 12
<211> 27
<212> DNA
<213> Homo sapiens

<400> 12
cagcagtgga gtagtaaccc attcacg 27

<210> 13
<211> 354
<212> DNA
<213> Homo sapiens

<400> 13

```
caggtccaac tgcagcagcc tggggctgag ctggtaaagc ctggggcttc agtgaagttg      60
tcctgcaagg cttctggcta cactttcacc agctactgga tgcactgggt gaaacaaagg     120
cctggacaag gccttgagtg gattgggatg attcatccta atagtggtag tactaactac     180
aatgagaagt tcaagagcaa ggccacactg actgtagaca aatcctccag cacagcctac     240
atgcgactca gcagcctgac atctgaggac tctgcggtct attactgtgc aagagggggt     300
aacatggtag gggggggcta ctggggccaa ggcaccactc tcacagtctc ctca           354
```

<210> 14
<211> 30
<212> DNA
<213> Homo sapiens

<400> 14
ggctacactt tcaccagcta ctggatgcac 30

<210> 15
<211> 51
<212> DNA
<213> Homo sapiens

<400> 15

atgattcatc ctaatagtgg tagtactaac tacaatgaga agttcaagag c 51

<210> 16
<211> 30
<212> DNA
<213> Homo sapiens

<400> 16
agagggggta acatggtagg ggggggctac 30

<210> 17
<211> 107
<212> PRT
<213> Homo sapiens

<400> 17

```
Asp Ile Val Leu Thr Gln Ser Pro Ala Thr Leu Ser Val Thr Pro Gly
1               5                   10                  15

Asp Ser Val Asn Leu Ser Cys Arg Ala Ser Gln Ser Ile Ser Asn Asn
            20                  25                  30

Leu His Trp Tyr Gln Gln Lys Ser His Glu Ser Pro Arg Leu Leu Ile
        35                  40                  45

Lys Tyr Val Phe Gln Ser Ile Ser Gly Ile Pro Ser Arg Phe Ser Gly
    50                  55                  60

Ser Gly Ser Gly Thr Asp Phe Thr Leu Ser Ile Asn Ser Val Glu Thr
65                  70                  75                  80

Glu Asp Phe Gly Met Tyr Phe Cys Gln Gln Ser Asn Ser Trp Pro Leu
                85                  90                  95

Thr Phe Gly Ala Gly Thr Lys Leu Glu Leu Lys
            100                 105
```

<210> 18
<211> 11
<212> PRT
<213> Homo sapiens

<400> 18

```
Arg Ala Ser Gln Ser Ile Ser Asn Asn Leu His
1               5                   10
```

<210> 19

<211> 7
<212> PRT
<213> Homo sapiens

<400> 19

```
Tyr Val Phe Gln Ser Ile Ser
1               5
```

<210> 20
<211> 9
<212> PRT
<213> Homo sapiens

<400> 20

```
Gln Gln Ser Asn Ser Trp Pro Leu Thr
1               5
```

<210> 21
<211> 120
<212> PRT
<213> Homo sapiens

<400> 21

```
Glu Val Lys Leu Val Glu Ser Gly Gly Gly Leu Val Gln Pro Gly Gly
1               5                   10                  15

Ser Leu Ser Leu Ser Cys Ala Ala Ser Gly Phe Thr Phe Thr Asp Tyr
            20                  25                  30

Ser Met Asn Trp Val Arg Gln Pro Pro Gly Lys Ala Leu Glu Trp Leu
        35                  40                  45

Gly Phe Ile Arg Asn Lys Ala Asn Asp Tyr Thr Thr Glu Tyr Ser Ala
    50                  55                  60

Ser Val Lys Gly Arg Phe Thr Ile Ser Arg Asp Asn Ser Gln Ser Ile
65                  70                  75                  80

Leu Tyr Leu Gln Met Asn Ala Leu Arg Ala Glu Asp Ser Ala Thr Tyr
                85                  90                  95

Tyr Cys Val Arg Tyr Pro Arg Tyr His Ala Met Asp Ser Trp Gly Gln
            100                 105                 110
```

Gly Thr Ser Val Thr Val Ser Ser
115 120

<210> 22
<211> 10
<212> PRT
<213> Homo sapiens

<400> 22

Gly Phe Thr Phe Thr Asp Tyr Ser Met Asn
1 5 10

<210> 23
<211> 19
<212> PRT
<213> Homo sapiens

<400> 23

Phe Ile Arg Asn Lys Ala Asn Asp Tyr Thr Thr Glu Tyr Ser Ala Ser
1 5 10 15

Val Lys Gly

<210> 24
<211> 9
<212> PRT
<213> Homo sapiens

<400> 24

Tyr Pro Arg Tyr His Ala Met Asp Ser
1 5

<210> 25
<211> 321
<212> DNA
<213> Homo sapiens

<400> 25

gatattgtgc taactcagtc tccagccacc ctgtctgtga ctccaggaga tagcgtcaat 60
ctttcctgca gggccagcca aagtattagc aacaacctac actggtatca acaaaaatca 120
catgagtctc caaggcttct catcaagtat gttttccagt ccatctctgg gatcccctcc 180
aggttcagtg gcagtggatc agggacagat ttcactctca gtatcaacag tgtggagact 240
gaagattttg gaatgtattt ctgtcaacag agtaacagct ggccgctcac gttcggtgct 300
gggaccaagc tggagctgaa a 321

<210> 26
<211> 27
<212> DNA
<213> Homo sapiens

<400> 26
agccaaagta ttagcaacaa cctacac 27

<210> 27
<211> 21
<212> DNA
<213> Homo sapiens

<400> 27
tatgttttcc agtccatctc t 21

<210> 28
<211> 27
<212> DNA
<213> Homo sapiens

<400> 28
caacagagta acagctggcc gctcacg 27

<210> 29
<211> 360
<212> DNA
<213> Homo sapiens

<400> 29

gaggtgaagc tggtggagtc tggaggaggc ttggtacagc ctggggggttc tctgagtctc 60
tcctgtgcag cttctggatt caccttcact gattactcca tgaactgggt ccgccagcct 120
ccagggaagg cacttgagtg gttgggtttt attagaaaca aagctaatga ttacacaaca 180
gagtacagtg catctgtgaa gggtcggttc accatctcca gagataattc ccaaagcatc 240
ctctatcttc aaatgaatgc cctgagagct gaggacagtg ccacttatta ctgtgtaaga 300
taccctaggt atcatgctat ggactcctgg ggtcaaggaa cctcagtcac cgtctcctca 360

<210> 30
<211> 30
<212> DNA
<213> Homo sapiens

<400> 30
ggattcacct tcactgatta ctccatgaac 30

<210> 31
<211> 57
<212> DNA
<213> Homo sapiens

<400> 31
tttattagaa acaaagctaa tgattacaca acagagtaca gtgcatctgt gaagggt 57

<210> 32
<211> 27
<212> DNA
<213> Homo sapiens

<400> 32
taccctaggt atcatgctat ggactcc 27

<210> 33
<211> 107
<212> PRT
<213> Homo Sapiens

<400> 33

```
Asp Ile Lys Met Thr Gln Ser Pro Ser Ser Met Tyr Ala Ser Leu Gly
1               5                   10                  15

Glu Arg Val Thr Ile Thr Cys Lys Ala Ser Gln Asp Ile Asn Asn Tyr
            20                  25                  30

Leu Ser Trp Phe Gln Gln Lys Pro Gly Lys Ser Pro Lys Thr Leu Ile
        35                  40                  45

Tyr Arg Ala Asn Arg Leu Val Asp Gly Val Pro Ser Arg Phe Ser Gly
    50                  55                  60

Ser Gly Ser Gly Gln Asp Tyr Ser Leu Thr Ile Ser Ser Leu Glu Tyr
65                  70                  75                  80

Glu Asp Met Gly Ile Tyr Tyr Cys Leu Lys Tyr Asp Glu Phe Pro Tyr
                85                  90                  95

Thr Phe Gly Gly Gly Thr Lys Leu Glu Ile Lys
            100                 105
```

<210> 34
<211> 11
<212> PRT
<213> Homo Sapiens

<400> 34

```
Lys Ala Ser Gln Asp Ile Asn Asn Tyr Leu Ser
1               5                   10
```

<210> 35
<211> 7
<212> PRT
<213> Homo Sapiens

<400> 35

```
Arg Ala Asn Arg Leu Val Asp
1               5
```

<210> 36
<211> 9
<212> PRT
<213> Homo Sapiens

<400> 36

```
Leu Lys Tyr Asp Glu Phe Pro Tyr Thr
1               5
```

<210> 37
<211> 115
<212> PRT
<213> Homo Sapiens

<400> 37

```
Asp Val Lys Leu Val Glu Ser Gly Gly Gly Leu Val Lys Pro Gly Gly
1               5                   10                  15

Ser Leu Lys Leu Ser Cys Ala Ala Ser Gly Phe Thr Phe Ser Ser Tyr
            20                  25                  30

Thr Met Ser Trp Val Arg Gln Thr Pro Glu Lys Arg Leu Glu Trp Val
        35                  40                  45

Ala Thr Ile Ser Ser Gly Gly Thr Tyr Thr Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Thr Arg Glu Ala Ile Phe Thr Tyr Trp Gly Gln Gly Thr Leu Val Thr
            100                 105                 110

Val Ser Ala
        115
```

<210> 38
<211> 10
<212> PRT
<213> Homo Sapiens

<400> 38

```
Gly Phe Thr Phe Ser Ser Tyr Thr Met Ser
1               5                   10
```

<210> 39
<211> 17
<212> PRT
<213> Homo Sapiens

<400> 39

```
Thr Ile Ser Ser Gly Gly Thr Tyr Thr Tyr Tyr Pro Asp Ser Val Lys
1               5                   10                  15

Gly
```

<210> 40

<211> 6
<212> PRT
<213> Homo Sapiens

<400> 40

```
Glu Ala Ile Phe Thr Tyr
1               5
```

<210> 41
<211> 321
<212> DNA
<213> Homo sapiens

<400> 41

```
gacatcaaga tgacccagtc tccatcttcc atgtatgcat ctctaggaga gagagtcact      60
atcacttgca aggcgagtca ggacattaat aactatttaa gctggttcca gcagaaacca     120
gggaaatctc ctaagaccct gatctatcgt gcaaacagat tggtagatgg ggtcccatca     180
aggttcagtg gcagtggatc tgggcaagat tattctctca ccatcagcag cctggagtat     240
gaagatatgg gaatttatta ttgtctgaaa tatgatgagt ttccgtacac gttcggaggg     300
gggaccaagc tggaaataaa a                                               321
```

<210> 42
<211> 30
<212> DNA
<213> Homo sapiens

<400> 42
gcgagtcagg acattaataa ctatttaagc 30

<210> 43
<211> 21
<212> DNA
<213> Homo sapiens

<400> 43
cgtgcaaaca gattggtaga t 21

<210> 44
<211> 21
<212> DNA
<213> Homo sapiens

<400> 44
aaatatgatg agtttccgta c 21

<210> 45
<211> 345
<212> DNA

<213> Homo sapiens

<400> 45

```
gacgtgaagc tggtggagtc tgggggaggc ttagtgaagc ctggagggtc cctgaaactc     60
tcctgtgcag cctctggatt cactttcagt agctatacca tgtcttgggt tcgccagact    120
ccggagaaga ggctggagtg ggtcgcaacc attagtagtg gtggtactta cacctactat    180
ccagacagtg tgaagggccg attcaccatc tccagagaca atgccaagaa caccctgtac    240
ctgcaaatga gcagtctgaa gtctgaggac acagccatgt attactgtac aagagaagct    300
atctttactt actggggcca agggactctg gtcactgtct ctgca                    345
```

<210> 46
<211> 30
<212> DNA
<213> Homo sapiens

<400> 46
ggattcactt tcagtagcta taccatgtct 30

<210> 47
<211> 51
<212> DNA
<213> Homo sapiens

<400> 47
accattagta gtggtggtac ttacacctac tatccagaca gtgtgaaggg c 51

<210> 48
<211> 21
<212> DNA
<213> Homo sapiens

<400> 48
agagaagcta tctttactta c 21

## Description

### 1. FIELD OF THE INVENTION

This application claims priority to U.S. Provisional Application Serial No. 60/379,322, filed May 10, 2002, U.S. Provisional Application Serial No. 60/418,213, filed October 14, 2002, and U.S. Provisional Application Serial No. 60/460,507, filed April 3, 2003.

The present invention relates to methods and compositions designed for the treatment, management, or prevention of hyperproliferative cell disease, particularly, cancer. The methods of the invention comprise the administration of an effective amount of one or more antibodies specific for EphA2, preferably monoclonal antibodies, that are EphA2 agonists, inhibit a cancer cell phenotype (such as colony formation in soft agar or tubular network formation in a three dimensional basement membrane or extracellular membrane preparation, such as MATRIGEL™), preferentially bind epitopes on EphA2 that are selectively exposed or increased on cancer cells relative to non-cancer cells and/or bind EphA2 with a $K_{off}$ of less than 3 X $10^{-3}$ $s^{-1}$. The invention also provides pharmaceutical compositions comprising one or more monoclonal antibodies of the invention either alone or in combination with one or more other agents useful for cancer therapy. Diagnostic methods and methods for screening for therapeutically useful EphA2 specific antibodies are also provided.

### 2. BACKGROUND OF THE INVENTION

#### Cancer

A neoplasm, or tumor, is a neoplastic mass resulting from abnormal uncontrolled cell growth which can be benign or malignant. Benign tumors generally remain localized. Malignant tumors are collectively termed cancers. The term "malignant" generally means that the tumor can invade and destroy neighboring body structures and spread to distant sites to cause death (for review, see Robbins and Angell, 1976, Basic Pathology, 2d Ed., W.B. Saunders Co., Philadelphia, pp. 68-122). Cancer can arise in many sites of the body and behave differently depending upon its origin. Cancerous cells destroy the part of the body in which they originate and then spread to other part(s) of the body where they start new growth and cause more destruction.

More than 1.2 million Americans develop cancer each year. Cancer is the second leading case of death in the United States and, if current trends continue, cancer is expected to be the leading cause of death by the year 2010. Lung and prostate cancer are the top cancer killers for men in the United States. Lung and breast cancer are the top cancer killers for women in the United States. One in two men in the United States will be diagnosed with cancer at some time during his lifetime. One in three women in the United States will be diagnosed with cancer at some time during her lifetime.

A cure for cancer has yet to be found. Current treatment options, such as surgery, chemotherapy and radiation treatment, are often either ineffective or present serious side effects.

#### Metastasis

The most life-threatening forms of cancer often arise when a population of tumor cells gains the ability to colonize distant and foreign sites in the body. These metastatic cells survive by overriding restrictions that normally constrain cell colonization into dissimilar tissues. For example, typical mammary epithelial cells will generally not grow or survive if transplanted to the lung, yet lung metastases are a major cause of breast cancer morbidity and mortality. Recent evidence suggests that dissemination of metastatic cells through the body can occur long before clinical presentation of the primary tumor. These micrometastatic cells may remain dormant for many months or years following the detection and removal of the primary tumor. Thus, a better understanding of the mechanisms that allow for the growth and survival of metastatic cells in a foreign microenvironment is critical for the improvement of therapeutics designed to fight metastatic cancer and diagnostics for the early detection and localization of metastases.

#### Cancer Cell Signaling

Cancer is a disease of aberrant signal transduction. Aberrant cell signaling overrides anchorage-dependent constraints on cell growth and survival (Rhim, et al., Critical Reviews in Oncogenesis 8:305, 1997; Patarca, Critical Reviews in Oncogenesis 7:343, 1996; Malik, et al., Biochimica et Biophysica Acta 1287:73, 1996; Cance, et al., Breast Cancer Res Treat 35:105, 1995). Tyrosine kinase activity is induced by ECM anchorage and indeed, the expression or function of tyrosine kinases is usually increased in malignant cells (Rhim, et al., Critical Reviews in Oncogenesis 8: 305,1997; Cance, et al., Breast Cancer Res Treat 35:105, 1995; Hunter, Cell 88:333, 1997). Based on evidence that tyrosine kinase activity is necessary for malignant cell growth, tyrosine kinases have been targeted with new therapeutics (Levitzki, et al., Science 267:1782, 1995; Kondapaka, et al., Molecular & Cellular Endocrinology 117:53, 1996; Fry, et al., Current Opinion in BioTechnology 6: 662, 1995). Unfortunately, obstacles associated with specific targeting to tumor cells often limit the application of these drugs. In particular, tyrosine kinase activity is often vital for the function and survival of benign tissues (Levitzki, et al., Science 267:1782, 1995). To minimize collateral toxicity, it is critical to identify and then target tyrosine kinases that are selectively overexpressed in tumor cells.

**EphA2**

EphA2 is a 130 kDa receptor tyrosine kinase that is expressed in adult epithelia, where it is found at low levels and is enriched within sites of cell-cell adhesion (Zantek, et al, Cell Growth & Differentiation 10:629, 1999; Lindberg, et al., Molecular & Cellular Biology 10: 6316, 1990). This subcellular localization is important because EphA2 binds ligands (known as EphrinsA1 to A5) that are anchored to the cell membrane (Eph Nomenclature Committee, 1997, Cell 90:403; Gale, et al., 1997, Cell & Tissue Research 290: 227). The primary consequence of ligand binding is EphA2 autophosphorylation (Lindberg, et al., 1990, *supra).* However, unlike other receptor tyrosine kinases, EphA2 retains enzymatic activity in the absence of ligand binding or phosphotyrosine content (Zantek, et al., 1999, *supra).* EphA2 is upregulated on a large number of aggressive carcinoma cells.

**Cancer Therapy**

One barrier to the development of anti-metastasis agents has been the assay systems that are used to design and evaluate these drugs. Most conventional cancer therapies target rapidly growing cells. However, cancer cells do not necessarily grow more rapidly but instead survive and grow under conditions that are non-permissive to normal cells (Lawrence and Steeg, 1996, World J. Urol. 14:124-130). These fundamental differences between the behaviors of normal and malignant cells provide opportunities for therapeutic targeting. The paradigm that micrometastatic tumors have already disseminated throughout the body emphasizes the need to evaluate potential chemotherapeutic drugs in the context of a foreign and three-dimensional microenvironment. Many standard cancer drug assays measure tumor cell growth or survival under typical cell culture conditions *(i. e.,* monolayer growth). However, cell behavior in two-dimensional assays often does not reliably predict tumor cell behavior *in vivo.*

Currently, cancer therapy may involve surgery, chemotherapy, hormonal therapy and/or radiation treatment to eradicate neoplastic cells in a patient (see, for example, Stockdale, 1998, "Principles of Cancer Patient Management," in Scientific American: Medicine, vol. 3, Rubenstein and Federman, eds., Chapter 12, Section IV). Recently, cancer therapy may also involve biological therapy or immunotherapy. All of these approaches can pose significant drawbacks for the patient. Surgery, for example, may be contraindicated due to the health of the patient or may be unacceptable to the patient. Additionally, surgery may not completely remove the neoplastic tissue. Radiation therapy is only effective when the neoplastic tissue exhibits a higher sensitivity to radiation than normal tissue, and radiation therapy can also often elicit serious side effects. Hormonal therapy is rarely given as a single agent and, although it can be effective, is often used to prevent or delay recurrence of cancer after other treatments have removed the majority of the cancer cells. Biological therapies/immunotherapies are limited in number and each therapy is generally effective for a very specific type of cancer.

With respect to chemotherapy, there are a variety of chemotherapeutic agents available for treatment of cancer. A significant majority of cancer chemotherapeutics act by inhibiting DNA synthesis, either directly, or indirectly by inhibiting the biosynthesis of the deoxyribonucleotide triphosphate precursors, to prevent DNA replication and concomitant cell division (see, for example, Gilman et al., Goodman and Gilman's: The Pharmacological Basis of Therapeutics, Eighth Ed. (Pergamom Press, New York, 1990)). These agents, which include alkylating agents, such as nitrosourea, anti-metabolites, such as methotrexate and hydroxyurea, and other agents, such as etoposides, campathecins, bleomycin, doxorubicin, daunorubicin, etc., although not necessarily cell cycle specific, kill cells during S phase because of their effect on DNA replication. Other agents, specifically colchicine and the vinca alkaloids, such as vinblastine and vincristine, interfere with microtubule assembly resulting in mitotic arrest. Chemotherapy protocols generally involve administration of a combination of chemotherapeutic agents to increase the efficacy of treatment.

Despite the availability of a variety of chemotherapeutic agents, chemotherapy has many drawbacks (see, for example, Stockdale, 1998, "Principles Of Cancer Patient Management" in Scientific American Medicine, vol. 3, Rubenstein and Federman, eds., ch. 12, sect. 10). Almost all chemotherapeutic agents are toxic, and chemotherapy causes significant, and often dangerous, side effects, including severe nausea, bone marrow depression, immunosuppression, etc. Additionally, even with administration of combinations of chemotherapeutic agents, many tumor cells are resistant or develop resistance to the chemotherapeutic agents. In fact, those cells resistant to the particular chemotherapeutic agents used in the treatment protocol often prove to be resistant to other drugs, even those agents that act by mechanisms different from the mechanisms of action of the drugs used in the specific treatment; this phenomenon is termed pleiotropic drug or multidrug resistance. Thus, because of drug resistance, many cancers prove refractory to standard chemotherapeutic treatment protocols. DI discloses a method of treating cancer comprising administering an EphA2 agonistic antibody.

There is a significant need for alternative cancer treatments, particularly for treatment of cancer that has proved refractory to standard cancer treatments, such as surgery, radiation therapy, chemotherapy, and hormonal therapy. Further, it is uncommon for cancer to be treated by only one method. Thus, there is a need for development of new therapeutic agents for the treatment of cancer and new, more effective, therapy combinations for the treatment of cancer.

## 3. SUMMARY OF THE INVENTION

The present invention provides antibodies according to claims 1-26.

EphA2 is overexpressed and functionally altered in a large number of malignant carcinomas. EphA2 is an oncoprotein and is sufficient to confer Metastatic potential to cancer cells. EphA2 is also associated with other hyperproliferating cells and is implicated in diseases caused by cell hyperproliferation. EphA2 that is overexpressed on malignant cells exhibits kinase activity independent from ligand binding. The present inventors have found that a decrease in EphA2 levels can decrease proliferation and/or metastatic behavior of a cell. In particular, the present inventors have discovered that, surprisingly, antibodies that agonize EphA2, *i.e.*, elicit EphA2 signaling, actually decrease EphA2 expression and inhibit tumor cell growth and/or metastasis. Although not intending to be bound by any mechanism of action, agonistic antibodies may repress hyperproliferation or malignant cell behavior by inducing EphA22 autophosphorylation, thereby causing subsequent EphA2 degradation to down-regulate expression. Thus, in one embodiment, the EphA2 antibodies of the invention agonize EphA2 signaling and increase phosphorylation of EphA2 ("EphA2 agonistic antibodies").

In addition, cancer cells exhibit phenotypic traits that differ from those of non-cancer cells, for example, formation of colonies in a three-dimensional substrate such as soft agar or the formation of tubular networks or weblike matrices in a three-dimensional basement membrane or extracellular matrix preparation, such as MATRIGEL™. Non-cancer cells do not form colonies in soft agar and form distinct sphere-like structures in three-dimensional basement membrane or extracellular matrix preparations. Accordingly, the invention also provides antibodies that specifically bind EphA2 and inhibit one or more cancer cell phenotypes, such as colony formation in soft agar or tubular network formation in three-dimensional basement membrane or extracellular matrix preparations ("cancer cell phenotype inhibitory EphA2 antibodies"). Exposing cancer cells to such cancer cell phenotype inhibitory EphA2 antibodies prevents or decreases the cells' ability to colonize or form tubular networks in these substrates. Furthermore, in certain embodiments, the addition of such cancer cell phenotype inhibitory EphA2 antibodies to already established colonies of cancer cells cause a reduction or elimination of an existing cancer cell colony, *i.e.*, leads to killing ofhyperproliferative and/or metastatic cells, for example through necrosis or apoptosis.

Differences in the subcellular localization, ligand binding properties or protein organization (*e.g.*, structure, orientation in the cell membrane) can further distinguish the EphA2 that is present on cancer cells from EphA2 on non-cancer cells. In non-cancer cells, EphA2 is expressed at low levels and is localized to sites of cell-cell contact, where it can engage its membrane-anchored ligands. However, cancer cells generally display decreased cell-cell contacts and this can decrease EphA2-ligand binding. Furthermore, the overexpression of EphA2 can cause an excess of EphA2 relative to ligand that increases the amount of non-ligand bound EphA2. Consequently, changes in the subcellular distribution or membrane orientation of EphA2 can cause EphA2 to localize to sites in a cancer cell where it is inaccessible to ligand. Additionally, EphA2 may have altered ligand binding properties (*e.g.*, due to an altered conformation) in cancer cells such that it is incapable of stable interactions with its ligand whether or not it is localized to the cell-cell junction. In each case, these changes can expose certain epitopes on the EphA2 in cancer cells that are not exposed in non-cancer cells. Accordingly, the invention also provides antibodies that specifically bind EphA2 but preferably bind an EphA2 epitope exposed on cancer cells but not on non-cancer cells ("exposed EphA2 epitope antibodies"). Exposing cancer cells to such EphA2 antibodies that preferentially bind epitopes on EphA2 that are selectively exposed or increased on cancer cells but not non-cancer cells targets the therapeutic/prophylactic antibody to cancer cells and prevents or decreases the cells' ability to proliferate while sparing non-cancer cells.

The present inventors have also found that antibodies that bind EphA2 with a very low $K_{off}$ rate are particularly effective to reduce EphA2 expression and/or induce EphA2 degradation and, thereby, inhibit tumor cell growth and/or metastasis and/or proliferation of hyperproliferative cells. Accordingly, the invention thus provides antibodies that bind EphA2 with a $K_{off}$ of less than $3 \times 10^{-3}$ $s^{-1}$ and are EphA2 agonists.

The present invention provides antibodies that bind to EphA2 and agonize EphA2, inhibit a cancer cell phenotype, preferentially bind epitopes on EphA2 that are selectively exposed or increased on cancer cells but not non-cancer cells and have a $K_{off}$ less than $3 \times 10^{-3}$ $s^{-1}$, preferably monoclonal antibodies. In particular, the antibodies of the invention bind to the extracellular domain of EphA2 and, preferably, elicit EphA2 signaling and EphA2 autophosphorylation, inhibit a cancer cell phenotype, preferentially bind an EphA2 epitope exposed on cancer cells but not non-cancer cells, and have a $K_{off}$ of less than 3 X $10^{-3}$ $s^{-1}$.

In a preferred embodiment, the antibodies of the invention are Eph099B-102.147 (PTA-4572), Eph099B-208.261 (PTA-4573), Eph099B-210.248 (PTA-4574), and Eph099B-233.152 (PTA-5194). In a more preferred embodiment, the antibodies of the invention are human or humanized. In a most preferred embodiment, the antibodies of the invention are humanized Eph099B-102.147, Eph099B-208.261, Eph099B-210.248, and Eph099B-233.152.

Accordingly, the present invention relates to pharmaceutical compositions and prophylactic and therapeutic regimens designed to prevent, treat, or manage a disease associated with overexpression of EphA2, particularly cancer, particularly metastatic cancer, in a subject comprising administering one or more antibodies that specifically bind to and agonize EphA2, inhibit a cancer cell phenotype (such as colony formation in soft agar or tubular network formation in a three dimensional basement membrane or extracellular membrane preparation, such as MATRIGEL™), preferentially bind epitopes on EphA2 that are selectively exposed or increased on cancer cells but not non-cancer cells and have a $K_{off}$ less than 3 X $10^{-3}$ $s^{-1}$. In preferred embodiments, the EphA2 antibody decreases the size of colonies already formed in soft agar and/or reduces the extent of tubular network formation in a three-dimensional basement membrane or extracellular matrix preparation. In one embodiment, the cancer is of an epithelial cell origin. In another embodiment, the cancer is a cancer of the skin, lung, colon, prostate, breast, bladder, or pancreas or is a renal cell carcinoma or a melanoma. In a preferred embodiment, the cancer cells in the cancer to be prevented, treated, or managed overexpress EphA2. In a preferred embodiment, some EphA2 is not bound to ligand, either as a result of decreased cell-cell contacts, altered subcellular localization, or increases in amount of EphA2 relative to ligand. In a preferred embodiment, the methods of the invention are used to prevent, treat, or manage metastasis of tumors. The antibodies of the invention can be administered in combination with one or more other cancer therapies. In particular, the present invention provides methods of preventing, treating, or managing cancer in a subject comprising administering to said subject a therapeutically or prophylactically effective amount of one or more EphA2 antibodies of the invention in combination with the administration of a therapeutically or prophylactically effective amount of one or more chemotherapies, hormonal therapies, biological therapies/immunotherapies and/or radiation therapies other than the administration of an EphA2 antibody of the invention or in combination with surgery.

In other embodiments, the EphA2 antibodies of the invention are used to treat, prevent and/or manage a non-cancer disease or disorder associated with cell hyperproliferation, such as but not limited to asthma, chronic obstructive pulmonary disease, restenosis (smooth muscle and/or endothelial), psoriasis, etc. In preferred embodiments, the hyperproliferative cells are epithelial. In preferred embodiments, the hyperproliferative cells overexpress EphA2. In a preferred embodiment, some EphA2 is not bound to ligand, either as a result of decreased cell-cell contacts, altered subcellular localization, or increases in amount of EphA2 relative to EphA2-ligand.

The methods and compositions of the invention are useful not only in untreated patients but are also useful in the treatment of patients partially or completely refractory to current standard and experimental cancer therapies, including but not limited to chemotherapies, hormonal therapies, biological therapies, radiation therapies, and/or surgery as well as to improve the efficacy of such treatments. In particular, EphA2 expression has been implicated in increasing levels of the cytokine IL-6, which has been associated with the development of cancer cell resistance to different treatment regimens, such as chemotherapy and hormonal therapy. In addition, EphA2 overexpression can override the need for estrogen receptor activity thus contributing to tamoxifen resistance in breast cancer cells. Accordingly, in a preferred embodiment, the invention provides therapeutic and prophylactic methods for the treatment or prevention of cancer that has been shown to be or may be refractory or non-responsive to therapies other than those comprising administration of EphA2 antibodies of the invention. In a specific embodiment, one or more EphA2 antibodies of the invention are administered to a patient refractory or non-responsive to a non-EphA2-based treatment, particularly tamoxifen treatment or a treatment in which resistance is associated with increased IL-6 levels, to render the patient non-refractory or responsive. The treatment to which the patient had previously been refractory or non-responsive can then be administered with therapeutic effect.

In addition, the present invention describes methods of screening for EphA2 antibodies of the invention. In particular, antibodies may be screened for binding to EphA2, particularly the extracellular domain of EphA2, using routine immunological techniques. In one area, to identify agonistic EphA2 antibodies, EphA2 antibodies may be screened for the ability to elicit EphA2 signaling, *e.g.*, increase EphA2 phosphorylation and/or to degrade EphA2.

In another embodiment, to identify cancer cell phenotype inhibiting antibodies, anti-EphA2 antibodies may be screened for the ability to prevent or reduce cancer cell colony formation in soft agar or reduce or inhibit tubular network formation in a three-dimensional basement membrane or extracellular matrix preparation, or any other method that detects a decrease in a cancer phenotype, for example, any assay that detects an increase in contact inhibition of cell proliferation (*e.g.*, reduction of colony formation in a monolayer cell culture). In preferred embodiments, the antibodies are screened for ability to decrease the size of existing colonies in soft agar or reduce the extent or tubular matrix formation in the three-dimensional basement membrane or extracellular matrix preparation, particular induces cell (particularly cancer cell, more particularly metastatic cancer cell, but also including other hyperproliferative cell) necrosis or apoptosis. Additionally, antibodies may be screened for their ability to inhibit or reduce colony formation in soft agar and/or tubular network formation in three-dimensional basement membrane or extracellular matrix preparations in the presence of other anti-cancer agents, *e.g.*, hormonal, chemotherapeutic, biologic or other anti-cancer agents.

In another embodiment, to identify antibodies that preferentially bind an EphA2 epitope exposed on cancer cells but not non-cancer cells, antibodies may be screened for the ability to preferentially bind EphA2 that is not bound to ligand, *e.g.*, Ephrin AI, and that is not localized to cell-cell contacts. Any method known in the art to determine antibody binding/localization on a cell can be used to screen candidate antibodies for desirable binding properties. In a specific embodiment, immunofluorescence microscopy or flow cytometry is used to determine the binding characteristics of an antibody. In this embodiment, antibodies that bind poorly to EphA2 when it is bound to its ligand and localized to cell-cell contacts but bind well to free EphA2 on a cell are encompassed by the invention. In another specific embodiment, EphA2 antibodies are selected for their ability to compete with ligands (*e.g.*, cell-anchored or purified ligands) for binding to EphA2 using cell-based or ELISA assays.

In another area, antibodies are screened using antibody binding kinetic assays well known in the art (*e.g.*, surface plasmon resonance based assays, such as a BIACORE™ assay) to identify antibodies having a $K_{off}$ rate less than $3 \times 10^{-3}$ $s^{-1}$.

In other embodiments, the invention provides antibodies for treating, preventing, or managing cancer, by administering therapeutic agents, other than EphA2 antibodies of the invention, that reduce EphA2 protein levels, for example but not by way of limitation, anti-sense nucleic acids specific for EphA2, double stranded EphA2 RNA that mediates RNA interference of EphA2 expression, anti-EphA2 ribozymes, etc., as well as other inhibitors of EphA2, for example, small molecule inhibitors of EphA2.

The present inventors have also found that increased EphA2 expression correlates with increased fibronectin expression. Moreover, high levels of exogenous fibronectin increase cells' ability to form colonies in soft agar while specific inhibitors of cell-fibronectin attachment decrease colony formation of tumor-derived cancer cells in soft agar. Thus, fibronectin appears to accommodate tumor cell colonization in foreign environments, *e.g.*, formation and growth of distal metastases. Accordingly, in a particular embodiment, the invention provides, either alone or in combination with the EphA2 antibodies of the invention, methods of treating, preventing, or managing cancer, particularly metastatic disease, by administering an agent that prevents cell-fibronectin binding and/or fibronectin expression.

The invention further provides diagnostic uses using the EphA2 antibodies of the invention to evaluate the efficacy of cancer treatment, either EphA2-based or not EphA2-based. In general, increased EphA2 expression is associated with increasingly invasive and metastatic cancers. Accordingly, a reduction in EphA2 expression with a particular treatment indicates that the treatment is reducing the invasiveness and/or metastatic potential of cancer. The diagnostic uses of the invention may also be used to prognose or predict the course of cancer or outcomes of cancer therapy. In particular embodiments, the diagnostic uses of the invention provide uses of imaging and localizing metastases and methods of diagnosis and prognosis using tissues and fluids distal to the primary tumor site (as well as methods using tissues and fluids of the primary tumor), for example, whole blood, sputum, urine, serum, fine needle aspirates (*i.e.*, biopsies). In other embodiments, the diagnostic uses of the invention provide uses of imaging and localizing metastases and methods of diagnosis and prognosis *in vivo*. In such embodiments, primary metastatic tumors are detected using an antibody of the invention, preferably an exposed EphA2 epitope antibody. The antibodies of the invention may also be used for immunohistochemical analyses of frozen or fixed cells or tissue assays. In addition, the antibodies and diagnostic methods of the invention may be used to diagnose, prognose or monitor therapy of (whether EphA2 or non-EphA2-based therapy) non-cancer hyperproliferative diseases (particularly associated with EphA2 overexpression), for example, but not limited to, asthma, psoriasis, restenosis, chronic obstructive pulmonary disease, etc.

In another embodiment, kits comprising the pharmaceutical compositions or diagnostic reagents of the invention are provided.

## 3.1 DEFINITIONS

As used herein, the term "agonist" refers to any compound including a protein, polypeptide, peptide, antibody, antibody fragment, large molecule, or small molecule (less than 10 kD), that increases the activity, activation or function of another molecule. EphA2 agonists cause increased phosphorylation and degradation of EphA2 protein. EphA2 antibodies that agonize EphA2 may or may not also inhibit cancer cell phenotype (*e.g.*, colony formation in soft agar or tubular network formation in a three-dimensional basement membrane or extracellular matrix preparation) and may or may not preferentially bind an EphA2 epitope that is exposed in a cancer cell relative to a non-cancer cell and have a low $K_{off}$ rate.

The term "antibodies or fragments thereof that immunospecifically bind to EphA2" as used herein refers to antibodies or fragments thereof that specifically bind to an EphA2 polypeptide or a fragment of an EphA2 polypeptide and do not specifically bind to other non-EphA2 polypeptides. Preferably, antibodies or fragments that immunospecifically bind to an EphA2 polypeptide or fragment thereof do not non-specifically cross-react with other antigens (*e.g.*, binding cannot be competed away with a non-EphA2 protein, *e.g.*, BSA in an appropriate immunoassay). Antibodies or fragments that immunospecifically bind to an EphA2 polypeptide can be identified, for example, by immunoassays or other techniques known to those of skill in the art. Antibodies of the invention include, but are not limited to, synthetic antibodies, monoclonal antibodies, recombinantly produced antibodies, intrabodies, multispecific antibodies (including bi-specific antibodies), human antibodies, humanized antibodies, chimeric antibodies, synthetic antibodies, single-chain Fvs (scFv) (including bi-specific scFvs), single chain antibodies Fab fragments, F(ab') fragments, disulfide-linked Fvs (sdFv), and anti-idiotypic (anti-Id) antibodies, and epitope-binding fragments of any of the above. In particular, antibodies of the present invention include immunoglobulin molecules and immunologically active portions of immunoglobulin molecules, *i.e.*, molecules that contain an antigen binding site that immunospecifically binds to an EphA2 antigen (*e.g.*; one or more complementarity determining regions (CDRs) of an anti-EphA2 antibody). Preferably agonistic antibodies or fragments thereof that immunospecifically bind to an EphA2 polypeptide or fragment thereof preferentially agonize EphA2 and do not significantly agonize other activities.

As used herein, the term "cancer" refers to a disease involving cells that have the potential to metastasize to distal sites and exhibit phenotypic traits that differ from those of non-cancer cells, for example, formation of colonies in a three-dimensional substrate such as soft agar or the formation of tubular networks or weblike matrices in a three-dimensional basement membrane or extracellular matrix preparation, such as MATRIGEL™. Non-cancer cells do not form colonies in soft agar and form distinct sphere-like structures in three-dimensional basement membrane or extracellular matrix preparations. Cancer cells acquire a characteristic set of functional capabilities during their development, albeit through various mechanisms. Such capabilities include evading apoptosis, self sufficiency in growth signals, insensitivity to anti-growth signals, tissue invasion/metastasis, limitless replicative potential, and sustained angiogenesis. The term "cancer cell" is meant to encompass both pre-malignant and malignant cancer cells.

As used herein, the phrase "cancer cell phenotype inhibiting" refers to the ability of a compound to prevent or reduce cancer cell colony formation in soft agar or tubular network formation in a three-dimensional basement membrane or extracellular matrix preparation or any other method that detects a reduction in a cancer cell phenotype, for example, assays that detect an increase in contact inhibition of cell proliferation (*e.g.*, reduction of colony formation in a monolayer cell culture). Cancer cell phenotype inhibiting compounds may also cause a reduction or elimination of colonies when added to established colonies of cancer cells in soft agar or the extent of tubular network formation in a three-dimensional basement membrane or extracellular matrix preparation. EphA2 antibodies that inhibit cancer cell phenotype may or may not also agonize EphA2 and have a low $K_{off}$ rate.

The term "derivative" as used herein refers to a polypeptide that comprises an amino acid sequence of an EphA2 polypeptide, a fragment of an EphA2 polypeptide, an antibody that immunospecifically binds to an EphA2 polypeptide, or an antibody fragment that immunospecifically binds to an EphA2 polypeptide, that has been altered by the introduction of amino acid residue substitutions, deletions or additions (*i.e.*, mutations). In some embodiments, an antibody derivative or fragment thereof comprises amino acid residue substitutions, deletions or additions in one or more CDRs. The antibody derivative may have substantially the same binding, better binding, or worse binding when compared to a non-derivative antibody. In specific embodiments, one, two or three, amino acid residues of the CDR have been substituted, deleted or added (*i.e.*, mutated). The term "derivative" as used herein also refers to an EphA2 polypeptide, a fragment of an EphA2 polypeptide, an antibody that immunospecifically binds to an EphA2 polypeptide, or an antibody fragment that immunospecifically binds to an EphA2 polypeptide which has been modified, *i.e*, by the covalent attachment of any type of molecule to the polypeptide. For example, but not by way of limitation, an EphA2 polypeptide, a fragment of an EphA2 polypeptide, an antibody, or antibody fragment may be modified, *e.g.*, by glycosylation, acetylation, pegylation, phosphorylation, amidation, derivatization by known protecting/blocking groups, proteolytic cleavage, linkage to a cellular ligand or other protein, etc. A derivative of an EphA2 polypeptide, a fragment of an EphA2 polypeptide, an antibody, or antibody fragment may be modified by chemical modifications using techniques known to those of skill in the art, including, but not limited to, specific chemical cleavage, acetylation, formylation, metabolic synthesis of tunicamycin, etc. Further, a derivative of an EphA2 polypeptide, a fragment of an EphA2 polypeptide, an antibody, or antibody fragment may contain one or more non-classical amino acids. In one embodiment, a polypeptide derivative possesses a similar or identical function as an EphA2 polypeptide, a fragment of an EphA2 polypeptide, an antibody, or antibody fragment described herein. In another embodiment, a derivative of EphA2 polypeptide, a fragment of an EphA2 polypeptide, an antibody, or antibody fragment has an altered activity when compared to an unaltered polypeptide. For example, a derivative antibody or fragment thereof can bind to its epitope more tightly or be more resistant to proteolysis.

The term "epitope" as used herein refers to a portion of an EphA2 polypeptide having antigenic or immunogenic activity in an animal, preferably in a mammal, and most preferably in a mouse or a human. An epitope having immunogenic activity is a portion of an EphA2 polypeptide that elicits an antibody response in an animal. An epitope having antigenic activity is a portion of an EphA2 polypeptide to which an antibody immunospecifically binds as determined by any method well known in the art, for example, by immunoassays. Antigenic epitopes need not necessarily be immunogenic.

The "fragments" described herein include a peptide or polypeptide comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least contiguous 90 amino acid residues, at least contiguous 100 amino acid residues, at least contiguous 125 amino acid residues, at least 150 contiguous amino acid residues, at least contiguous 175 amino acid residues, at least contiguous 200 amino acid residues, or at least contiguous 250 amino acid residues of the amino acid sequence of an EphA2 polypeptide or an antibody that immunospecifically binds to an EphA2 polypeptide. Preferably, antibody fragments are epitope-binding fragments.

As used herein, the term "humanized antibody" refers to forms of non-human (*e.g.*, murine) antibodies that are chimeric antibodies which contain minimal sequence derived from non-human immunoglobulin. For the most part, humanized antibodies are human immunoglobulins (recipient antibody) in which hypervariable region residues of the recipient are replaced by hypervariable region residues from a non-human species (donor antibody) such as mouse, rat, rabbit or non-human primate having the desired specificity, affinity, and capacity. In some instances, Framework Region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. that immunospecifically binds to an EphA2 polypeptide, that has been altered by the introduction of amino acid residue substitutions, deletions or additions (*i.e.*, mutations). In some embodiments, a humanized antibody is a derivative. Such a humanized antibody comprises amino acid residue substitutions, deletions or additions in one or more non-human CDRs. The humanized antibody derivative may have substantially the same binding, better binding, or worse binding when compared to a non-derivative humanized antibody. In specific embodiments, one, two, three, four, or five amino acid residues of the CDR have been substituted, deleted or added (*i.e.*, mutated). For further details in humanizing antibodies, see European Patent Nos. EP 239,400, EP 592,106, and EP 519,596; International Publication Nos. WO 91/09967 and WO 93/17105; U.S. Patent Nos. 5,225,539, 5,530,101, 5,565,332, 5,585,089, 5,766,886, and 6,407,213; and Padlan, 1991, Molecular Immunology 28(4/5):489-498; Studnicka et al., 1994, Protein Engineering 7(6):805-814; Roguska et al., 1994, PNAS 91:969-973; Tan et al., 2002, J. Immunol. 169:1119-25; Caldas et al., 2000, Protein Eng. 13:353-60; Morea et al., 2000, Methods 20:267-79; Baca et al., 1997, J. Biol. Chem. 272:10678-84; Roguska et al., 1996, Protein Eng. 9:895-904; Couto et al., 1995, Cancer Res. 55 (23 Supp): 5973s-5977s; Couto et al., 1995, Cancer Res. 55:1717-22; Sandhu, 1994, Gene 150:409-10; Pedersen et al., 1994, J. Mol. Biol. 235:959-73; Jones et al., 1986, Nature 321:522-525; Reichmann et al., 1988, Nature 332:323-329; and Presta, 1992, Curr. Op. Struct. Biol. 2:593-596.

As used herein, the term "hypervariable region" refers to the amino acid residues of an antibody which are responsible for antigen binding. The hypervariable region comprises amino acid residues from a "Complementarity Determining Region" or "CDR" (*i.e.*, residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (*i.e.*, residues 26-32 (L1), 50-52 (L2) and 91-96 (L3) in the light chain variable domain and 26-32 (H1), 53-55 (H2) and 96-101 (H3) in the heavy chain variable domain; Chothia and Lesk, 1987, J. Mol. Biol. 196:901-917). CDR residues for Eph099B-208.261 and Eph099B-233.152 are listed in Table 1. "Framework Region" or "FR" residues are those variable domain residues other than the hypervariable region residues as herein defined.

As used herein, the term "in combination" refers to the use of more than one prophylactic and/or therapeutic agents. The use of the term "in combination" does not restrict the order in which prophylactic and/or therapeutic agents are administered to a subject with a hyperproliferative cell disorder, especially cancer. A first prophylactic or therapeutic agent can be administered prior to (*e.g.*, 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.g.*, 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second prophylactic or therapeutic agent to a subject which had, has, or is susceptible to a hyperproliferative cell disorder, especially cancer. The prophylactic or therapeutic agents are administered to a subject in a sequence and within a time interval such that the agent of the invention can act together with the other agent to provide an increased benefit than if they were administered otherwise. Any additional prophylactic or therapeutic agent can be administered in any order with the other additional prophylactic or therapeutic agents.

As used herein, the phrase "low tolerance" refers to a state in which the patient suffers from side effects from treatment so that the patient does not benefit from and/or will not continue therapy because of the adverse effects and/or the harm from the side effects outweighs the benefit of the treatment.

As used herein, the terms "manage," "managing" and "management" refer to the beneficial effects that a subject derives from administration of a prophylactic or therapeutic agent, which does not result in a cure of the disease. In certain embodiments, a subject is administered one or more prophylactic or therapeutic agents to "manage" a disease so as to prevent the progression or worsening of the disease.

As used herein, the phrase "non-responsive/refractory" is used to describe patients treated with one or more currently available therapies (*e.g.*, cancer therapies) such as chemotherapy, radiation therapy, surgery, hormonal therapy and/or biological therapy/immunotherapy, particularly a standard therapeutic regimen for the particular cancer, wherein the therapy is not clinically adequate to treat the patients such that these patients need additional effective therapy, *e.g.*, remain unsusceptible to therapy. The phrase can also describe patients who respond to therapy yet suffer from side effects, relapse, develop resistance, etc. In various embodiments, "non-responsive/refractory" means that at least some significant portion of the cancer cells are not killed or their cell division arrested. The determination of whether the cancer cells are "non-responsive/refractory" can be made either *in vivo* or *in vitro* by any method known in the art for assaying the effectiveness of treatment on cancer cells, using the art-accepted meanings of "refractory" in such a context. In various embodiments, a cancer is "non-responsive/refractory" where the number of cancer cells has not been significantly reduced, or has increased during the treatment.

As used herein, the term "potentiate" refers to an improvement in the efficacy of a therapeutic agent at its common or approved dose.

As used herein, the terms "Prevent," "preventing" and "prevention" refer to the prevention of the onset, recurrence, or spread of a disease in a subject resulting from the administration of a prophylactic or therapeutic agent.

As used herein, the term prophylactic agent" refers to any agent that can be used in the prevention of the onset, recurrence or spread of a disease or disorder associated with EphA2 overexpression and/or cell hyperproliferative disease, particularly cancer. In certain embodiments, the term "prophylactic agent" refers to an EphA2 agonistic antibody, an EphA2 cancer cell phenotype inhibiting antibody, an exposed EphA2 epitope antibody, or an antibody that binds EphA2 with a $K_{off}$ of less than 3 X $10^{-3}$ $s^{-1}$ (*e.g.*, Eph099B-102.147, Eph099B-208.261, Eph099B-210.248, Eph099B-233.152). In certain other embodiments, the term "prophylactic agent" refers to cancer chemotherapeutics, radiation therapy, hormonal therapy, biological therapy (*e.g.*, immunotherapy), and/or EphA2 antibodies of the invention. In other embodiments, more than one prophylactic agent may be administered in combination.

As used herein, a "prophylactically effective amount" refers to that amount of the prophylactic agent sufficient to result in the prevention of the onset, recurrence or spread of cell hyperproliferative disease, preferably, cancer. A prophylactically effective amount may refer to the amount of prophylactic agent sufficient to prevent the onset, recurrence or spread of hyperproliferative disease, particularly cancer, including but not limited to those predisposed to hyperproliferative disease, for example, those genetically predisposed to cancer or previously exposed to carcinogens. A prophylactically effective amount may also refer to the amount of the prophylactic agent that provides a prophylactic benefit in the prevention of hyperproliferative disease. Further, a prophylactically effective amount with respect to a prophylactic agent of the invention means that amount of prophylactic agent alone, or in combination with other agents, that provides a prophylactic benefit in the prevention of hyperproliferative disease. Used in connection with an amount of an EphA2 antibody of the invention, the term can encompass an amount that improves overall prophylaxis or enhances the prophylactic efficacy of or synergies with another prophylactic agent.

A used herein, a "protocol" includes dosing schedules and dosing regimens.

As used herein, the phrase "side effects" encompasses unwanted and adverse effects of a prophylactic or therapeutic agent. Adverse effects are always unwanted, but unwanted effects are not necessarily adverse. An adverse effect from a prophylactic or therapeutic agent might be harmful or uncomfortable or risky. Side effects from chemotherapy include, but are not limited to, gastrointestinal toxicity such as, but not limited to, early and late-forming diarrhea and flatulence, nausea, vomiting, anorexia, leukopenia, anemia, neutropenia, asthenia, abdominal cramping, fever, pain, loss of body weight, dehydration, alopecia, dyspnea, insomnia, dizziness, mucositis, xerostomia, and kidney failure, as well as constipation, nerve and muscle effects, temporary or permanent damage to kidneys and bladder, flu-like symptoms, fluid retention, and temporary or permanent infertility. Side effects from radiation therapy include but are not limited to fatigue, dry mouth, and loss of appetite. Side effects from biological therapies/immunotherapies include but are not limited to rashes or swellings at the site of administration, flu-like symptoms such as fever, chills and fatigue, digestive tract problems and allergic reactions. Side effects from hormonal therapies include but are not limited to nausea, fertility problems, depression, loss of appetite, eye problems, headache, and weight fluctuation. Additional undesired effects typically experienced by patients are numerous and known in the art. Many are described in the Physicians' Desk Reference (56th ed., 2002).

As used herein, the terms "single-chain Fv" or "scFv" refer to antibody fragments comprise the VH and VL domains of antibody, wherein these domains are present in a single polypeptide chain. Generally, the Fv polypeptide further comprises a polypeptide linker between the VH and VL domains which enables the scFv to form the desired structure for antigen binding. For a review of sFv see Pluckthun in The Pharmacology of Monoclonal Antibodies, vol. 113, Rosenburg and Moore eds. Springer-Verlag, New York, pp. 269-315 (1994). In specific embodiments, scFvs include bi-specific scFvs and humanized scFvs.

As used herein, the terms "subject" and "patient" are used interchangeably. As used herein, a subject is preferably a mammal such as a non-primate (*e.g.*, cows, pigs, horses, cats, dogs, rats etc.) and a primate (*e.g.*, monkey and human), most preferably a human.

As used herein, the terms "treat," "treating" and "treatment" refer to the eradication, reduction or amelioration of symptoms of a disease or disorder, particularly, the eradication, removal, modification, or control of primary, regional, or metastatic cancer tissue that results from the administration of one or more therapeutic agents. In certain embodiments, such terms refer to the minimizing or delaying the spread of cancer resulting from the administration of one or more therapeutic agents to a subject with such a disease.

As used herein, the term "therapeutic agent" refers to any agent that can be used in the prevention, treatment, or management of a disease or disorder associated with overexpression of EphA2 and/or cell hyperproliferative diseases or disorders, particularly, cancer. In certain embodiments, the term "therapeutic agent" refers to an EphA2 agonistic antibody, an EphA2 cancer cell phenotype inhibiting antibody, an exposed EphA2 epitope antibody, or an antibody that binds EphA2 with a $K_{off}$ of less than 3 X $10^{-3}$ $s^{-1}$ (*e.g.*, Eph099B-102.147, Eph099B-208.261, Eph099B-210.248, Eph099B-233.152). In certain other embodiments, the term "therapeutic agent" refers to cancer chemotherapeutics, radiation therapy, hormonal therapy, biological therapy/immunotherapy, and/or EphA2 antibody of the invention. In other embodiments, more than one therapeutic agent may be administered in combination.

As used herein, a "therapeutically effective amount" refers to that amount of the therapeutic agent sufficient to treat or manage a disease or disorder associated with EphA2 overexpression and/or cell hyperproliferative disease and, preferably, the amount sufficient to destroy, modify, control or remove primary, regional or metastatic cancer tissue. A therapeutically effective amount may refer to the amount of therapeutic agent sufficient to delay or minimize the onset of the hyperproliferative disease, *e.g.*, delay or minimize the spread of cancer. A therapeutically effective amount may also refer to the amount of the therapeutic agent that provides a therapeutic benefit in the treatment or management of cancer. Further, a therapeutically effective amount with respect to a therapeutic agent of the invention means that amount of therapeutic agent alone, or in combination with other therapies, that provides a therapeutic benefit in the treatment or management of hyperproliferative disease or cancer. Used in connection with an amount of an EphA2 antibody of the invention, the term can encompass an amount that improves overall therapy, reduces or avoids unwanted effects, or enhances the therapeutic efficacy of or synergies with another therapeutic agent.

## 4. DESCRIPTION OF THE FIGURES

**FIG.1:** Eph099B-208.261 can compete with EA2 for binding to EphA2 in a competitive ELISA assay. The ability of labeled EA2 monoclonal antibody to bind EphA2-Fc was assayed by competitive ELISA in presence of either unlabeled monoclonal antibodies EA2 or Eph099B-208.261. Ratios of unlabeled to labeled antibody used in the assay are indicated on the x-axis. EA2 is indicated by diamonds and Eph099B-208.261 is indicated by squares.

**FIGS. 2A-2D:** EphA2 antibodies promote EphA2 tyrosine phosphorylation in MDA-MB-231 cells. Monolayers of MDA-MB-231 cells were incubated in the presence of a single dose of 5μg/ml (**A, C**) Eph099B-208.261 or (**B, D**) EA2 for the indicated time at 37°C. Cell lysates were then immunoprecipitated with an EphA2-specific antibody, resolved by SDS-PAGE and subjected to western blot analysis with a phosphotyrosine-specific antibody (**A, B**). The membranes were stripped and re-probed with the EphA2-specific antibody used in the immunoprecipitation as a loading control (**C, D**).

**FIGS. 3A-3D:** EphA2 antibodies promote EphA2 degradation in MDA-MB-231 cells. Monolayers of MDA-MB-231 cells were incubated in the presence of a single dose of 5μg/ml (**A, C**) Eph099B-208.261 or (**B, D**) EA2 for the indicated time at 37°C. Cell lysates were then resolved by SDS-PAGE and subjected to western blot analysis with an EphA2-specific antibody (**A, B**). The membranes were stripped and re-probed with a β-catenin-specific antibody as a loading control (**C, D**).

**FIGS. 4A-4B:** EphA2 Eph099B-233.152 antibody promotes EphA2 tyrosine phosphorylation and EphA2 degradation in MDA-MB-231 cells. Monolayers of MDA-MB-231 cells were incubated in the presence of a single dose of 5μg/ml Eph099B-233.152 at 37°C. Cell lysates were then immunoprecipitated with D7 (an EphA2-specific antibody), resolved by SDS-PAGE and subjected to western blot analysis with (**A**) a phosphotyrosine-specific antibody or (**B**) an EphA2-specific antibody.

**FIG. 5:** EphA2 antibodies inhibit malignant tumor cell growth in soft agar. A single dose of 5 μg/ml of Eph099B-208.261 (black bar), EA2 (white bar) purified EphA2 antibodies or a negative control antibody, 1A7 (gray bar) were incubated with malignant MDA-MB-231 tumor cells for the indicated time at 37°C in soft agar. Results are reported as colonies per high-powered field (HPF).

**FIGS. 6A-6B:** EphA2 Eph099B-233.152 antibody inhibits tumor cell growth *in vivo.* MDA-MB-231 cells were implanted subcutaneously into athymic mice. After the tumors had grown to an average volume of $100mm^3$, mice were administered 6mg/ml Eph099B-233.152 or PBS control intraperitoneally twice a week for 3 weeks. (**A**) Tumor Growth. Tumor growth was assessed and expressed as a ratio of the tumor volume divided by initial tumor volume (100 $mm^3$).

Control mice are indicated by circles and Eph099B-233.152-treated mice are indicated by squares. Arrows indicate time of Eph099B-233.152 or PBS administration. (**B**) Survival. Tumor growth was allowed to proceed until tumor volume reached $1000mm^3$. Survival of the mice was assessed by scoring the percent of mice living each day post treatment. Control mice are indicated by grey and Eph099B-233.152-treated mice are indicated by black.

**FIGS. 7A-7D:** The EphA2 antibodies, EA2, Eph099B-208.261, and Eph099B-233.152, inhibit tumor cell growth *in vivo.* MDA-MB-231 breast cancer cells were implanted (**A**) orthotopically or (**B**) subcutaneously into athymic mice. (**C**) A549 lung cancer cells were implanted subcutaneously into athymic mice. After the tumors had grown to an average volume of $100mm^3$, mice were administered 6 mg/kg of the indicated antibody or negative control (PBS or 1A7 antibody) intraperitoneally twice a week for 3 weeks. Tumor growth was assessed and expressed as a ratio of the tumor volume divided by initial tumor volume ($100\ mm^3$). (**D**) MDA-MB-231 breast cancer cells were implanted subcutaneously into athymic mice. After the tumors had grown to an average volume of $100mm^3$, mice were administered 6 mg/kg of the indicated antibody or negative control intraperitoneally twice a week for 3 weeks. Total tumor volume was determined after sacrifice. Negative control is black, EA2 is white, Eph099B-208.261 is dark grey, and Eph099B-233.152 is light grey.

**FIGS. 8A-8B:** EphA2 overexpression selectively increases malignant cell growth. (A) $1x10^5$ control (white bar) or MCF-$7^{EphA2}$ cells (black bar) were suspended in soft agar in the presence of 1 mg/ml 17β-estradiol for 14 days prior to microscopic evaluation. EphA2-transfected cells formed more colonies (47 colonies/high powered field (HPF)) than matched controls (1 colony/HPF; P<0.01). (**B**) Monolayer growth assays did not distinguish between the growth of control (white circles) and MCF-$7^{EphA2}$ cells (black squares).

**FIGS. 9A-9B:** EphA2 overexpression increases tumorigenic potential. (**A**) $1x10^6$ control (white circle) or MCF-$7^{EphA2}$ cells (black square) were implanted into the mammary fatpad of athymic mice (n=20 mice per group) in the presence of supplemental estrogen (1 μM 17β-estradiol). The tumors formed by MCF-$7^{EphA2}$ cells were significantly larger than tumors formed by matched controls (P = 0.027). (**B**) Equal amounts of protein lysate, isolated from input cells or resected tumors (T) were evaluated by western blot analyses with an EphA2 antibody (D7). The membranes were stripped and re-probed with a β-catenin-specific antibody as a loading control.

**FIGS. 10A-10C:** EphA2 overexpression decreases estrogen dependence. (A) $1x10^5$ control (white bar) or MCF-$7^{EphA2}$ cells (black bar) were suspended in soft agar in the absence of exogenous estrogen and colony formation was evaluated microscopically after 14 days. The monolayer growth (**B**) and tumorigenic potential (**C**) of MCF-$7^{EphA2}$ (black square) cells were increased relative to matched controls (white circle) in the absence of supplemental estrogen (P<0.01 and P<0.004, respectively).

**FIGS. 11A-11B:** EphA2 overexpression decreases tamoxifen sensitivity. (**A**) $1x10^5$ MCF-7 or MCF-$7^{EphA2}$ cells were suspended in soft agar in the presence of 1μM tamoxifen (TAM) and or 1μM 17β-estradiol and colony formation was evaluated microscopically after 14 days. (**B**) MCF-7 (circles) or MCF-$7^{EphA2}$ cells (squares) were implanted into the mammary fatpad (n=15 mice per group) in the presence of supplemental estrogen. Tamoxifen treatment was initiated 17 days post-implantation. Tumor volume of tamoxifen treated (black circles and squares) and saline treated (white circles and squares) animals was measured at the indicated time. Note the lower inhibitory effects of tamoxifen on MCF-$7^{EphA2}$ relative to control cells (P=0.01).

**FIGS. 12A-12F:** Estrogen receptor is expressed but functionally altered in MCF-$7^{EphA2}$ cells. (**A**) ERα and (**B**) ERβ levels were evaluated in MCF-$7^{neo}$ control cells and MCF-$7^{EphA2}$ cells by western blot analyses with an EphA2-specific antibody (D7). (**C**, **D**) The membranes were stripped and re-probed with a β-catenin-specific antibody as a loading control. (**E, F**) Estrogen receptor activity was measured using a CAT reporter system, revealing comparable estrogen receptor activity in control and MCF-$7^{EphA2}$ cells. The average results from three experiments are graphed in (**F**). E2 indicates estrogen treatment; TAM indicates tamoxifen treatment; % conversion indicates the amount of substrate converted from non-acetylated substrate (non-AC) to acetylated substrate (AC) by CAT enzyme.

**FIGS. 13A-13C:** EphA2 agonistic antibody EA2 decreases malignant growth. MCF-$7^{EphA2}$ cells were incubated in the presence of 3 μg/ml of EA2 for the time indicated prior to sample extraction and western blot analyses with an EphA2-specific antibody (D7). (**B**) The membrane was stripped and re-probed with a β-catenin-specific antibody as a loading control. (**C**) $1x10^5$ control or MCF-$7^{EphA2}$ cells were suspended in soft agar in the presence or absence of tamoxifen (TAM, 1μM) and EphA2 agonistic antibody (EA2, 10μg/ml). Note that EA2 increased the sensitivity of MCF-$7^{EphA2}$ cells to tamoxifen.

**FIGS. 14A-14B:** Decreased EphA2 protein levels are sufficient to reduce tumor cell colonization of soft agar. Monolayers of MDA-MB-231 cells were transfected with 2 μg/ml of EphA2 antisense or inverse antisense (IAS) oligonucleotides at 37°C for 24 hours. (**A, B**) Western blot analysis of whole cell lysates with EphA2-specific D7 antibody confirms that transfection with antisense oligonucleotides decreases EphA2 protein levels (**A**). The membranes were stripped and reprobed with paxillin antibodies as a loading control (**B**). The relative mobility of molecular mass standards is shown on the left of panels A and B. (C) MDA-MB-231 cell monolayers, treated with antisense oligonucleotides as detailed above, were suspended in soft agar for 7 days before microscopic analysis of colony formation. Note that colony formation by MDA-MB -231 cells was significantly impaired by EphA2 antisense oligonucleotides as compared to the inverted antisense control (P < 0.002). Results are reported as colonies per high-powered field (HPF).

**FIG. 15:** Kinetic analysis of EphA2 monoclonal antibodies. BIACORE™ (surface plasmon resonance-based) assays were used to assay the kinetics of EphA2 monoclonal antibody binding to immobilized EphA2-Fc. Eph099B-208.261 is indicated by a solid line, Eph099B-233.152 is indicated by a dotted line, EA2 is indicated by a dashed line, and the negative control is indicated by squares.

**FIGS. 16A-16D.:** EphA2 EA2 antibody preferentially binds cancer cells. Non-transformed MCF-10A (**A, C**) or transformed MDA-MB-231 (**B, D**) cells were incubated with 10 μg/ml (**A, B**) Eph099B-233.152 or (**C, D**) EA2 at 4°C prior to fixation and immunolabeling with fluorophore-conjugated anti-mouse IgG.

**FIGS. 17A-17D:** EphA2 EA2 antibody preferentially binds EphA2 epitopes exposed by decreasing cell-cell contacts. (**A, B**) Non-transformed MCF-10A cells were labeled with EA2 at 4°C either before (**A**) or after (**B**) treatment with EGTA and prior to fixation and immunolabeling with fluorophore-conjugated anti-mouse IgG. (**C, D**) Non-transformed MCF-10A (**C**) or transformed MDA-MB-231 (**D**) cells were labeled with EA2 either before (middle) or after (top) treatment with EGTA. Control cells were incubated with secondary antibody alone (bottom). The amount of EA2-EphA2 binding was measured using flow cytometry.

**FIGS.18A-18B:** EphA2 EA2 epitope is distinct from Eph099B-233.152 epitope and ligand binding site. (**A**) EphA2- $F_c$ was incubated with and bound to immobilized Ephrin AI-$F_c$. Labeled Ephrin AI-$F_c$ (black), EA2 (white) or Eph099B-233.152 (grey) was incubated with the EphA2-Ephrin AI-$F_c$ complex and amount of binding was measured. (**B**) EphA2-$F_c$ was incubated with and bound to immobilized Ephrin A1-$F_{c.}$ Labeled EA2 was then incubated with the EphA2-Ephrin A1 complex. Unlabeled competitor was incubated with EphA2-Ephrin A1-EA2 complex in the indicated amount. Competitors were Ephrin A1-$F_c$ (black), EA2 (white) or Eph099B-233.152 (grey).

**FIG.19:** Sequences of VL and VH of EphA2 antibodies. Amino acid and nucleic acid sequences of Eph099B-208.261 (**A**) VL (SEQ ID NOs:1 and 9, respectively) and (**B**) VH (SEQ ID NOs:5 and 13, respectively); Eph099B-233.152 (**C**) VL (SEQ ID NOs.:17 and 25, respectively) and (**D**) VH (SEQ ID NOs:21 and 29, respectively); and EA2 (**E**) VL (SEQ ID NOs:33 and 41, respectively) and (**F**) VH (SEQ ID NOs:37 and 45, respectively). Sequences of the CDRs are indicated.

## 5. DETAILED DESCRIPTION OF THE INVENTION

The present invention is based, in part, on the inventors' discovery that EphA2 monoclonal antibodies can inhibit cancer cell proliferation and invasiveness by reducing the levels of EphA2 expression in these cancer cells. Decreased EphA2 activity selectively inhibits malignant cancer cell growth. In particular, such decreased levels of EphA2 can be achieved with EphA2 agonistic monoclonal antibodies. Although not intending to be bound by any mechanism of action, this inhibition of cell growth and/or metastasis is achieved by stimulating *(i.e.,* agonizing) EphA2 signaling thereby causing EphA2 phosphorylation which leads to the degradation of EphA2. Cancer cell growth is decreased due to the decreased EphA2 levels and, therefore, the decreased ligand-independent EphA2 signaling. Decreased EphA2 activity may also be achieved with EphA2 cancer cell phenotype inhibiting antibodies or antibodies that preferentially bind an EphA2 epitope exposed on cancer cells but not non-cancer cells. Additionally, antibodies that bind EphA2 with a low $K_{off}$ (*e.g*., less than less than 3 X $10^{-3}$ $s^{-1}$) can also decrease EphA2 levels.

Accordingly, the present invention relates to antibodies and compositions that provide for the treatment, inhibition, and management of diseases and disorders associated with overexpression of EphA2 and/or cell hyperproliferative diseases and disorders. A particular aspect of the invention relates to antibodies and compositions containing compounds that inhibit cancer cell proliferation and invasion, particularly those cancer cells that overexpress EphA2. The present invention further relates to antibodies and compositions for the treatment, inhibition, or management of metastases of cancers of epithelial cell origin, especially human cancers of the breast, lung, skin, prostate, bladder, and pancreas, and renal cell carcinomas and melanomas. Further compositions and antibodies of the invention include other types of active ingredients in combination with the EphA2 antibodies of the invention. In other embodiments, the antibodies of the invention are used to treat, prevent or manage other diseases or disorders associated with cell hyperproliferation, for example but not limited to asthma, psoriasis, restenosis, COPD, etc.

The present invention also relates to antibodies for the treatment, inhibition, and management of cancer or other hyperproliferative cell disorder or disease that has become partially or completely refractory to current or standard cancer treatment, such as chemotherapy, radiation therapy, hormonal therapy, and biological therapy.

The invention further provides diagnostic uses using the EphA2 antibodies of the invention, particularly the exposed EphA2 epitope antibodies, to evaluate the efficacy of cancer treatment, either EphA2-based or not EphA2-based. The diagnostic uses of the invention can also be used to prognose or predict cancer progression. In particular embodiments, the diagnostic uses of the invention provide uses for imaging and localizing metastases and uses for diagnosis and prognosis using tissues and fluids distal to the primary tumor site (as well as methods using tissues and fluids of the primary tumor). In other embodiments, the diagnostic uses of the invention provide uses for imagine and localizing metastases and methods of diagnosis and prognosis in

In an additional area, the invention describes methods of screening for anti-cancer agents, particularly anti-metastatic cancer agents, by screening agents for the ability to decrease cell colonization in soft agar and/or tubular network formation in three-dimensional basement membrane and extracellular matrix preparations, such as MATRIGEL™. In other areas, the invention describes methods of screening for agents for the treatment and prevention of hyperproliferative diseases and disorders by assaying for the ability to reduce the extent of existing cell colonization in soft agar and/or tubular network formation in three-dimensional basement membrane. The present inventors found that inhibition of cell colonization in soft agar and/or tubular network formation in MATRIGEL™ is a far better indication of antimetastatic activity and may identify potential anti-metastatic agents that would not have been identified by standard cell culture assays.

### 5.1 Antibodies

As discussed above, the invention encompasses administration of antibodies (preferably monoclonal antibodies) or fragments thereof that immunospecifically bind to and agonize EphA2 signaling ("EphA2 agonistic antibodies"); inhibit a cancer cell phenotype, *e.g.*, inhibit colony formation in soft agar or tubular network formation in a three-dimensional basement membrane or extracellular matrix preparation, such as MATRIGEL™ ("cancer cell phenotype inhibiting antibodies"), preferentially bind epitopes on EphA2 that are selectively exposed or increased on cancer cells but not non-cancer cells("exposed EphA2 epitope antibodies"); and bind EphA2 with a $K_{off}$ of less than 3 X $10^{-3}$ $s^{-1}$. In one embodiment, the antibody binds to the extracellular domain of EphA2 and, agonizes EphA2, *e.g.*, increases EphA2 phosphorylation and, preferably, causes EphA2 degradation In another embodiment, the antibody binds to the extracellular domain of EphA2 and, preferably, also inhibits and, even more preferably, reduces the extent of (*e.g.*, by cell killing mechanisms such as necrosis and apoptosis) colony formation in soft agar or tubular network formation in a three-dimensional basement membrane or extracellular matrix preparation. In other embodiments, the antibodies inhibit or reduce a cancer cell phenotype in the presence of another anti-cancer agent, such as a hormonal, biologic, chemotherapeutic or other agent. In another embodiment, the antibody binds to the extracellular domain of EphA2 at an epitope that is exposed in a cancer cell but occluded in a non-cancer cell. In a specific embodiment, the antibody is not EA2. In another embodiment, the antibody binds to the extracellular domain of EphA2, with a $K_{off}$ of less than 3 X $10^{-3}$ $s^{-1}$, more preferably less than 1 X $10^{-3}$ $s^{-1}$. In other embodiments, the antibody binds to EphA2 with a $K_{off}$ of less than 5 X $10^{-3}$ $s^{-1}$, less than $10^{-3}s^{-1}$, less than 8 X $10^{-4}s^{-1}$, less than 5 X $10^{-4}$ $s^{-1}$, less than $10^{-4}$ $s^{-1}$, less than 9 X $10^{-5}$ $s^{-1}$, less than 5 X $10^{-5}$ $s^{-1}$, less than $10^{-5}$ $s^{-1}$, less than 5 X $10^{-6}$ $s^{-1}$, less than $10^{-6}$ $s^{-1}$, less than 5 X $10^{-7}$ $s^{-1}$, less than $10^{-7}$ $s^{-1}$, less than 5 X $10^{-8}$ $s^{-1}$, less than $10^{-8}$ $s^{-1}$, less than 5 X $10^{-9}$ $s^{-1}$, less than $10^{-9}$ $s^{-1}$, or less than 10-$^{10}$ $s^{-1}$.

In a more preferred embodiment, the antibody is Eph099B-102.147, Eph099B-208.261, Eph099B-210.248, Eph099B-233.152. In another embodiment, the antibody binds to an epitope bound by Eph099B-102.147, Eph099B-208.261, Eph099B-210.248, Eph099B-233.152 and/or competes for EphA2 binding with Eph099B-102.147, Eph099B-208.261, Eph099B-210.248, Eph099B-233.152, *e.g.* as assayed by ELISA or any other appropriate immunoassay. In other embodiments, the antibody of the invention immunospecifically binds to and agonizes EphA2 signaling, inhibits a cancer cell phenotype, preferentially binds an epitope on EphA2 that is selectively exposed or increased on cancer cells but not non-cancer cells, and has a $K_{off}$ of less than 3 X $10^{-3}$ $s^{-1}$ and may or may not compete for binding with an EphA2 ligand, *e.g.*, Ephrin A1.

Hybridomas producing Eph099B-102.147, Eph099B-208.261, and Eph099B-210.248 have been deposited with the American Type Culture Collection (ATCC, P.O. Box 1549, Manassas, VA 20108) on August 7, 2002 under the provisions of the Budapest Treaty on the International Recognition of the Deposit of Microorganisms for the Purposes of Patent Procedures, and assigned accession numbers PTA-4572, PTA-4573, and PTA-4574, respectively. A hybridoma producing Eph099B-233.152 has been deposited with the American Type Culture Collection (ATCC, P.O. Box 1549, Manassas, VA 20108) on May 12, 2003 under the provisions of the Budapest Treaty on the International Recognition of the Deposit of Microorganisms for the Purposes of Patent Procedures, and assigned accession number 5194. The amino acid and nucleic acid sequences of VL and VH of Eph099B-208.261 and Eph099B-233.152 are shown in FIGS. 19A-19D. The sequences of the Eph099B-208.261 and Eph099B-233.152 CDRs are indicated in Table 1. In a most preferred embodiment, the antibody is human or has been humanized.

Antibodies of the invention include, but are not limited to, monoclonal antibodies, synthetic antibodies, recombinantly produced antibodies, intrabodies, multispecific antibodies (including bi-specific antibodies), human antibodies, humanized antibodies, chimeric antibodies, single-chain Fvs (scFv) (including bi-specific scFvs), single chain antibodies, Fab fragments, F(ab') fragments, disulfide-linked Fvs (sdFv), and epitope-binding fragments of any of the above. In particular, antibodies used in the methods of the present invention include immunoglobulin molecules and immunologically active portions of immunoglobulin molecules, *i.e.*, molecules that contain an antigen binding site that immunospecifically binds to EphA2 and is an agonist of EphA2, inhibits or reduces a cancer cell phenotype, preferentially binds an EphA2 epitope exposed on cancer cells but not non-cancer cells, and binds EphA2 with a $K_{off}$ of less than 3 X $10^{-3}$ $s^{-1}$. The immunoglobulin molecules of the invention can be of any type (*e.g.*, IgG, IgE, IgM, IgD, IgA and IgY), class (*e.g.*, $IgG_1$, $IgG_2$, $IgG_3$, $IgG_4$, $IgA_1$ and $IgA_2$) or subclass of immunoglobulin molecule.

The antibodies used in the methods of the invention may be from any animal origin including birds and mammals (*e.g.*, human, murine, donkey, sheep, rabbit, goat, guinea pig, camel, horse, or chicken). Preferably, the antibodies are human or humanized monoclonal antibodies. As used herein, "human" antibodies include antibodies having the amino acid sequence of a human immunoglobulin and include antibodies isolated from human immunoglobulin libraries or from mice or other animals that express antibodies from human genes.

The antibodies used in the methods of the present invention may be monospecific, bispecific, trispecific or of greater multispecificity. Multispecific antibodies may immunospecifically bind to different epitopes of an EphA2 polypeptide or may immunospecifically bind to both an EphA2 polypeptide as well a heterologous epitope, such as a heterologous polypeptide or solid support material. See, *e.g.*, International Publication Nos. WO 93/17715, WO 92/08802, WO 91/00360, and WO 92/05793; Tutt, et al., 1991, J. Immunol. 147:60-69; U.S. Patent Nos. 4,474,893, 4,714,681, 4,925,648, 5,573,920, and 5,601,819; and Kostelny et al.,1992, J. Immunol. 148:1547-1553.

In a specific embodiment, an antibody used in the present invention is Eph099B-102.147, Eph099B-208.261, Eph099B-210.248, Eph099B-233.152, or an antigen-binding fragment thereof (*e.g.*, one or more complementarity determining regions (CDRs) of the afore-mentioned antibodies of the invention; *e.g.*, see Table 1)). In another embodiment, an agonistic antibody used in the methods of the present invention binds to the same epitope as Eph099B-102.147, Eph099B-208.261, Eph099B-210.248, Eph099B-233.152, or competes with Eph099B-102.147, Eph099B-208.261, Eph099B-210.248, Eph099B-233.152, for binding to EphA2, *e.g.*, in an ELISA assay.

The present invention also encompasses antibodies or fragments thereof that immunospecifically bind to EphA2 and agonize EphA2, inhibit a cancer cell phenotype, preferentially bind an EphA2 epitope exposed in cancer cells, and bind EphA2 with a $K_{off}$ of less than 3 X 10-3 $s^{-1}$, said antibodies comprising a VH CDR having an amino acid sequence of any one of the VH CDRs of Eph099B-102.147, Eph099B-208.261, Eph099B-210.248, Eph099B-233.152. The present invention also encompasses the use of antibodies that immunospecifically bind to EphA2 and agonize EphA2, inhibit a cancer cell phenotype, preferentially bind an EphA2 epitope exposed in cancer cells, and bind EphA2 with a $K_{off}$ of less than 3 X 10-3 $s^{-1}$, said antibodies comprising a VL CDR having an amino acid sequence of any one of the VL CDRs of Eph099B-102.147, Eph099B-208.261, Eph099B-210.248, Eph099B-233.152.
The present invention also encompasses the use of antibodies that immunospecifically bind to EphA2 and agonize EphA2, inhibit a cancer cell phenotype, preferentially bind an EphA2 epitope exposed in cancer cells, and bind EphA2 with a $K_{off}$ of less than 3 X 10-3 $s^{-1}$, said antibodies comprising one or more VH CDRs and one or more VL CDRs of Eph099B-102.147, Eph099B-208.261, Eph099B-210.248, Eph099B-233.152. In particular, the invention encompasses the use of antibodies that immunospecifically bind to EphA2 and agonize EphA2, inhibit a cancer cell phenotype, preferentially bind an EphA2 epitope exposed in cancer cells, and bind EphA2 with a $K_{off}$ of less than 3 X 10-3 $s^{-1}$, said antibodies comprising a VH CDR1 and a VL CDR1; a VH CDR1 and a VL CDR2; a VH CDR1 and a VL CDR3; a VH CDR2 and a VL CDR1; VH CDR2 and VL CDR2; a VH CDR2 and a VL CDR3; a VH CDR3 and a VL CDR1; a VH CDR3 and a VL CDR2; a VH CDR3 and a VL CDR3; a VH1 CDR1, a VH CDR2 and a VL CDR1; a VH CDR1, a VH CDR2 and a VL CDR2; a VH CDR1, a VH CDR2 and a VL CDR3; a VH CDR2, a VH CDR3 and a VL CDR1, a VH CDR2, a VH CDR3 and a VL CDR2; a VH CDR2, a VH CDR3 and a VL CDR3; a VH1 CDR1, a VH CDR3 and a VL CDR1; a VH CDR1, a VH CDR3 and a VL CDR2; a VH CDR1, a VH CDR3 and a VL CDR3; a VH CDR1, a VL CDR1 and a VL CDR2; a VH CDR1, a VL CDR1 and a VL CDR3; a VH CDR1, a VL CDR2 and a VL CDR3; a VH CDR2, a VL CDR1 and a VL CDR2; a VH CDR2, a VL CDR1 and a VL CDR3; a VH CDR2, a VL CDR2 and a VL CDR3; a VH CDR3, a VL CDR1 and a VL CDR2; a VH CDR3, a VL CDR1 and a VL CDR3; a VH CDR3, a VL CDR2 and a VL CDR3; a VH CDR1, a VH CDR2, a VH CDR3 and a VL CDR1; a VH CDR1, a VH CDR2, a VH CDR3 and a VL CDR2; a VH CDR1, a VH CDR2, a VH CDR3 and a VL CDR3; a VH CDR1, a VL CDR1, a VL CDR2 and a VL CDR3; a VH CDR2, a VL CDR1, a VL CDR2 and a VL CDR3; a VH CDR3, a VL CDR1, a VL CDR2 and a VL CDR3; a VH CDR1, a VH CDR2, a VL CDR1 and a VL CDR2; a VH CDR1, a VH CDR2, a VL CDR1 and a VL CDR3; a VH CDR1, a VH CDR2, a VL CDR2 and a VL CDR3; a VH CDR1, a VH CDR3, a VL CDR1 and a VL CDR2; a VH CDR1, a VH CDR3, a VL CDR1 and a VL CDR3; ; a VH CDR1, a VH CDR3, a VL CDR2 and a VL CDR3; a VH CDR2, a VH CDR3, a VL CDR1 and a VL CDR2; a VH CDR2, a VH CDR3, a VL CDR1 and a VL CDR3; a VH CDR2, a VH CDR3, a VL CDR2 and a VL CDR3; a VH CDR1, a VH CDR2, a VH CDR3, a VL CDR1 and a VL CDR2; a VH CDR1, a VH CDR2, a VH CDR3, a VL CDR1 and a VL CDR3; a VH CDR1, a VH CDR2, a VH CDR3, a VL CDR2 and a VL CDR3; a VH CDR1, a VH CDR2, a VL CDR1, a VL CDR2, and a VL CDR3; a VH CDR1, a VH CDR3, a VL CDR1, a VL CDR2, and a VL CDR3; a VH CDR2, a VH CDR3, a VL CDR1, a VL CDR2, and a VL CDR3; a VH CDR1, a VH CDR2, a VH CDR3, a VL CDR1, a VL CDR2, and a VL CDR3 or any combination thereof of the VH CDRs and VL CDRs of Eph099B-102.147, Eph099B-208.261, Eph099B-210.248, Eph099B-233.152. In specific embodiments, the VH CDR1 is SEQ ID NO:6 or 22; the VH CDR2 is SEQ ID NO:7 or 23; the VH CDR3 is SEQ ID NO:8 or 24; the VL CDR1 is SEQ ID NO:2 or 18; the VL CDR2 is SEQ ID NO:3 or 19; and the VL CDR3 is SEQ ID NO:4 or 20 (see, *e.g.,* Table 1). In a more specific embodiment, the VH CDR1 is SEQ ID NO:6; the VH CDR2 is SEQ ID NO:7; the VH CDR3 is SEQ ID NO:8; the VL CDR1 is SEQ ID NO:2; the VL CDR2 is SEQ ID NO:3; and the VL CDR3 is SEQ ID NO:4. In another more specific embodiment, the VH CDR1 is SEQ ID NO:22; the VH CDR2 is SEQ ID NO:23; the VH CDR3 is SEQ ID NO:24; the VL CDR1 is SEQ ID NO:18; the VL CDR2 is SEQ ID NO: 19; and the VL CDR3 is SEQ ID NO:20. The invention also encompasses any of the foregoing with one, two, or, three, amino acid substitutions, additions, or deletions that bind EphA2.

In one embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR1 having the amino acid sequence of SEQ ID NO:6 and a VL CDR1 having the amino acid sequence of SEQ ID NO:2. In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR1 having the amino acid sequence of SEQ ID NO:6 and a VL CDR2 having the amino acid sequence of SEQ ID NO:3. In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR1 having the amino acid sequence of SEQ ID NO:6 and a VL CDR3 having the amino acid sequence of SEQ ID NO:4.

In another embodiment, an antibody that immunospecifically binds to EphA2 and agonizes EphA2, inhibits a cancer cell phenotype, preferentially binds an EphA2 epitope exposed in cancer cells, and binds EphA22 with a $K_{off}$ of less than 3 X 10-3 $s^{-1}$ comprises a VH CDR1 having the amino acid sequence of SEQ ID NO:22 and a VL CDR1 having the amino acid sequence of SEQ ID NO: 18. In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR1 having the amino acid sequence of SEQ ID NO:22 and a VL CDR2 having the amino acid sequence of SEQ ID NO: 19. In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR1 having the amino acid sequence of SEQ ID NO:22 and a VL CDR3 having the amino acid sequence of SEQ ID NO:20.

In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR2 having the amino acid sequence of SEQ ID NO:7 and a VL CDR1 having the amino acid sequence of SEQ ID NO:2. In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR2 having the amino acid sequence of SEQ ID NO:7 and a VL CDR2 having the amino acid sequence of SEQ ID NO:3. In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR2 having the amino acid sequence of SEQ ID NO:7 and a VL CDR3 having the amino acid sequence of SEQ ID NO:4.

In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR2 having the amino acid sequence of SEQ ID NO:23 and a VL CDR1 having the amino acid sequence of SEQ ID NO: 18. In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR2 having the amino acid sequence of SEQ ID NO:23 and a VL CDR2 having the amino acid sequence of SEQ ID NO: 19. In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR2 having the amino acid sequence of SEQ ID NO:23 and a VL CDR3 having the amino acid sequence of SEQ ID NO:20.

In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR3 having the amino acid sequence of SEQ ID NO:8 and a VL CDR1 having the amino acid sequence of SEQ ID NO:2. In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR3 having the amino acid sequence of SEQ ID NO:8 and a VL CDR2 having the amino acid sequence of SEQ ID NO:3. In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR3 having the amino acid sequence of SEQ ID NO:8 and a VL CDR3 having the amino acid sequence of SEQ ID NO:4.

In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR3 having the amino acid sequence of SEQ ID NO:24 and a VL CDR1 having the amino acid sequence of SEQ ID NO:18. In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR3 having the amino acid sequence of SEQ ID NO:24 and a VL CDR2 having the amino acid sequence of SEQ ID NO: 19. In another embodiment, an antibody that immunospecifically binds to EphA2 and agonizes EphA2, inhibits a cancer cell phenotype, preferentially binds an EphA2 epitope exposed in cancer cells, and binds EphA2 with a $K_{off}$ of less than 3 X 10-3 $s^{-1}$ comprises a VH CDR3 having the amino acid sequence of SEQ ID NO:24 and a VL CDR3 having the amino acid sequence of SEQ ID NO:20.

The antibodies used in the methods of the invention include derivatives that are modified, i. e, by the covalent attachment of any type of molecule to the antibody. For example, but not by way of limitation, the antibody derivatives include antibodies that have been modified, *e.g.,* by glycosylation, acetylation, pegylation, phosphorylation, amidation, derivatization by known protecting/blocking groups, proteolytic cleavage, linkage to a cellular ligand or other protein, etc. Any of numerous chemical modifications may be carried out by known techniques, including, but not limited to, specific chemical cleavage, acetylation, formylation, metabolic synthesis of tunicamycin, etc. Additionally, the derivative may contain one or more non-classical amino acids.

The present invention also provides antibodies of the invention or fragments thereof that comprise a framework region known to those of skill in the art. Preferably, the antibody of the invention or fragment thereof is human or humanized. In a specific embodiment, the antibody of the invention or fragment thereof comprises one or more CDRs from Eph099B-102.147, Eph099B-208.261, Eph099B-210.248, Eph099B-233.152, (or any other EphA2 agonistic antibody or EphA2 cancer cell phenotype inhibiting antibody or an EphA2 antibody that binds EphA2 with a $K_{off}$ of less than 3 X $10^{-3}$ $s^{-1}$), binds EphA2, and, agonizes EphA2 and/or inhibits a cancer cell phenotype and binds EphA2 with a $K_{off}$ of less than 3 X $10^{-3}$ $s^{-1}$.

The present invention encompasses single domain antibodies, including camelized single domain antibodies (see *e.g.*, Muyldermans et al., 2001, Trends Biochem. Sci. 26:230; Nuttall et al., 2000, Cur. Pharm. Biotech. 1:253; Reichmann and Muyldermans, 1999, J. Immunol. Meth. 231:25; International Publication Nos. WO 94/04678 and WO 94/25591; U.S. Patent No. 6,005,079. In one embodiment, the present invention provides single domain antibodies comprising two VH domains having the amino acid sequence of any of the VH domains of Eph099B-102.147, Eph099B-208.261, Eph099B-210.248, Eph099B-233.152, (or any other EphA2 agonistic antibody, EphA2 cancer cell phenotype inhibiting antibody, exposed EphA2 epitope antibody, and an EphA2 antibody that binds EphA2 with a $K_{off}$ of less than 3 X $10^{-3}$ $s^{-1}$) with modifications such that single domain antibodies are formed. In another embodiment, the present invention also provides single domain antibodies comprising two VH domains comprising one or more of the VH CDRs of Eph099B-102.147, Eph099B-208.261, Eph099B-210.248, Eph099B-233.152, (or any other EphA2 agonistic antibody, phA2 cancer cell phenotype inhibiting antibody, exposed EphA2 epitope antibody, and an EphA2 antibody that binds EphA2 with a $K_{off}$ of less than 3 X $10^{-3}$ $s^{-1}$).

The methods of the present invention also encompass the use of antibodies or fragments thereof that have half-lives (*e.g.*, serum half-lives) in a mammal, preferably a human, of greater than 15 days, preferably greater than 20 days, greater than 25 days, greater than 30 days, greater than 35 days, greater than 40 days, greater than 45 days, greater than 2 months, greater than 3 months, greater than 4 months, or greater than 5 months. The increased half-lives of the antibodies of the present invention or fragments thereof in a mammal, preferably a human, result in a higher serum titer of said antibodies or antibody fragments in the mammal, and thus, reduce the frequency of the administration of said antibodies or antibody fragments and/or reduces the concentration of said antibodies or antibody fragments to be administered. Antibodies or fragments thereof having increased *in vivo* half-lives can be generated by techniques known to those of skill in the art. For example, antibodies or fragments thereof with increased *in vivo* half-lives can be generated by modifying (*e.g.*, substituting, deleting or adding) amino acid residues identified as involved in the interaction between the Fc domain and the FcRn receptor (see, *e.g.*, International Publication Nos. WO 97/34631 and WO 02/060919). Antibodies or fragments thereof with increased in vivo half-lives can be generated by attaching to said antibodies or antibody fragments polymer molecules such as high molecular weight polyethyleneglycol (PEG). PEG can be attached to said antibodies or antibody fragments with or without a multifunctional linker either through site-specific conjugation of the PEG to the N- or C- terminus of said antibodies or antibody fragments or via epsilon-amino groups present on lysine residues. Linear or branched polymer derivatization that results in minimal loss of biological activity will be used. The degree of conjugation will be closely monitored by SDS-PAGE and mass spectrometry to ensure proper conjugation of PEG molecules to the antibodies. Unreacted PEG can be separated from antibody-PEG conjugates by, *e.g.*, size exclusion or ionexchange chromatography.

The present invention also encompasses the use of antibodies or antibody fragments comprising the amino acid sequence of one or both variable domains of Eph099B-102.147, Eph099B-208.261, Eph099B-210.248, Eph099B-233.152, (*e.g.*, one or more amino acid substitutions) in the variable regions. Preferably, mutations in these or more amino acid substitutions) in the variable regions. Preferably, mutations in these antibodies maintain or enhance the avidity and/or affinity of the antibodies for the particular antigen(s) to which they immunospecifically bind. Standard techniques known to those skilled in the art (*e.g.*, immunoassays) can be used to assay the affinity of an antibody for a particular antigen.

Standard techniques known to those skilled in the art can be used to introduce mutations in the nucleotide sequence encoding an antibody, or fragment thereof, including, *e.g.*, site-directed mutagenesis and PCR-mediated mutagenesis, which results in amino acid substitutions. Preferably, the derivatives include less than 15 amino acid substitutions, less than 10 amino acid substitutions, less than 5 amino acid substitutions, less than 4 amino acid substitutions, less than 3 amino acid substitutions, or less than 2 amino acid substitutions relative to the original antibody or fragment thereof. In a preferred embodiment, the derivatives have conservative amino acid substitutions made at one or more predicted non-essential amino acid residues.

The present invention also encompasses antibodies or fragments thereof that immunospecifically bind to EphA2 and agonize EphA2 and/or inhibit a cancer cell phenotype, preferentially bind an EphA2 epitope exposed in cancer cells, and bind EphA2 with a $K_{off}$ of less than 3 X $10^{-3}$ $s^{-1}$, said antibodies or antibody fragments comprising an amino acid sequence of a variable light chain and/or variable heavy chain that is at least 45%, at least 50%, at least 55%, at least 60%, at least 65%, at least 70%, at least 75%, at least 80%, at least 85%, at least 90%, at least 95%, or at least 99% identical to the amino acid sequence of the variable light chain and/or heavy chain of Eph099B-102.147, Eph099B-208.261, Eph099B-210.248, Eph099B-233.152.

In some embodiments, antibodies or antibody fragments of the invention immunospecifically bind to EphA2 and comprise an amino acid sequence of a variable light chain that is at least 45%, at least 50%, at least 55%, at least 60%, at least 65%, at least 70%, at least 75%, at least 80%, at least 85%, at least 90%, at least 95%, or at least 99% identical to SEQ ID NO: 1 or 17. In other embodiments, antibodies or antibody fragments of the invention immunospecifically bind to EphA2 and comprise an amino acid sequence of a variable heavy chain that is at least 45%, at least 50%, at least 55%, at least 60%, at least 65%, at least 70%, at least 75%, at least 80%, at least 85%, at least 90%, at least 95%, or at least 99% identical to SEQ ID NO:5 or 21. In other embodiments, antibodies or antibody fragments of the invention immunospecifically bind to EphA2 and comprise an amino acid sequence of a variable light chain that is at least 45%, at least 50%, at least 55%, at least 60%, at least 65%, at least 70%, at least 75%, at least 80%, at least 85%, at least 90%, at least 95%, or at least 99% identical to SEQ ID NO:1 or 17 and a variable heavy chain that is at least 45%, at least 50%, at least 55%, at least 60%, at least 65%, at least 70%, at least 75%, at least 80%, at least 85%, at least 90%, at least 95%, or at least 99% identical to SEQ ID NO: 5 or 21.

The present invention further encompasses antibodies or fragments thereof that immunospecifically bind to EphA2 and agonize EphA2 and/or inhibit a cancer cell phenotype, preferentially bind an EphA2 epitope exposed in cancer cells, and bind EphA2 with a $K_{off}$ of less than 3 X $10^{-3}$ $s^{-1}$, said antibodies or antibody fragments comprising an amino acid sequence of one or more CDRs that is at least 45%, at least 50%, at least 55%, at least 60%, at least 65%, at least 70%, at least 75%, at least 80%, at least 85%, at least 90%, at least 95%, or at least 99% identical to the amino acid sequence of one or more CDRs of Eph099B-102.147, Eph099B-208.261, Eph099B-210.248, Eph099B-233.152.

In one embodiment, antibodies or antibody fragments of the invention immunospecifically bind to EphA2 and comprise an amino acid sequence of a CDR that is at least 45%, at least 50%, at least 55%, at least 60%, at least 65%, at least 70%, at least 75%, at least 80%, at least 85%, at least 90%, at least 95%, or at least 99% identical to SEQ ID NO:2, 3, or 4. In another embodiment, antibodies or antibody fragments of the invention immunospecifically bind to EphA2 and comprise an amino acid sequence of a CDR that is at least 45%, at least 50%, at least 55%, at least 60%, at least 65%, at least 70%, at least 75%, at least 80%, at least 85%, at least 90%, at least 95%, or at least 99% identical to SEQ ID NO:18, 19, or 20. In another embodiment, antibodies or antibody fragments of the invention immunospecifically bind to EphA2 and comprise an amino acid sequence of a CDR that is at least 45%, at least 50%, at least 55%, at least 60%, at least 65%, at least 70%, at least 75%, at least 80%, at least 85%, at least 90%, at least 95%, or at least 99% identical to SEQ ID NO:6, 7, or 8. In another embodiment, antibodies or antibody fragments of the invention immunospecifically bind to EphA2 and comprise an amino acid sequence of a CDR that is at least 45%, at least 50%, at least 55%, at least 60%, at least 65%, at least 70%, at least 75%, at least 80%, at least 85%, at least 90%, at least 95%, or at least 99% identical to SEQ ID NO:22, 23, or 24.

The determination of percent identity of two amino acid sequences can be determined by any method known to one skilled in the art, including BLAST protein searches.

The present invention further encompasses antibodies or fragments thereof that immunospecifically bind to EphA2 and agonize EphA2 and/or inhibit a cancer cell phenotype, preferentially bind an EphA2 epitope exposed in cancer cells, and bind EphA2 with a $K_{off}$ of less than 3 X $10^{-3}$ $s^{-1}$, said antibodies or antibody fragments comprising an amino acid sequence of one or more CDRs comprising amino acid residue substitutions, deletions or additions as compared to SEQ ID NO: 2, 3, 4, 6, 7, 8,18, 19, 20, 22, 23, or 24. The antibody comprising the one or more CDRs comprising amino acid residue substitutions, deletions or additions may have substantially the same binding, better binding, or worse binding when compared to an antibody comprising one or more CDRs without amino acid residue substitutions, deletions or additions. In specific embodiments, one, two, three, four, or five amino acid residues of the CDR have been substituted, deleted or added (i.e., mutated).

The present invention also encompasses the use of antibodies or antibody fragments that immunospecifically bind to EphA2 and agonize EphA2 and optionally inhibit a cancer cell phenotype, preferentially bind epitopes on EphA2 that are selectively exposed or increased on cancer cells but not non-cancer cells and bind EphA2 with a $K_{off}$ less than 3 X $10^{-3}$ $s^{-1}$, where said antibodies or antibody fragments are encoded by a nucleotide sequence that hybridizes to the nucleotide sequence of Eph099B-102.147, Eph099B-208.261, Eph099B-210.248, Eph099B-233.152, under stringent conditions. In one embodiment, the invention provides antibodies or fragments thereof that immunospecifically bind to EphA2 and agonize EphA2 and optionally inhibit a cancer cell phenotype, preferentially bind an epitope on EphA2 that is selectively exposed or increased on cancer cells but not non-cancer cells and bind EphA2 with a $K_{off}$ less than 3 X $10^{-3}$ $s^{-1}$, said antibodies or antibody fragments comprising a variable light chain encoded by a nucleotide sequence that hybridizes under stringent conditions to the nucleotide sequence of the variable light chain of Eph099B-102.147, Eph099B-208.261, Eph099B-210.248, Eph099B-233.152. In a preferred embodiment, the invention provides antibodies or fragments that immunospecifically bind to EphA2 and comprise a variable light chain encoded by a nucleotide sequence that hybridizes under stringent conditions to the nucleotide sequence of SEQ ID NO:9 or 25. In another embodiment, the invention provides antibodies or fragments thereof that immunospecifically bind to EphA2 and agonize EphA2 and optionally inhibit a cancer cell phenotype, preferentially bind an epitope on EphA2 that is selectively exposed or increased on cancer cells but not non-cancer cells and bind EphA2 with a $K_{off}$ less than 3 X $10^{-3}$ $s^{-1}$, said antibodies or antibody fragments comprising a variable heavy chain encoded by a nucleotide sequence that hybridizes under stringent conditions to the nucleotide sequence of the variable heavy chain of Eph099B-102.147, Eph099B-208.261, Eph099B-210.248, Eph099B-233.152. In a preferred embodiment, the invention provides antibodies or fragments thereof that immunospecifically bind to EphA2 and comprise a variable heavy chain encoded by a nucleotide sequence that hybridizes under stringent conditions to the nucleotide sequence of SEQ ID NO:13 or 29. In other embodiments, antibodies or antibody fragments of the invention immunospecifically bind to EphA2 and comprise a variable light chain encoded by a nucleotide sequence that hybridizes under stringent conditions to the nucleotide sequence of SEQ ID NO:9 or 25 and a variable heavy chain encoded by a nucleotide sequence that hybridizes under stringent conditions to the nucleotide sequence of SEQ ID NO: 13 or 29.

In another embodiment, the invention provides antibodies or fragments thereof that immunospecifically bind to EphA2 and agonize EphA2 and/or inhibit a cancer cell phenotype, preferentially bind an EphA2 epitope exposed on cancer cells but not non-cancer cells and/or bind EphA2 with a $K_{off}$ less than 3 X $10^{-3}$ $s^{-1}$, said antibodies or antibody fragments comprising one or more CDRs encoded by a nucleotide sequence that hybridizes under stringent conditions to the nucleotide sequence of one or more CDRs of Eph099B-102.147, Eph099B-208.261, Eph099B-210.248, Eph099B-233.152.

In a preferred embodiment, the antibodies or fragments of the invention immunospecifically bind to EphA2 and comprise a CDR encoded by a nucleotide sequence that hybridizes under stringent conditions the nucleotide sequence of SEQ ID NO: 10, 11, or 12. In another preferred embodiment, the antibodies or fragments of the invention immunospecifically bind to EphA2 and comprise a CDR encoded by a nucleotide sequence that hybridizes under stringent conditions the nucleotide sequence of SEQ ID NO:26, 27, or 28. In another preferred embodiment, the antibodies or fragments of the invention immunospecifically bind to EphA2 and comprise a CDR encoded by a nucleotide sequence that hybridizes under stringent conditions the nucleotide sequence of SEQ ID NO:14, 15, or 16. In another preferred embodiment, the antibodies or fragments of the invention immunospecifically bind to EphA2 and comprise a CDR encoded by a nucleotide sequence that hybridizes under stringent conditions the nucleotide sequence of SEQ ID NO:30, 31, or 32.

Stringent hybridization conditions include, but are not limited to, hybridization to filter-bound DNA in 6X sodium chloride/sodium citrate (SSC) at about 45°C followed by one or more washes in 0.2X SSC/0.1% SDS at about 50-65°C, highly stringent conditions such as hybridization to filter-bound DNA in 6X SSC at about 45°C followed by one or more washes in 0.1X SSC/0.2% SDS at about 60°C, or any other stringent hybridization conditions known to those skilled in the art (see, for example, Ausubel, F.M. et al., eds. 1989 Current Protocols in Molecular Biology, vol. 1, Green Publishing Associates, Inc. and John Wiley and Sons, Inc., NY at pages 6.3.1 to 6.3.6 and 2.10.3).

The present invention further encompasses antibodies or fragments thereof that immunospecifically bind to EphA2 and agonize EphA2 and/or inhibit a cancer cell phenotype, preferentially bind an EphA2 epitope exposed in cancer cells, and bind EphA2 with a $K_{off}$ of less than 3 X $10^{-3}$ $s^{-1}$, said antibodies or antibody fragments said antibodies or antibody fragments comprising one or more CDRs encoded by a nucleotide sequence of one or more CDRs comprising nucleic acid residue substitutions, deletions or additions as compared to SEQ ID NO:10,11, 12,14, 15, 16, 26, 27, 28, 30, 31, or 32. The antibody comprising the one or more CDRs comprising nucleic acid residue substitutions, deletions or additions may have substantially the same binding, better binding, or worse binding when compared to an antibody comprising one or more CDRs without nucleic acid residue substitutions, deletions or additions. In specific embodiments, one, two, or three, residues of the CDR have been substituted, deleted or added *(i.e.,* mutated). The nucleic acid substitutions may or may not change the amino acid sequence of the mutated CDR

**TABLE 1**

| Antibody | V chain | CDR | SEQ ID NO. (amino acid) | SEQ ID NO. (nucleic acid) | ATCC Deposit No. |
|---|---|---|---|---|---|
| Eph099B-208.261 | | | | | PTA-4573 |

(continued)

| Antibody | V chain | CDR | SEQ ID NO. (amino acid) | SEQ ID NO. (nucleic acid) | ATCC Deposit No. |
|---|---|---|---|---|---|
| | VL | | 1 | 9 | |
| | | VL1 | 2 | 10 | |
| | | VL2 | 3 | 11 | |
| | | VL3 | 4 | 12 | |
| | VH | | 5 | 13 | |
| | | VH1 | 6 | 14 | |
| | | VH2 | 7 | 15 | |
| | | VH3 | 8 | 16 | |
| Eph099B-233.152 | | | | | PTA-5194 |
| | VL | | 17 | 25 | |
| | | VL1 | 18 | 26 | |
| | | VL2 | 19 | 27 | |
| | | VL3 | 20 | 28 | |
| | VH | | 21 | 29 | |
| | | VH1 | 22 | 30 | |
| | | VH2 | 23 | 31 | |
| | | VH3 | 24 | 32 | |
| EA2 | | | | | PTA-4380 |
| | VL | | 33 | 41 | |
| | | VL1 | 34 | 42 | |
| | | VL2 | 35 | 43 | |
| | | VL3 | 36 | 44 | |
| | VH | | 37 | 45 | |
| | | VH1 | 38 | 46 | |
| | | VH2 | 39 | 47 | |
| | | VH3 | 40 | 48 | |

### 5.1.1 Antibody conjugates

The present invention encompasses the use of antibodies or fragments thereof recombinantly fused or chemically conjugated (including both covalent and noncovalent conjugations) to a heterologous agent to generate a fusion protein. The heterologous agent may be a polypeptide (or portion thereof, preferably to a polypeptide of at least 10, at least 20, at least 30, at least 40, at least 50, at least 60, at least 70, at least 80, at least 90 or at least 100 amino acids), nucleic acid, small molecule (less than 1000 daltons), or inorganic or organic compound. The fusion does not necessarily need to be direct, but may occur through linker sequences. Antibodies fused or conjugated to heterologous agents may be used *in vivo* to detect, treat, manage, or, monitor the progression of a disorder using methods known in the art. See *e.g.*, International Publication WO 93/21232; EP 439,095; Naramura et al., 1994, Immunol. Lett. 39:91-99; U.S. Patent 5,474,981; Gillies et al., 1992, PNAS 89:1.428-1432; and Fell et al., 1991, J. Immunol. 146:2446-2452. In some embodiments, the disorder to be detected, treated, managed, or monitored is malignant cancer that overexpresses EphA2. In other embodiments, the disorder to be detected, treated, managed, or monitored is a pre-cancerous condition associated with cells that overexpress EphA2. In a specific embodiments, the pre-cancerous condition is high-grade prostatic intraepithelial neoplasia (PIN), fibroadenoma of the breast, fibrocystic disease, or compound nevi.

The present invention further includes compositions comprising heterologous agents fused or conjugated to antibody fragments. For example, the heterologous polypeptides maybe fused or conjugated to a Fab fragment, Fd fragment, Fv fragment, $F(ab)_2$ fragment, or portion thereof. Methods for fusing or conjugating polypeptides to antibody portions are known in the art. See, *e.g.*, U.S. Patent Nos. 5,336,603, 5,622,929, 5,359,046, 5,349,053, 5,447,851, and 5,112,946; EP 307,434; EP 367,166; International Publication Nos. WO 96/043 88 and WO 91/06570; Ashkenazi et al., 1991, PUNAS 88: 10535-10539; Zheng et al.,1995, J. Immunol. 154:5590-5600; and Vi1 et al., 1992, PNAS 89: 11337-11341.

Additional fusion proteins, *e.g.*, of Eph099B-102.147, Eph099B-208.261, Eph099B-210.248, Eph099B-233.152, or any of the antibodies listed in Table 6 (or any other EphA2 agonistic antibody or EphA2 cancer cell phenotype inhibiting antibody or exposed EphA2 epitope antibody or EphA2 antibody that binds EphA2 with a $K_{off}$ of less than 3 X $10^{-3}$ $s^{-1}$), maybe generated through the techniques of gene-shuffling, motif-shuffling, exon-shuffling, and/or codon-shuffling (collectively referred to as "DNA shuffling"). DNA shuffling may be employed to alter the activities of antibodies of the invention or fragments thereof (*e.g.*, antibodies or fragments thereof with higher affinities and lower dissociation rates). See, generally, U.S. Patent Nos. 5,605,793; 5,811,238; 5,830,721; 5,834,252; and 5,837,458, and Patten et al., 1997, Curr. Opinion Biotechnol. 8:724-33; Harayama, 1998, Trends Biotechnol. 16:76; Hansson, et al., 1999, J. Mol. Biol. 287:265; and Lorenzo and Blasco, 1998, BioTechniques 24:308. Antibodies or fragments thereof, or the encoded antibodies or fragments thereof, may be altered by being subjected to random mutagenesis by error-prone PCR, random nucleotide insertion or other methods prior to recombination. One or more portions of a polynucleotide encoding an antibody or antibody fragment, which portions immunospecifically bind to EphA2 may be recombined with one or more components, motifs, sections, parts, domains, fragments, etc. of one or more heterologous agents.

In one embodiment, antibodies of the present invention or fragments or variants thereof are conjugated to a marker sequence, such as a peptide, to facilitate purification. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., 1989, PNAS 86: 821, for instance, hexa-histidine provides for convenient purification of the fusion protein. Other peptide tags useful for purification include, but are not limited to, the hemagglutinin "HA" tag, which corresponds to an epitope derived from the influenza hemagglutinin protein (Wilson et al., 1984, Cell 37:767) and the "flag" tag.

In other embodiments, antibodies of the present invention or fragments or variants thereof are conjugated to a diagnostic or detectable agent. Such antibodies can be useful for monitoring or prognosing the development or progression of a cancer as part of a clinical testing procedure, such as determining the efficacy of a particular therapy. Additionally, such antibodies can be useful for monitoring or prognosing the development or progression of a pre-cancerous condition associated with cells that overexpress EphA2 (*e.g.*, high-grade prostatic intraepithelial neoplasia (PIN), fibroadenoma of the breast, fibrocystic disease ,or compound nevi). In one embodiment, an exposed EphA2 epitope antibody is conjugated to a diagnostic or detectable agent. In another embodiment, the antibody is not EA2.

Such diagnosis and detection can accomplished by coupling the antibody to detectable substances including, but not limited to various enzymes, such as but not limited to horseradish peroxidase, alkaline phosphatase, beta-galactosidase, or acetylcholinesterase; prosthetic groups, such as but not limited to streptavidin/biotin and avidin/biotin; fluorescent materials, such as but not limited to, umbelliferone, fluorescein, fluorescein isothiocynate, rhodamine, dichlorotriazinylamine fluorescein, dansyl chloride or phycoerythrin; luminescent materials, such as but not limited to, luminol; bioluminescent materials, such as but not limited to, luciferase, luciferin, and aequorin; radioactive materials, such as but not limited to, bismuth ($^{213}Bi$), carbon ($^{14}C$), chromium ($^{51}Cr$), cobalt ($^{57}Co$), fluorine ($^{18}F$) gadolinium ($^{153}Gd$, $^{159}Gd$) gallium ($^{68}Ga$, $^{67}Ga$), germanium ($^{68}Ge$), holmium ($^{166}Ho$), indium ($^{115}In$, $^{113}In$, $^{112}In$, $^{111}In$), iodine ($^{131}I$, $^{125}I$, $^{123}I$, $^{121}I$), lanthanium ($^{140}La$), lutetium ($^{177}Lu$), manganese ($^{54}Mn$), molybdenum ($^{99}Mo$), palladium ($^{103}Pd$), phosphorous ($^{32}P$), praseodymium ($^{142}Pr$), promethium ($^{149}Pm$), rhenium ($^{186}Re$, $^{188}Re$), rhodium ($^{105}Rh$), ruthemium ($^{97}Ru$), samarium ($^{153}Sm$), scandium ($^{44}Sc$), selenium ($^{75}Se$), strontium ($^{85}Sr$), sulfur ($^{35}S_)$, technetium ($^{99}Tc$), thallium ($^{201}Ti$), tin ($^{113}Sn$, $^{117}Sn$), tritium ($^{3}H$), xenon ($^{133}Xe$), ytterbium ($^{169}Yb$, $^{175}Yb$), yttrium ($^{90}Y$), zinc ($^{65}Zn$); positron emitting metals using various positron emission tomographies, and nonradioactive paramagnetic metal ions.

In other embodiments, antibodies of the present invention or fragments or variants thereof are conjugated to a therapeutic agent such as a cytotoxin, *e.g.*, a cytostatic or cytocidal agent, a therapeutic agent or a radioactive metal ion, *e.g.*, alpha-emitters. A cytotoxin or cytotoxic agent includes any agent that is detrimental to cells. Examples include paclitaxel, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, epirubicin, and cyclophosphamide and analogs or homologs thereof. Therapeutic agents include, but are not limited to, antimetabolites (*e.g.*, methotrexate, 6-mercaptopurine, 6-thioguanine, cytarabine, 5-fluorouracil decarbazine), alkylating agents (*e.g.*, mechlorethamine, thioepa chlorambucil, melphalan, carmustine (BCNU) and lomustine (CCNU), cyclothosphamide, busulfan, dibromomannitol, streptozotocin, mitomycin C, and cisdichlorodiamine platinum (II) (DDP) cisplatin), anthracyclines (*e.g.*, daunorubicin (formerly daunomycin) and doxorubicin), antibiotics (*e.g.*, dactinomycin (formerly actinomycin), bleomycin, mithramycin, and anthramycin (AMC)), and anti-mitotic agents (*e.g.*, vincristine and vinblastine).

In other embodiments, antibodies of the present invention or fragments or variants thereof are conjugated to a therapeutic agent or drug moiety that modifies a given biological response. Therapeutic agents or drug moieties are not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, cholera toxin, or diphtheria toxin; a protein such as tumor necrosis factor, α-interferon, β-interferon, nerve growth factor, platelet derived growth factor, tissue plasminogen activator, an apoptotic agent, *e.g.*, TNF-α, TNF-β, AIM I (see, International Publication No. WO 97/33899), AIM II (see, International Publication No. WO 97/34911), Fas Ligand (Takahashi et al., 1994, J. Iminunol., 6:1567), and VEGI (see, International Publication No. WO 99/23105), a thrombotic agent or an anti-angiogenic agent, *e.g.*, angiostatin or endostatin; or, a biological response modifier such as, for example, a lymphokine (*e.g.*, interleukin-1 ("IL-1"), interleukin-2 ("IL-2"), interleukin-6 ("IL-6"), granulocyte macrophage colony stimulating factor ("GM-CSF"), and granulocyte colony stimulating factor ("G-CSF")), or a growth factor (*e.g.*, growth hormone ("GH")).

In other embodiments, antibodies of the present invention or fragments or variants thereof are conjugated to a therapeutic agent such as a radioactive materials or macrocyclic chelators useful for conjugating radiometal ions (see above for examples of radioactive materials). In certain embodiments, the macrocyclic chelator is 1,4,7,10-tetraazacyclododecane-N,N',N",N"-tetraacetic acid (DOTA) which can be attached to the antibody via a linker molecule. Such linker molecules are commonly known in the art and described in Denardo et al., 1998, Clin Cancer Res. 4:2483-90; Peterson et al., 1999, Bioconjug. Chem. 10:553; and Zimmerman et al., 1999, Nucl. Med. Biol. 26:943-50.

In a specific embodiment, the conjugated antibody is an EphA2 antibody that preferably binds an EphA2 epitope exposed on cancer cells but not on non-cancer cells (*i.e.*, exposed EphA2 epitope antibody). In another specific embodiment, the conjugated antibody is not EA2.

Techniques for conjugating therapeutic moieties to antibodies are well known. Moieties can be conjugated to antibodies by any method known in the art, including, but not limited to aldehyde/Schiff linkage, sulphydryl linkage, acid-labile linkage, cis-aconityl linkage, hydrazone linkage, enzymatically degradable linkage (see generally Garnett, 2002, Adv. Drug Deliv. Rev. 53:171-216). Additional techniques for conjugating therapeutic moieties to antibodies are well known, see, *e.g.*, Amon et al., "Monoclonal Antibodies For Immunotargeting Of Drugs In Cancer Therapy," in Monoclonal Antibodies And Cancer Therapy, Reisfeld et al. (eds.), pp. 243-56 (Alan R. Liss, Inc. 1985); Hellstrom et al., "Antibodies For Drug Delivery," in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review," in Monoclonal Antibodies '84: Biological And Clinical Applications, Pinchera et al. (eds.), pp. 475-506 (1985*);* "Analysis, Results, And Future Prospective Of The Therapeutic Use Of Radiolabeled Antibody In Cancer Therapy," in Monoclonal Antibodies For Cancer Detection And Therapy, Baldwin et al. (eds.), pp. 303-16 (Academic Press 1985), and Thorpe et al., 1982, Immunol. Rev. 62:119-58. Methods for fusing or conjugating antibodies to polypeptide moieties are known in the art. See, *e.g.*, U.S. Patent Nos. 5,336,603, 5,622,929, 5,359,046,5,349,053, 5,447,851, and 5,112,946; EP 307,434; EP 367,166; International Publication Nos. WO 96/04388 and WO 91/06570; Ashkenazi et al., 1991, PNAS 88: 10535-10539; Zheng et al., 1995, J. Immunol. 154:5590-5600; and Vil et al., 1992, PNAS 89:11337-11341. The fusion of an antibody to a moiety does not necessarily need to be direct, but may occur through linker sequences. Such linker molecules are commonly known in the art and described in Denardo et al., 1998, Clin Cancer Res. 4:2483-90; Peterson et al., 1999, Bioconjug. Chem. 10: 553; Zimmerman et al., 1999, Nucl. Med. Biol. 26:943-50; Garnett, 2002, Adv. Drug Deliv. Rev. 53:171-216.

Alternatively, an antibody can be conjugated to a second antibody to form an antibody heteroconjugate as described by Segal in U.S. Patent No. 4,676,980.

Antibodies may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

### 5.1.2 Methods Of Producing Antibodies

The antibodies or fragments thereof can be produced by any method known in the art for the synthesis of antibodies, in particular, by chemical synthesis or preferably, by recombinant expression techniques.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981).

The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art. Briefly, mice can be immunized with EphA2 (either the full length protein or a domain thereof, *e.g.*, the extracellular domain or the ligand binding domain) and once an immune response is detected, *e.g.*, antibodies specific for EphA2 are detected in the mouse serum, the mouse spleen is harvested and splenocytes isolated. The splenocytes are then fused by well known techniques to any suitable myeloma cells, for example cells from cell line SP20 available from the ATCC. Hybridomas are selected and cloned by limited dilution. Hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

Accordingly, monoclonal antibodies can be generated by culturing a hybridoma cell secreting an antibody of the invention wherein, preferably, the hybridoma is generated by fusing splenocytes isolated from a mouse immunized with EphA2 or fragment thereof with myeloma cells and then screening the hybridomas resulting from the fusion for hybridoma clones that secrete an antibody able to bind EphA2.

Antibody fragments which recognize specific EphA2 epitopes maybe generated by any technique known to those of skill in the art. For example, Fab and F(ab')2 fragments of the invention may be produced by proteolytic cleavage of immunoglobulin molecules, using enzymes such as papain (to produce Fab fragments) or pepsin (to produce F(ab') 2 fragments). F(ab')2 fragments contain the variable region, the light chain constant region and the CH1 domain of the heavy chain. Further, the antibodies of the present invention can also be generated using various phage display methods known in the art.

In phage display methods, functional antibody domains are displayed on the surface of phage particles which carry the polynucleotide sequences encoding them. In particular, DNA sequences encoding VH and VL domains are amplified from animal cDNA libraries (*e.g.*, human or murine cDNA libraries of lymphoid tissues). The DNA encoding the VH and VL domains are recombined together with an scFv linker by PCR and cloned into a phagemid vector (*e.g.*, p CANTAB 6 or pComb 3 HSS). The vector is electroporated in *E. coli* and the *E. coli* is infected with helper phage. Phage used in these methods are typically filamentous phage including fd and M13 and the VH and VL domains are usually recombinantly fused to either the phage gene III or gene VIII. Phage expressing an antigen binding domain that binds to the EphA2 epitope of interest can be selected or identified with antigen, *e.g.*, using labeled antigen or antigen bound or captured to a solid surface or bead. Examples of phage display methods that can be used to make the antibodies of the present invention include those disclosed in Brinkman et al., 1995, J. Immunol. Methods 182:41-50; Ames et al., 1995, J. Immunol. Methods 184:177; Kettleborough et al., 1994, Eur. J. Immunol. 24:952-958; Persic et al., 1997, Gene 187:9; Burton et al., 1994, Advances in Immunology 57:191-280; International Application No. PCT/GB91/01134; International Publication Nos. WO 90/02809, WO 91/10737, WO 92/01047, WO 92/18619, WO 93/1 1236, WO 95/15982, WO 95/20401, and WO97/13844; and U.S. Patent Nos. 5,698,426, 5,223,409, 5,443, 484, 5,580,717, 5,427,908, 5,750,753, 5,521,047, 5,571,698, 5,427,908, 5,516,637, 5,780,225, 5,655,727, 5,733,743 and 5,969,108.

Phage may be screened for EphA2 binding, particularly to the extracellular domain of EphA2. Agonizing EphA2 activity (*e.g.*, increasing EphA2 phosphorylation, reducing EphA2 levels) or cancer cell phenotype inhibiting activity (*e.g.*, reducing colony formation in soft agar or tubular network formation in a three-dimensional basement membrane or extracellular matrix preparation, such as MATRIGEL™) or preferentially binding to an EphA2 epitope exposed on cancer cells but not non-cancer cells (*e.g.*, binding poorly to EphA2 that is bound to ligand in cell-cell contacts while binding well to EphA2 that is not bound to ligand or in cell-cell contacts) may also be screened.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, *e.g.*, as described below. Techniques to recombinantly produce Fab, Fab' and F(ab')2 fragments can also be employed using methods known in the art such as those disclosed in International Publication No. WO 92/22324; Mullinax et al., 1992, BioTechniques 12:864; Sawai et al., 1995, AJRI 34:26*;* and Better et al., 1988, Science 240:1041.

To generate whole antibodies, PCR primers including VH or VL nucleotide sequences, a restriction site, and a flanking sequence to protect the restriction site can be used to amplify the VH or VL sequences in scFv clones. Utilizing cloning techniques known to those of skill in the art, the PCR amplified VH domains can be cloned into vectors expressing a VH constant region, *e.g.*, the human gamma 4 constant region, and the PCR amplified VL domains can be cloned into vectors expressing a VL constant region, *e.g* , human kappa or lambda constant regions. Preferably, the vectors for expressing the VH or VL domains comprise an EF-1$\alpha$ promoter, a secretion signal, a cloning site for the variable domain, constant domain, and a selection marker such as neomycin. The VH and VL domains may also be cloned into one vector expressing the necessary constant regions. The heavy chain conversion vectors and light chain conversion vectors are then co-transfected into cell lines to generate stable or transient cell lines that express full-length antibodies, *e.g.*, IgG, using techniques known to those of skill in the art.

For some uses, including *in vivo* use of antibodies in humans and *in vitro* detection assays, it may be preferable to use human or chimeric antibodies. Completely human antibodies are particularly desirable for therapeutic treatment of human subjects. Human antibodies can be made by a variety of methods known in the art including page display methods described above using antibody libraries derived from human immunoglobulin sequences. See also U.S. Patent Nos. 4,444,887 and 4,716,111; and International Publication Nos. WO 98/46645, WO 98/50433, WO 98/24893, WO 98/16654, WO 96/34096, WHO 96/33735, and WO 91110741.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the $J_H$ region prevents endogenous antibody production. The modified embryonic stem cells are expanded and microinjected into blastocysts to produce chimeric mice. The chimeric mice are then be bred to produce homozygous offspring which express human antibodies. The transgenic mice are immunized in the normal fashion with a selected antigen, *e.g.*, all or a portion of a polypeptide of the invention. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13: 65-93). For a detailed discussion of this technology for producing human antibodies and human monoclonal antibodies and protocols for producing such antibodies, *see, e.g.,* International Publication Nos. WO 98/24893, WO 96/34096, and WO 96/33735; and U.S. Patent Nos. 5,413,923,5,625,126, 5,633,425, 5,569,825, 5,661,016, 5,545,806, 5,814,318, and 5,939,598.

In addition, companies such as Abgenix, Inc. (Freemont, CA) and Medarex (Princeton, NJ) can be engaged to provide human antibodies directed against a selected antigen using technology similar to that described above.

A chimeric antibody is a molecule in which different portions of the antibody are derived from different immunoglobulin molecules such as antibodies having a variable region derived from a non-human antibody and a human immunoglobulin constant region. Methods for producing chimeric antibodies are known in the art. See *e.g.*, Morrison, 1985, Science 229:1202; Oi et al., 1986, BioTechniques 4:214; Gillies et al., 1989, J. Immunol. Methods 125:191-202; and U.S. Patent Nos, 6,311,415, 5,807,715, 4,816,567, and 4,816,397. Chimeric antibodies comprising one or more CDRs from a non-human species and framework regions from a human immunoglobulin molecule can be produced using a variety of techniques known in the art including, for example, CDR-grafting (EP 239,400; International Publication No. WO 91/09967; and U.S. Patent Nos. 5,225,539, 5,530,101, and 5,585,089), veneering or resurfacing (EP 592,106; EP 519,596; Padlan, 1991, Molecular Immunology 28(4/5):489-498; Studnicka et al., 1994, Protein Engineering 7:805; and Roguska et al., 1994, PNAS 91:969), and chain shuffling (U.S. Patent No. 5,565,332). In one embodiment, a chimeric antibody of the invention immunospecifically binds EphA2 and comprises one, two, or three VL CDRs having an amino acid sequence of any of the VL CDRs of Eph099B-102.147, Eph099B-208.261, Eph0998-210.248, Eph099B-233.152 within human framework regions. In a specific embodiment, a chimeric antibody of the invention immunospecifically binds EphA2 and comprises a VL CDR having an amino acid sequence of SEQ ID NO: 2, 3, 4,18, 19, or 20. In another embodiment, a chimeric antibody of the invention immunospecifically binds EphA2 and comprises one, two, or three VH CDRs having an amino acid sequence of any of the VH CDRs of Eph099B-102.147, Eph099B-208.261, Eph099B-210,248, or Eph099B-233.152 within human framework regions. In a specific embodiment, a chimeric antibody of the invention immunospecifically binds EphA2 and comprises a VH CDR having an amino acid sequence of SEQ ID NO:6, 7,8,22, 23, or 24. In a preferred embodiment, a chimeric antibody of the invention immunospecifically binds EphA2 and comprises one, two, or three VL CDRs having an amino acid sequence of any of the VL CDRs of Eph099B-102.147, Eph099B-208.261, Eph099B-210.248, or Eph099B-233.152 and further comprises one, two, or three VH CDRs having an amino acid sequence of any of the VH CDRs of Eph099B-102.147, Eph099B-205.261, Eph099B-210.248, Eph099B-233.152 within human framework regions. In a specific preferred embodiment, a chimeric antibody of the invention immunospecifically binds EphA2 and comprises a VL CDR having an amino acid sequence of SEQ ID NO: 2, 3, 4, 18, 19, or 20 and further comprises a VH CDR having an amino acid sequence of SEQ ID NO:6, 7, 8, 22, 23, or 24. In a more preferred embodiment, a chimeric antibody of the invention immunospecifically binds EphA2 and comprises three VL CDRs having an amino acid sequence of any of the VL CDRs of Eph099B-102.147, Eph099B-208.261, Eph099B-210.248, Eph099B-233.152 and three VH CDRs having an amino acid sequence of any of the VH CDRs of Eph099B-102.147, Eph099B-208.261, Eph099E-210.248, Eph099B-233.152 within human framework regions. In an even more preferred embodiment, a chimeric antibody of the invention immunospecifically binds EphA2 and comprises VL CDRs having an amino acid sequence selected from the group consisting of SEQ ID NO: 2, 3, 4, 18, 19, or 20 and further comprises VH CDRs having an amino acid sequence selected from the group consisting of SEQ ID NO:6, 7, 8, 22, 23, or 24.

Often, framework residues in the framework regions will be substituted with the corresponding residue from the CDR donor antibody to alter, preferably improve, antigen binding. These framework substitutions are identified by methods well known in the art, *e.g.*, by modeling of the interactions of the CDR and framework residues to identify framework residues important for antigen binding and sequence comparison to identify unusual framework residues at particular positions. (See, *e.g.*, U.S. Patent No. 5,585,089; and Riechmann et al., 1988, Nature 332:323.

A humanized antibody is an anti-body or its variant or fragment thereof which is capable of binding to a predetermined antigen and which comprises a framework region having substantially the amino acid sequence of a human immunoglobulin and a CDR having substantially the amino acid sequence of a non-human immunoglobulin. A humanized antibody comprises substantially all of at least one, and typically two, variable domains in which all or substantially all of the CDR regions correspond to those of a non-human immunoglobulin (*i.e.*, donor antibody) and all or substantially all of the framework regions are those of a human immunoglobulin consensus sequence. Preferably, a humanized antibody also comprises at least a portion of an immunoglobulin constant region (Fc), typically that of a human immunoglobulin. Ordinarily, the antibody will contain both the light chain as well as at least the variable domain of a heavy chain. The antibody also may include the CH1, hinge, CH2, CH3, and CH4 regions of the heavy chain. The humanized antibody can be selected from any class of immunoglobulins, including IgM, IgG, IgD, IgA and IgE, and any isotype, including $IgG_1$, $IgG_2$, $IgG_3$ and $IgG_4$. Usually the constant domain is a complement fixing constant domain where it is desired that the humanized antibody exhibit cytotoxic activity, and the class is typically $IgG_1$. Where such cytotoxic activity is not desirable, the constant domain may be of the $IgG_2$ class. The humanized antibody may comprise sequences from more than one class or isotype, and selecting particular constant domains to optimize desired effector functions is within the ordinary skill in the art. The framework and CDR regions of a humanized antibody need not correspond precisely to the parental sequences, *e.g.*, the donor CDR or the consensus framework may be mutagenized by substitution, insertion or deletion of at least one residue so that the CDR or framework residue at that site does not correspond to either the consensus or the import antibody. Such mutations, however, will not be extensive. Usually, at least 75% of the humanized antibody residues will correspond to those of the parental, framework region (FR) and CDR sequences, more often 90%, and most preferably greater than 95%. Humanized antibodies can be produced using variety of techniques known in the art, including but not limited to, CDR-gafting (European Patent No. EP 239,400; International Publication No. WO 91/09967; and U.S. Patent Nos. 5,225,539, 5,530,101, and 5,585,089), veneering or resurfacing (European Patent Nos. EP 592,106 and EP 519,596; Padlan, 1991, Molecular Immunology 28(4/5):489-498; Studnicka et al., 1994, Protein Engineering 7(6):805-814; and Roguska et al., 1994, PNAS 91:969-973), chain shuffling (U.S. Patent No. 5,565,332), and techniques disclosed in, *e.g.*, U.S. Patent Nos. 6,407,213, 5,766,886, 5,585,089, International Publication No. WO 9317105, Tan et al., 2002, J. Immunol. 169:1119-25, Caldas et al., 2000, Protein Eng. 13:353-60, Morea et al., 2000, Methods 20:267-79, Baca et al., 1997, J. Biol. Chem. 272:10678-84, Roguska et al., 1996, Protein Eng. 9:895-904, Couto et al, 1995, Cancer Res. 55 (23 Supp):5973s-5977s, Couto et al., 1995, Cancer Res. 55:1717-22, Sandhu, 1994, Gene 150:409-10, Pedersen et al., 1994, J. Mol. Biol. 235:959-73, Jones et al.,1986, Nature 321:522-525, Riechmann et al., 1988, Nature 332:323, and Presta, 1992, Curr. Op. Struct. Biol. 2:593-596. Often, framework residues in the framework regions will be substituted with the corresponding residue from the CDR donor antibody to alter, preferably improve, antigen binding. These framework substitutions are identified by methods well known in the art, *e.g.*, by modeling of the interactions of the CDR and framework residues to identify framework residues important for antigen binding and sequence comparison to identify unusual framework residues at particular positions. *(See, e.g.,* Queen et al., U.S. Patient No. 5,585,089; and Riechmann et al., 1988, Nature 332:323).

Further, the antibodies of the invention can, in turn, be utilized to generate anti-idiotype antibodies using techniques well known to those skilled in the art. (See, *e.g.*, Greenspan & Bona,1989, FASEB J. 7:437-444; and Nissinoff, 1991, J. Immunol. 147:2429-2438). The invention provides methods employing the use of polynucleotides comprising a nucleotide sequence encoding an antibody of the invention or a fragment thereof.

### 5.1.3 <u>Polynucleotides Encoding An Antibody</u>

The methods of the invention also encompass polynucleotides that hybridize under high stringency, intermediate or lower stringency hybridization conditions, *e.g.*, as defined *supra,* to polynucleotides that encode an antibody of the invention.

The polynucleotides may be obtained, and the nucleotide sequence of the polynucleotides determined, by any method known in the art. Since the amino acid sequences of the antibodies are known, nucleotide sequences encoding these antibodies can be determined using methods well known in the art, *i.e.,* nucleotide codons known to encode particular amino acids are assembled in such a way to generate a nucleic acid that encodes the antibody or fragment thereof of the invention. Such a polynucleotide encoding the antibody may be assembled from chemically synthesized oligonucleotides (*e.g.*, as described in Kutmeier et al., 1994, BioTechniques 17:242), which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding an antibody may be generated from nucleic acid from a suitable source. If a clone containing a nucleic acid encoding a particular antibody is not available, but the sequence of the antibody molecule is known (*e.g.*, see FIG. 19), a nucleic acid encoding the immunoglobulin may be chemically synthesized or obtained from a suitable source (*e.g.*, an antibody cDNA library, or a cDNA library generated from, or nucleic acid, preferably poly A+ RNA, isolated from, any tissue or cells expressing the antibody, such as hybridoma cells selected to express an antibody of the invention, *e.g.*, clones deposited in the ATCC as PTA-4572, PTA-4573, and PTA-4574) by PCR amplification using synthetic primers hybridizable to the 3' and 5' ends of the sequence or by cloning using an oligonucleotide probe specific for the particular gene sequence to identify, *e.g.*, a cDNA clone from a cDNA library that encodes the antibody. Amplified nucleic acids generated by PCR may then be cloned into replicable cloning vectors using any method well known in the art.

Once the nucleotide sequence of the antibody is determined, the nucleotide sequence of the antibody may be manipulated using methods well known in the art for the manipulation of nucleotide sequences, *e.g.*, recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, NY and Ausubel et al., eds., 1998, Current Protocols in Molecular Biology, John Wiley & Sons, NY), to generate antibodies having a different amino acid sequence, for example to create amino acid substitutions, deletions, and/or insertions.

In a specific embodiment, one or more of the CDRs is inserted within framework regions using routine recombinant DNA techniques. The framework regions may be naturally occurring or consensus framework regions, and preferably human framework regions (see, *e.g.*, Chothia et al., 1998, J. Mol. Biol. 278: 457-479 for a listing of human framework regions). Preferably, the polynucleotide generated by the combination of the framework regions and CDRs encodes an antibody that specifically binds to EphA2. Preferably, as discussed *supra,* one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present invention and within the skill of the art.

### 5.1.4 <u>Recombinant Expression Of An Antibody</u>

Recombinant expression of an antibody of the invention, derivative, analog or fragment thereof, (*e.g.*, a heavy or light chain of an antibody of the invention or a portion thereof or a single chain antibody of the invention), requires construction of an expression vector containing a polynucleotide that encodes the antibody. Once a polynucleotide encoding an antibody molecule or a heavy or light chain of an antibody, or portion thereof (preferably, but not necessarily, containing the heavy or light chain variable domain), of the invention has been obtained, the vector for the production of the antibody molecule may be produced by recombinant DNA technology using techniques well known in the art. Thus, methods for preparing a protein by expressing a polynucleotide containing an antibody, encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule of the invention, a heavy or light chain of an antibody, a heavy or light chain variable domain of an antibody or a portion thereof, or a heavy or light chain CDR, operably linked to a promoter. Such vectors may include the nucleotide sequence encoding the constant region of the antibody molecule (see, *e.g.*, International Publication Nos. WO 86/05807 and WO 89/01036; and U.S. Patent No. 5,122,464) and the variable domain of the antibody may be cloned into such a vector for expression of the entire heavy, the entire light chain, or both the entire heavy and light chains.

The expression vector is transferred to a host cell by conventional techniques and the transfected cells are then cultured by conventional techniques to produce an antibody of the invention. Thus, the invention includes host cells containing a polynucleotide encoding an antibody of the invention or fragments thereof, or a heavy or light chain thereof, or portion thereof, or a single chain antibody of the invention, operably linked to a heterologous promoter. In preferred embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibody molecules of the invention (see, *e.g.*, U.S. Patent No. 5,807,715). Such host-expression systems represent vehicles by which the coding sequences of interest may be produced and subsequently purified, but also represent cells which may, when transformed or transfected with the appropriate nucleotide coding sequences, express an antibody molecule of the invention *in situ.* These include but are not limited to microorganisms such as bacteria (*e.g., E. coli* and *B. subtilis*) transformed with recombinant bacteriophage DNA, plasmid DNA or cosmid DNA expression vectors containing antibody coding sequences; yeast (*e.g., Saccharomyces Pichia*) transformed with recombinant yeast expression vectors containing antibody coding sequences; insect cell systems infected with recombinant virus expression vectors (*e.g.*, baculovirus) containing antibody coding sequences; plant cell systems infected with recombinant virus expression vectors (*e.g.*, cauliflower mosaic virus, CaMV; tobacco mosaic virus, TMV) or transformed with recombinant plasmid expression vectors (*e.g.*, Ti plasmid) containing antibody coding sequences; or mammalian cell systems (*e.g.*, COS, CHO, BHK, 293, NS0, and 3T3 cells) harboring recombinant expression constructs containing promoters derived from the genome of mammalian cells (*e.g.*, metallothionein promoter) or from mammalian viruses (e.g., the adenovirus late promoter; the vaccinia virus 7.5K promoter). Preferably, bacterial cells such as *Escherichia coli,* and more preferably, eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; and Cockett et al., 1990, BioTechnology 8:2). In a specific embodiment, the expression of nucleotide sequences encoding antibodies or fragments thereof which immunospecifically bind to EphA2 and agonize EphA2, inhibit a cancer cell phenotype, preferentially bind epitopes on EphA2 that are selectively exposed or increased on cancer cells but not non-cancer cells and/or have a $K_{off}$ less than 3 X $10^{-3}S^{-1}$ is regulated by a constitutive promoter, inducible promoter or tissue specific promoter.

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited to, the *E. coli* expression vector pUR278 (Ruther et al., 1983, EMBO 12:1791), in which the antibody coding sequence may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione 5-transferase (GST). In general, such fusion proteins are soluble and can easily be purified from lysed cells by adsorption and binding to matrix glutathione-agarose beads followed by elution in the presence of free glutathione. The pGEX vectors are designed to include thrombin or factor Xa protease cleavage sites so that the cloned target gene product can be released from the GST moiety.

In an insect system, Autographa californica nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, *e.g.*, the late promoter and tripartite leader sequence. This chimeric gene may then be inserted in the adenovirus genome by *in vitro* or *in vivo* recombination. Insertion in a non-essential region of the viral genome (*e.g.*, region E1 or E3) will result in a recombinant virus that is viable and capable of expressing the antibody molecule in infected hosts (*e.g.*, see Logan & Shenk, 1984, PNAS 8 1:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see, *e.g.*, Bittner et al., 1987, Methods in Enzymol. 153:516-544).

In addition, a host cell strain may be chosen which modulates the expression of the inserted sequences, or modifies and processes the gene product in the specific fashion desired. Such modifications (*e.g.*, glycosylation) and processing (*e.g.*, cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERO, BHK, HeLa, COS, MDCK, 293, 3T3, W138, BT483, Hs578T, HTB2, BT2O, NS1 and T47D, NS0 (a murine myeloma cell line that does not endogenously produce any immunoglobulin chains), CRL7O30 and HsS78Bst cells.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (*e.g.*, promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compositions that interact directly or indirectly with the antibody molecule.

A number of selection systems may be used, including but not limited to, the herpes simplex virus thymidine kinase (Wigler et al., 1977, Cell 11:223), glutamine synthetase, hypoxanthine guanine phosphoribosyltransferase (Szybalska & Szybalski, 1992, Proc. Natl. Acad. Sci. USA 48:202), and adenine phosphoribosyltransferase (Lowy et al., 1980, Cell 22:8-17) genes can be employed in tk-, gs-, hgprt- or aprt- cells, respectively. Also, antimetabolite resistance can be used as the basis of selection for the following genes: *dhfr*, which confers resistance to methotrexate (Wigler et al., 1980, PNAS 77:357; O'Hare et al., 1981, PNAS 78:1527); *gpt,* which confers resistance to mycophenolic acid (Mulligan & Berg, 1981, PNAS 78:2072); neo, which confers resistance to the aminoglycoside G-418 (Wu and Wu, 1991, Biotherapy 3:87; Tolstoshev, 1993, Ann. Rev. Pharmacol. Toxicol. 32:573; Mulligan, 1993, Science 260:926; and Morgan and Anderson, 1993, Ann. Rev. Biochem. 62: 191; May, 1993, TIB TECH 11:155-); and *hygro,* which confers resistance to hygromycin (Santerre et al., 1984, Gene 30:147). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., 1981, J. Mol. Biol. 150:1.

The expression levels of an antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, 1986, Nature 322:52; and Kohler, 1980, PNAS 77:2197). The coding sequences for the heavy and light chains may comprise cDNA or genomic DNA.

Once an antibody molecule of the invention has been produced by recombinant expression, it may be purified by any method known in the art for purification of an immunoglobulin molecule, for example, by chromatography (*e.g.*, ion exchange, affinity, particularly by affinity for the specific antigen after Protein A, and sizing column chromatography), centrifugation, differential solubility, or by any other standard technique for the purification of proteins. Further, the antibodies of the present invention or fragments thereof may be fused to heterologous polypeptide sequences described herein or otherwise known in the art to facilitate purification.

### 5.2 <u>Prophylactic/Therapeutic uses</u>

The present invention encompasses methods for treating, preventing, or managing a disease or disorder associated with overexpression of EphA2 and/or cell hyperproliferative disorders, preferably cancer, in a subject comprising administering one or more EphA2 agonistic antibodies that bind EphA2 with a $K_{off}$ less than 3 X $10^{-1}$ $s^{-1}$, and which are optionally EphA2 cancer cell phenotype inhibiting antibodies or exposed EphA2 epitope antibodies preferably one or more monoclonal (or antibodies from some other source of a single antibody species) EphA2 agonistic antibodies that bind EphA2 with a $K_{off}$ less than 3 X $10^{-1}s^{-1}$ and which are optionally EphA2 cancer cell phenotype inhibiting antibodies or exposed EphA2 epitope antibodies. In a specific embodiment, the disorder to be treated, prevented, or managed is malignant cancer. In another specific embodiment, the disorder to be treated, prevented, or managed is a pre-cancerous condition associated with cells that overexpress EphA2. In more specific embodiments, the pre-cancerous condition is high-grade prostatic intraepithelial neoplasia (PIN), fibroadenoma of the breast, fibrocystic disease, or compound nevi.

In one embodiment, the antibodies of the invention can be administered in combination with one or more other therapeutic agents useful in the treatment, prevention or management of diseases or disorders associated with EphA2 overexpression, hyperproliferative disorders, and/or cancer. In certain embodiments, one or more EphA2 antibodies of the invention are administered to a mammal, preferably a human, concurrently with one or more other therapeutic agents useful for the treatment of cancer. The term "concurrently" is not limited to the administration of prophylactic or therapeutic agents at exactly the same time, but rather it is meant that the EphA2 antibodies of the invention and the other agent are administered to a subject in a sequence and within a time interval such that the antibodies of the invention can act together with the other agent to provide an increased benefit than if they were administered otherwise. For example, each prophylactic or therapeutic agent may be administered at the same time or sequentially in any order at different points in time; however, if not administered at the same time, they should be administered sufficiently close in time so as to provide the desired therapeutic or prophylactic effect Each therapeutic agent can be administered separately, in any appropriate form and by any suitable route. In other embodiments, the EphA2 antibodies of the invention are administered before, concurrently or after surgery. Preferably the surgery completely removes localized tumors or reduces the size of large tumors. Surgery can also be done as a preventive measure or to relieve pain.

In preferred embodiments, the one or more EphA2 antibodies of the invention consist of Eph099B-102.147, Eph099B-208.261, Eph099B-210.248, Eph099B-233.152. In a more preferred embodiment, the antibodies consist of Eph099B-102.147, Eph099B-208.261, Eph099B-210.248, Eph099B-233.152, that have been humanized. In other embodiments, variants of Eph099B-102.147, Eph099B-208.261, Eph099B-210.248, Eph099B-233.152, *e.g.*, with one or more amino acid substitutions, particularly in the variable domain, are provided that have increased activity, binding ability, etc., as compared to Eph099B-102.147, Eph099B-208.261, Eph099B-210.248, Eph099B-233.152.

In another specific embodiment, the therapeutic and prophylactic methods of the invention comprise administration of an inhibitor of EphA2 expression, such as but not limited to, antisense nucleic acids specific for EphA2, double stranded EphA2 RNA that mediates RNAi, anti-EphA2 ribozymes, etc. (see Section 5.4 *infra)* or an agonist of EphA2 activity other than an EphA2 antibody, such as small molecule inhibitors or agonists of EphA2 activity.

In various embodiments, the prophylactic or therapeutic agents are administered less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, no more than 24 hours apart or no more than 48 hours apart. In preferred embodiments, two or more components are administered within the same patient visit.

The dosage amounts and frequencies of administration provided herein are encompassed by the terms therapeutically effective and prophylactically effective. The dosage and frequency further will typically vary according to factors specific for each patient depending on the specific therapeutic or prophylactic agents administered, the severity and type of cancer, the route of administration, as well as age, body weight, response, and the past medical history of the patient. Suitable regimens can be selected by one skilled in the art by considering such factors and by following, for example, dosages reported in the literature and recommended in the Physician's Desk Reference (56th ed., 2002).

### 5.2.1 Patient Population

The invention provides antibodies for treating, preventing, and managing a disease or disorder associated with EphA2 overexpression and/or hyperproliferative cell disease, particularly cancer, by administrating to a subject in need thereof a therapeutically or prophylactically effective amount of one or more EphA2 antibodies of the invention. In another embodiment, the EphA2 antibodies of the invention can be administered in combination with one or more other therapeutic agents. The subject is preferably a mammal such as non-primate (*e.g.*, cows, pigs, horses, cats, dogs, rats, etc.) and a primate (*e.g.*, monkey, such as a cynomolgous monkey and a human). In a preferred embodiment, the subject is a human.

Specific examples of cancers that can be treated by the antibodies encompassed by the invention include, but are not limited to, cancers that overexpress EphA2. In a further embodiment, the cancer is of an epithelial origin. Examples of such cancers are cancer of the lung, colon, prostate, breast, and skin. Other cancers include cancer of the bladder and pancreas and renal cell carcinoma and melanoma. Additional cancers are listed by example and not by limitation in the following section 5.2.1.1. In particular embodiments, antibodies of the invention can be used to treat and/or prevent metastasis from primary tumors.

The antibodies and compositions of the invention comprise the administration of one or more EphA2 antibodies of the invention to subjects/patients suffering from or expected to suffer from cancer, *e.g.*, have a genetic predisposition for a particular type of cancer, have been exposed to a carcinogen, or are in remission from a particular cancer. As used herein, "cancer" refers to primary or metastatic cancers. Such patients may or may not have been previously treated for cancer. The antibodies and compositions of the invention may be used as a first line or second line cancer treatment. Included in the invention is also the treatment of patients undergoing other cancer therapies and the antibodies and compositions of the invention can be used before any adverse effects or intolerance of these other cancer therapies occurs. The invention also encompasses antibodies for administering to treat or ameliorate symptoms in refractory patients. In a certain embodiment, that a cancer is refractory to a therapy means that at least some significant portion of the cancer cells are not killed or their cell division arrested. The determination of whether the cancer cells are refractory can be made either *in vivo* or *in vitro* by any method known in the art for assaying the effectiveness of treatment on cancer cells, using the art-accepted meanings of "refractory" in such a context. In various embodiments, a cancer is refractory where the number of cancer cells has not been significantly reduced, or has increased. The invention also encompasses one or more EphA2 agonistic antibodies to prevent the onset or recurrence of cancer in patients predisposed to having cancer. Preferably, the monoclonal antibody is one or more of Eph099B-102.147, Eph099B-208.261, Eph099B-210.248, Eph099B-233.152.

In particular embodiments, the EphA2 antibodies of the invention, or other therapeutics that reduce EphA2 expression, are administered to reverse resistance or reduced sensitivity of cancer cells to certain hormonal, radiation and chemotherapeutic agents thereby resensitizing the cancer cells to one or more of these agents, which can then be administered (or continue to be administered) to treat or manage cancer, including to prevent metastasis. In a specific embodiment, EphA2 antibodies of the invention are administered to patients with increased levels of the cytokine IL-6, which has been associated with the development of cancer cell resistance to different treatment regimens, such as chemotherapy and hormonal therapy. In another specific embodiment, EphA2 antibodies of the invention are administered to patients suffering from breast cancer that have a decreased responsiveness or are refractory to tamoxifen treatment. In another specific embodiment, EphA2 antibodies of the invention are administered to patients with increased levels of the cytokine IL-6, which has been associated with the development of cancer cell resistance to different treatment regimens, such as chemotherapy and hormonal therapy.

In alternate embodiments, the invention provides antibodies for treating patients' cancer by administering one or more EphA2 antibodies of the invention in combination with any other treatment or to patients who have proven refractory to other treatments but are no longer on these treatments. Preferably, the EphA2 antibody is one or more of Eph099B-102.147, Eph099B-208.261, Eph099B-210.248, Eph099B-233.152. In certain embodiments, the patients being treated by the antibodies of the invention are patients already being treated with chemotherapy, radiation therapy, hormonal therapy, or biological therapy/immunotherapy. Among these patients are refractory patients and those with cancer despite treatment with existing cancer therapies. In other embodiments, the patients have been treated and have no disease activity and one or more agonistic antibodies of the invention are administered to prevent the recurrence of cancer.

In preferred embodiments, the existing treatment is chemotherapy. In particular embodiments, the existing treatment includes administration of chemotherapies including, but not limited to, methotrexate, taxol, mercaptopurine, thioguanine, hydroxyurea, cytarabine, cyclophosphamide, ifosfamide, nitrosoureas, cisplatin, carboplatin, mitomycin, dacarbazine, procarbizine, etoposides, campathecins, bleomycin, doxorubicin, idarubicin, daunorubicin, dactinomycin, plicamycin, mitoxantrone, asparaginase, vinblastine, vincristine, vinorelbine, paclitaxel, docetaxel, etc. Among these patients are patients treated with radiation therapy, hormonal therapy and/or biological therapy/immunotherapy. Also among these patients are those who have undergone surgery for the treatment of cancer.

Alternatively, the invention also encompasses antibodies for heating patients undergoing or having undergone radiation therapy. Among these are patients being treated or previously treated with chemotherapy, hormonal therapy and/or biological therapy/immunotherapy. Also among these patients are those who have undergone surgery for the treatment of cancer.

In other embodiments, the invention encompasses antibodies for treating patients undergoing or having undergone hormonal therapy and/or biological therapy/immunotherapy. Among these are patients being treated or having been treated with chemotherapy and/or radiation therapy. Also among these patients are those who have undergone surgery for the treatment of cancer.

Additionally, the invention also provides antibodies for treatment of cancer as an alternative to chemotherapy, radiation therapy, hormonal therapy, and/or biological therapy/immunotherapy where the therapy has proven or may prove too toxic, *i.e.*, results in unacceptable or unbearable side effects, for the subject being treated. The subject being treated with the antibodies of the invention may, optionally, be treated with other cancer treatments such as surgery, chemotherapy, radiation therapy, hormonal therapy or biological therapy, depending on which treatment was found to be unacceptable or unbearable.

In other embodiments, the invention provides administration of one or more agonistic monoclonal antibodies of the invention without any other cancer therapies for the treatment of cancer, but who have proved refractory to such treatments. In specific embodiments, patients refractory to other cancer therapies are administered one or more agonistic monoclonal antibodies in the absence of cancer therapies.

In other embodiments, patients with a pre-cancerous condition associated with cells that overexpress EphA2 can be administered antibodies of the invention to treat the disorder and decrease the likelihood that it will progress to malignant cancer. In a specific embodiments, the pre-cancerous condition is high-grade prostatic intraepithelial neoplasia (PIN), fibroadenoma of the breast, fibrocystic disease, or compound nevi.

In yet other embodiments, the invention provides antibodies for treating, preventing and managing non-cancer hyperproliferative cell disorders, particularly those associated with overexpression of EphA2, including but not limited to, asthma, chromic obstructive pulmonary disorder (COPD), restenosis (smooth muscle and/or endothelial), psoriasis, etc. These methods include methods analogous to those described above for treating, preventing and managing cancer, for example, by administering the EphA2 antibodies of the invention, as well as agents that inhibit EphA2 expression, combination therapy, administration to patients refractory to particular treatments, etc.

#### 5.2.1.1. Cancers

Cancers and related disorders that can be treated, prevented, or managed by methods and compositions of the present invention include but are not limited to cancers of an epithelial cell origin. Examples of such cancers include the following: leukemias, such as but not limited to, acute leukemia, acute lymphocytic leukemia, acute myelocytic leukemias, such as, myeloblastic, promyelocytic, myelomonocytic, monocytic, and erythroleukemia leukemias and myelodysplastic syndrome; chronic leukemias, such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myelomas such as but not limited to smoldering multiple myeloma, nonsecretory myeloma, osteosclerotic myeloma, plasma cell leukemia, solitary plasmacytoma and extramedullary plasmacytoma; Waldenström's macroglobulinemia; monoclonal gammopathy of undetermined significance; benign monoclonal gammopathy; heavy chain disease; bone and connective tissue sarcomas such as but not limited to bone sarcoma, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, synovial sarcoma; brain tumors such as but not limited to, glioma, astrocytoma, brain stem glioma, ependymoma, oligodendroglioma, nonglial tumor, acoustic neurinoma, craniopharyngioma, medulloblastoma, meningioma, pineocytoma, pineoblastoma, primary brain lymphoma; breast cancer including but not limited to ductal carcinoma, adenocarcinoma, lobular (small cell) carcinoma, intraductal carcinoma, medullary breast cancer, mucinous breast cancer, tubular breast cancer, papillary breast cancer, Paget's disease, and inflammatory breast cancer; adrenal cancer such as but not limited to pheochromocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to, squamous cell carcinoma, and adenocarcinoma; uterine cancers such as but not limited to endometrial carcinoma and uterine sarcoma; ovarian cancers such as but not limited to, ovarian epithelial carcinoma, borderline tumor, germ cell tumor, and stromal tumor; esophageal cancers such as but not limited to, squamous cancer, adenocarcinoma, adenoid cystic carcinoma, mucoepidermoid carcinoma, adenosquamous carcinoma, sarcoma, melanoma, plasmacytoma, verrucous carcinoma, and oat cell (small cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers such as but not limited to hepatocellular carcinoma and hepatoblastoma; gallbladder cancers such as adenocarcinoma; cholangiocarcinomas such as but not limited to pappillary, nodular, and diffuse; lung cancers such as non-small cell lung cancer, squamous cell carcinoma (epidermoid carcinoma), adenocarcinoma, large-cell carcinoma and small-cell lung cancer; testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, prostatic intraepithelial neoplasia, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoidcystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acral lentiginous melanoma; kidney cancers such as but not limited to renal cell carcinoma, adenocarcinoma, hypernephroma, fibrosarcoma, transitional cell cancer (renal pelvis and/ or uterer); Wilms' tumor; bladder cancers such as but not limited to transitional cell carcinoma, squamous cell cancer, adenocarcinoma, carcinosarcoma. In addition, cancers include myxosarcoma, osteogenic sarcoma, endotheliosarcoma, lymphangioendotheliosarcoma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, see Fishman et al., 1985, Medicine, 2d Ed., J.B. Lippincott Co., Philadelphia and Murphy et al., 1997, Informed Decisions: The Complete Book of Cancer Diagnosis, Treatment, and Recovery, Viking Penguin, Penguin Books U.S.A., Inc., United States of America)

Accordingly, the antibodies and compositions of the invention are also useful in the treatment or prevention of a variety of cancers or other abnormal proliferative diseases, including (but not limited to) the following: carcinoma, including that of the bladder, breast, colon, kidney, liver, lung, ovary, pancreas, stomach, cervix, thyroid and skin; including squamous cell carcinoma; hematopoietic tumors of lymphoid lineage, including leukemia, acute lymphocytic leukemia, acute lymphoblastic leukemia, B-cell lymphoma, T-cell lymphoma, Burkitt's lymphoma; hematopoietic tumors of myeloid lineage, including acute and chronic myelogenous leukemias and promyelocytic leukemia; tumors of mesenchymal origin, including fibrosarcoma and rhabdomyoscarcoma; other tumors, including melanoma, seminoma, tetratocarcinoma, neuroblastoma and glioma; tumors of the central and peripheral nervous system, including astrocytoma, neuroblastoma, glioma, and schwannomas; tumors of mesenchymal origin, including fibrosarcoma, rhabdomyoscarama, and osteosarcoma; and other tumors, including melanoma, xeroderma pigmentosum, keratoactanthoma, seminoma, thyroid follicular cancer and teratocarcinoma. It is also contemplated that cancers caused by aberrations in apoptosis would also be treated by the methods and compositions of the invention. Such cancers may include but not be limited to follicular lymphomas, carcinomas with p53 mutations, hormone dependent tumors of the breast, prostate and ovary, and precancerous lesions such as familial adenomatous polyposis, and myelodysplastic syndromes. In specific embodiments, malignancy or dysproliferative changes (such as metaplasias and dysplasias), or hyperproliferative disorders, are treated or prevented in the skin, lung, colon, breast, prostate, bladder, kidney, pancreas, ovary, or uterus. In other specific embodiments, sarcoma, melanoma, or leukemia is treated or prevented.

In some embodiments, the cancer is malignant and overexpresses EphA2. In other embodiments, the disorder to be treated is a pre-cancerous condition associated with cells that overexpress EphA2. In a specific embodiments, the pre-cancerous condition is high-grade prostatic intraepithelial neoplasia (PIN), fibroadenoma of the breast, fibrocystic disease, or compound nevi.

In preferred embodiments, the antibodies and compositions of the invention are used for the treatment and/or prevention of breast, colon, ovarian, lung, and prostate cancers and melanoma and are provided below by example rather than by limitation.

#### 5.2.1.2. Treatment of Breast Cancer

In specific embodiments, patients with breast cancer are administered an effective amount of one or more monoclonal antibodies of the invention. In another embodiment, the antibodies of the invention can be administered in combination with an effective amount of one or more other agents useful for breast cancer therapy including but not limited to: doxorubicin, epirubicin, the combination of doxorubicin and cyclophosphamide (AC), the combination of cyclophosphamide, doxorubicin and 5-fluorouracil (CAF), the combination of cyclophosphamide, epirubicin and 5-fluorouracil (CEF), herceptin, tamoxifen, the combination of tamoxifen and cytotoxic chemotherapy, taxanes (such as docetaxel and paclitaxel). In a further embodiment, antibodies of the invention can be administered with taxanes plus standard doxorubicin and cyclophosphamide for adjuvant treatment of node-positive, localized breast cancer.

In a specific embodiment, patients with pre-cancerous fibroadenoma of the breast or fibrocystic disease are administered an EphA2 antibody of the invention to treat the disorder and decrease the likelihood that it will progress to malignant breast cancer. In another specific embodiment, patients refractory to treatment, particularly hormonal therapy, more particulatly tamoxifen therapy, are administered an EphA2 antibody of the invention to treat the cancer and/or render the patient non-refractory or responsive.

#### 5.2.1.3. Treatment of Colon Cancer

In specific embodiments, patients with colon cancer are administered an effective amount of one or more monoclonal antibodies of the invention. In another embodiment, the antibodies of the invention can be administered in combination with an effective amount of one or more other agents useful for colon cancer therapy including but not limited to: the combination of 5-FU and leucovorin, the combination of 5-FU and levamisole, irinotecan (CPT-11) or the combination of irinotecan, 5-FU and leucovorin (IFL).

#### 5.2.1.4. Treatment of Prostate Cancer

In specific embodiments, patients with prostate cancer are administered an effective amount of one or more monoclonal antibodies of the invention. In another embodiment, the antibodies of the invention can be administered in combination with an effective amount of one or more other agents useful for prostate cancer therapy including but not limited to: external-beam radiation therapy, interstitial implantation of radioisotopes (*i.e.,* $I^{125}$, palladium, iridium), leuprolide or other LHRH agonists, non-steroidal antiandrogens (flutamide, nilutamide, bicalutamide), steroidal antiandrogens (cyproterone acetate), the combination of leuprolide and flutamide, estrogens such as DES, chlorotrianisene, ethinyl estradiol, conjugated estrogens U.S.P., DES-diphosphate, radioisotopes, such as strontium-89, the combination of external-beam radiation therapy and strontium-89, second-line hormonal therapies such as aminoglutethimide, hydrocortisone, flutamide withdrawal, progesterone, and ketoconazole, low-dose prednisone, or other chemotherapy regimens reported to produce subjective improvement in symptoms and reduction in PSA level including docetaxel, paclitaxel, estramustine/docetaxel, estramustine/etoposide, estramustine/vinblastine, and estramustine/paclitaxel.

In a specific embodiment, patients with pre-cancerous high-grade prostatic intraepithelial neoplasia (PIN) are administered an EphA2 antibody of the invention to treat the disorder and decrease the likelihood that it will progress to malignant prostate cancer.

5.2.1.5. Treatment of Melanoma

In specific embodiments, patients with melanoma are administered an effective amount of one or more monoclonal antibodies of the invention. In another embodiment, the antibodies of the invention can be administered in combination with an effective amount of one or more other agents useful for melanoma cancer therapy including but not limited to: dacarbazine (DTIC), nitrosoureas such as carmustine (BCNU) and lomustine (CCNU), agents with modest single agent activity including vinca alkaloids, platinum compounds, and taxanes, the Dartmouth regimen (cisplatin, BCNU, and DTIC), interferon alpha (IFN-A), and interleukin-2 (IL-2). In a specific embodiment, an effective amount of one or more agonistic monoclonal antibodies of the invention can be administered in combination with isolated hyperthermic limb perfusion (ILP) with melphalan (L-PAM), with or without tumor necrosis factor-alpha (TNF-alpha) to patients with multiple brain metastases, bone metastases, and spinal cord compression to achieve symptom relief and some shrinkage of the tumor with radiation therapy.

In a specific embodiment, patients with pre-cancerous compound nevi are administered an EphA2 antibody of the invention to treat the disorder and decrease the likelihood that it will progress to malignant melanoma.

5.2.1.6. Treatment of Ovarian Cancer

In specific embodiments, patients with ovarian cancer are administered an effective amount of one or more monoclonal antibodies of the invention. In another embodiment, the antibodies of the invention can be administered in combination with an effective amount of one or more other agents useful for ovarian cancer therapy including but not limited to: intraperitoneal radiation therapy, such as $P^{32}$ therapy, total abdominal and pelvic radiation therapy, cisplatin, the combination of paclitaxel (Taxol) or docetaxel (Taxotere) and cisplatin or carboplatin, the combination of cyclophosphamide and cisplatin, the combination of cyclophosphamide and carboplatin, the combination of 5-FU and leucovorin, etoposide, liposomal doxorubicin, gemcitabine or topotecan. It is contemplated that an effective amount of one or more agonistic monoclonal antibodies of the invention is administered in combination with the administration Taxol for patients with platinum-refractory disease. Included is the treatment of patients with refractory ovarian cancer including administration of: ifosfamide in patients with disease that is platinum-refractory, hexamethylmelamine (HMM) as salvage chemotherapy after failure of cisplatin-based combination regimens, and tamoxifen in patients with detectable levels of cytoplasmic estrogen receptor on their tumors.

5.2.1.7. Treatment of Lung Cancers

In specific embodiments, patients with small lung cell cancer are administered an effective amount of one or more monoclonal antibodies of the invention. In another embodiment, the antibodies of the invention can be administered in combination with an effective amount of one or more other agents useful for lung cancer therapy including but not limited to: thoracic radiation therapy, cisplatin, vincristine, doxorubicin, and etoposide, alone or in combination, the combination of cyclophosphamide, doxorubicin, vincristine/etoposide, and cisplatin (CAV/EP), local palliation with endobronchial laser therapy, endobronchial stents, and/or brachytherapy.

In other specific embodiments, patients with non-small lung cell cancer are administered an effective amount of one or more monoclonal antibodies of the invention in combination with an effective amount of one or more other agents useful for lung cancer therapy including but not limited to: palliative radiation therapy, the combination of cisplatin, vinblastine and mitomycin, the combination of cisplatin and vinorelbine, paclitaxel, docetaxel or gemcitabine, the combination of carboplatin and paclitaxel, interstitial radiation therapy for endobronchial lesions or stereotactic radiosurgery.

5.2.2 Other Prophylactic/Therapeutic Agents

In some embodiments, therapy by administration of one or more monoclonal antibodies is combined with the administration of one or more therapies such as, but not limited to, chemotherapies, radiation therapies, hormonal therapies, and/or biological therapies/immunotherapies. Prophylactic/therapeutic agents include, but are not limited to, proteinaceous molecules, including, but not limited to, peptides, polypeptides, proteins, including post-translationally modified proteins, antibodies etc.; or small molecules (less than 1000 daltons), inorganic or organic compounds; or nucleic acid molecules including, but not limited to, double-stranded or single-stranded DNA, or double-stranded or single-stranded RNA, as well as triple helix nucleic acid molecules. Prophylavtic/therapeutic agents can be derived from any known organism (including, but not limited to, animals, plants, bacteria, fungi, and protista, or viruses) or from a library of synthetic molecules.

In a specific embodiment, the methods of the invention encompass administration of an antibody of the invention in combination with the administration of one or more prophylactic/therapeutic agents that are inhibitors of kinases such as, but not limited to, ABL, ACK, AFK, AKT (*e.g.*, AKT-1, AKT-2, and AKT-3), ALK, AMP-PK, ATM, Aurora1, Aurora2, bARK1, bArk2, BLK, BMX, BTK, CAK, CaM kinase, CDC2, CDK, CK, COT, CTD, DNA-PK, EGF-R, ErbB-1, ErbB-2, ErbB-3, ErbB-4, ERK (*e.g.*, ERK1, ERK2, ERK3, ERK4, ERK5, ERK6, ERK7), ERT-PK, FAK, FGR (*e.g.*, FGF1R, FGF2R), FLT (*e.g.,* FLT-1, FLT-2, FLT-3, FLT-4), FRK, FYN, GSK (*e.g.*, GSK1, GSK2, GSK3-alpha, GSK3-beta, GSK4, GSK5), G-protein coupled receptor kinases (GRKs), HCK, HER2, HKII, JAK (*e.g.*, JAK1, JAK2, JAK3, JAK4), JNK (*e.g.*, JNK1, JNK2, JNK3), KDR, KIT, IGF-1 receptor, IKK-1, IKK-2, INSR (insulin receptor), IRAK1, IRAK2, IRK, ITK, LCK, LOK, LYN, MAPK, MAPKAPK-1, MAPKAPK-2, MEK, MET, MFPK, MHCK, MLCK, MLK3, NEU, NIK, PDGF receptor alpha, PDGF receptor beta, PHK, PI-3 kinase, PKA, PKB, PKC, PKG, PRK1, PYK2, p38 kinases, p135tyk2, p34cdc2, p42cdc2, p42mapk, p44mpk, RAF, RET, RIP, RIP-2, RK, RON, RS kinase, SRC, SYK, S6K, TAK1, TEC, TIE1, TIE2, TRKA, TXK, TYK2, UL13, VEGFR1, VEGFR2, YES, YRK, ZAP-70, and all subtypes of these kinases (see *e.g.*, Hardie and Hanks (1995) The Protein Kinase Facts Book, I and II, Academic Press, San Diego, Calif.). In preferred embodiments, an antibody of the invention is administered in combination with the administration of one or more prophylactic/therapeutic agents that are inhibitors of Eph receptor kinases (*e.g.*, EphA2, EphA4). In a most preferred embodiment, an antibody of the invention is administered in combination with the administration of one or more prophylactic/therapeutic agents that are inhibitors of EphA2.

In another specific embodiment, the methods of the invention encompass administration of an antibody of the invention in combination with the administration of one or more prophylactic/therapeutic agents that are angiogenesis inhibitors such as, but not limited to: Angiostatin (plasminogen fragment); antiangiogenic antithrombin III; Angiozyme; ABT-627; Bay 12-9566; Benefin; Bevacizumab; BMS-275291; cartilage-derived inhibitor (CDI); CAI; CD59 complement fragment; CEP-7055; Col 3; Combretastatin A-4; Endostatin (collagen XVIII fragment); fibronectin fragment; Gro-beta; Halofuginone; Heparinases; Heparin hexasaccharide fragment; HMV833; Human chorionic gonadotropin (hCG); IM-862; Interferon alpha/beta/gamma; Interferon inducible protein (IP-10); Interleukin-12; Kringle 5 (plasminogen fragment); Marimastat; Metalloproteinase inhibitors (TIMPs); 2-Methoxyestradiol; MMI 270 (CGS 27023A); MoAb IMC-1C11; Neovastat; NM-3; Panzem; PI-88; Placental ribonuclease inhibitor; Plasminogen activator inhibitor; Platelet factor-4 (PF4); Prinomastat; Prolactin 16kD fragment; Proliferin-related protein (PRP); PTK 787/ZK 222594; Retinoids; Solimastat; Squalamine; SS 3304; SU 5416; SU6668; SU11248; Tetrahydrocortisol-S; tetrathiomolybdate; thalidomide; Thrombospondin-1 (TSP-1); TNP-470; Transforming growth factor-beta (TGF-$\beta$); Vasculostatin; Vasostatin (calreticulin fragment); ZD6126; ZD6474; farnesyl transferase inhibitors (FTI); and bisphosphonates.

In another specific embodiment, the invention encompasses an antibody of the invention in combination with the administration of one or more prophylactic/therapeutic agents that are anti-cancer agents such as, but not limited to: acivicin, aclarubicin, acodazole hydrochloride, acronine, adozelesin, aldesleukin, altretamine, ambomycin, ametantrone acetate, aminoglutethimide, amsacrine, anastrozole, anthramycin, asparaginase, asperlin, azacitidine, azetepa, azotomycin, batimastat, benzodepa, bicalutamide, bisantrene hydrochloride, bisnafide dimesylate, bizelesin, bleomycin sulfate, brequinar sodium, bropirimine, busulfan, cactinomycin, calusterone, caracemide, carbetimer, carboplatin, carmustine, carubicin hydrochloride, carzelesin, cedefingol, chlorambucil, cirolemycin, cisplatin, cladribine, crisnatol mesylate, cyclophosphamide, cytarabine, dacarbazine, dactinomycin, daunorubicin hydrochloride, decarbazine, decitabine, dexormaplatin, dezaguanine, dezaguanine mesylate, diaziquone, docetaxel, doxorubicin, doxorubicin hydrochloride, droloxifene, droloxifene citrate, dromostanolone propionate, duazomycin, edatrexate, eflornithine hydrochloride, elsamitrucin, enloplatin, enpromate, epipropidine, epirubicin hydrochloride, erbulozole, esorubicin hydrochloride, estramustine, estramustine phosphate sodium, etanidazole, etoposide, etoposide phosphate, etoprine, fadrozole hydrochloride, fazarabine, fenretinide, floxuridine, fludarabine phosphate, fluorouracil, flurocitabine, fosquidone, fostriecin sodium, gemcitabine, gemcitabine hydrochloride, hydroxyurea, idarubicin hydrochloride, ifosfamide, ilmofosine, interleukin 2 (including recombinant interleukin 2, or rIL2), interferon alpha-2a, interferon alpha-2b, interferon alpha-n1, interferon alpha-n3, interferon beta-I a, interferon gamma-I b, iproplatin, irinotecan hydrochloride, lanreotide acetate, letrozole, leuprolide acetate, liarozole hydrochloride, lometrexol sodium, lomustine, losoxantrone hydrochloride, masoprocol, maytansine, mechlorethamine hydrochloride, megestrol acetate, melengestrol acetate, melphalan, menogaril, mercaptopurine, methotrexate, methotrexate sodium, metoprine, meturedepa, mitindomide, mitocarcin, mitocromin, mitogillin, mitomalcin, mitomycin, mitosper, mitotane, mitoxantrone hydrochloride, mycophenolic acid, nitrosoureas, nocodazole, nogalamycin, ormaplatin, oxisuran, paclitaxel, pegaspargase, peliomycin, pentamustine, peplomycin sulfate, perfosfamide, pipobroman, piposulfan, piroxantrone hydrochloride, plicamycin, plomestane, porfimer sodium, porfiromycin, prednimustine, procarbazine hydrochloride, puromycin, puromycin hydrochloride, pyrazofurin, riboprine, rogletimide, safingol, safingol hydrochloride, semustine, simtrazene, sparfosate sodium, sparsomycin, spirogermanium hydrochloride, spiromustine, spiroplatin, streptonigrin, streptozocin, sulofenur, talisomycin, tecogalan sodium, tegafur, teloxantrone hydrochloride, temoporfin, teniposide, teroxirone, testolactone, thiamiprine, thioguanine, thiotepa, tiazofurin, tirapazamine, toremifene citrate, trestolone acetate, triciribine phosphate, trimetrexate, trimetrexate glucuronate, triptorelin, tubulozole hydrochloride, uracil mustard, uredepa, vapreotide, verteporfin, vinblastine sulfate, vincristine sulfate, vindesine, vindesine sulfate, vinepidine sulfate, vinglycinate sulfate, vinleurosine sulfate, vinorelbine tartrate, vinrosidine sulfate, vinzolidine sulfate, vorozole, zeniplatin, zinostatin, zorubicin hydrochloride. Other anti-cancer drugs include, but are not limited to: 20-epi-1,25 dihydroxyvitamin D3, 5-ethynyluracil, abiraterone, aclarubicin, acylfulvene, adecypenol, adozelesin, aldesleukin, ALL-TK antagonists, altretamine, ambamustine, amidox, amifostine, aminolevulinic acid, amrubicin, amsacrine, anagrelide, anastrozole, andrographolide, angiogenesis inhibitors, antagonist D, antagonist G, antarelix, anti-dorsalizing morphogenetic protein-1, antiandrogens, antiestrogens, antineoplaston, aphidicolin glycinate, apoptosis gene modulators, apoptosis regulators, apurinic acid, ara-CDP-DL-PTBA, arginine deaminase, asulacrine, atamestane, atrimustine, axinastatin 1, axinastatin 2, axinastatin 3, azasetron, azatoxin, azatyrosine, baccatin III derivatives, balanol, batimastat, BCR/ABL antagonists, benzochlorins, benzoylstaurosporine, beta lactam derivatives, betaalethine, betaclamycin B, betulinic acid, bFGF inhibitor, bicalutamide, bisantrene, bisaziridinylspermine, bisnafide, bistratene A, bizelesin, breflate, bropirimine, budotitane, buthionine sulfoximine, calcipotriol, calphostin C, camptothecin derivatives, canarypox IL-2, capecitabine, carboxamide-amino-triazole, carboxyamidotriazole, CaRest M3, CARN 700, cartilage derived inhibitor, carzelesin, casein kinase inhibitors (ICOS), castanospermine, cecropin B, cetrorelix, chloroquinoxaline sulfonamide, cicaprost, cis-porphyrin, cladribine, clomifene analogues, clotrimazole, collismycin A, collismycin B, combretastatin A4, combretastatin analogue, conagenin, crambescidin 816, crisnatol, cryptophycin 8, cryptophycin A derivatives, curacin A, cyclopentanthraquinones, cycloplatam, cypemycin, cytarabine ocfosfate, cytolytic factor, cytostatin, dacliximab, decitabine, dehydrodidemnin B, deslorelin, dexamethasone, dexifosfamide, dexrazoxane, dexverapamil, diaziquone, didemnin B, didox, diethylnorspermine, dihydro-5-azacytidine, dihydrotaxol, dioxamycin, diphenyl spiromustine, docetaxel, docosanol, dolasetron, doxifluridine, droloxifene, dronabinol, duocarmycin SA, ebselen, ecomustine, edelfosine, edrecolomab, eflomithine, elemene, emitefur, epirubicin, epristeride, estramustine analogue, estrogen agonists, estrogen antagonists, etanidazole, etoposide phosphate, exemestane, fadrozole, fazarabine, fenretinide, filgrastim, finasteride, flavopiridol, flezelastine, fluasterone, fludarabine, fluorodaunorunicin hydrochloride, forfenimex, formestane, fostriecin, fotemustine, gadolinium texaphyrin, gallium nitrate, galocitabine, ganirelix, gelatinase inhibitors, gemcitabine, glutathione inhibitors, hepsulfam, heregulin, hexamethylene bisacetamide, hypericin, ibandronic acid, idarubicin, idoxifene, idramantone, ilmofosine, ilomastat, imidazoacridones, imiquimod, immunostimulant peptides, insulin-like growth factor-1 receptor inhibitor, interferon agonists, interferons, interleukins, iobenguane, iododoxorubicin, ipomeanol, iroplact, irsogladine, isobengazole, isohomohalicondrin B, itasetron, jasplakinolide, kahalalide F, lamellarin-N triacetate, lanreotide, leinamycin, lenograstim, lentinan sulfate, leptolstatin, letrozole, leukemia inhibiting factor, leukocyte alpha interferon, leuprolide+estrogen+progesterone, leuprorelin, levamisole, liarozole, linear polyamine analogue, lipophilic disaccharide peptide, lipophilic platinum compounds, lissoclinamide 7, lobaplatin, lombricine, lometrexol, lonidamine, losoxantrone, lovastatin, loxoribine, lurtotecan, lutetium texaphyrin, lysofylline, lytic peptides, maitansine, mannostatin A, marimastat, masoprocol, maspin, matrilysin inhibitors, matrix metalloproteinase inhibitors, menogaril, merbarone, meterelin, methioninase, metoclopramide, MIF inhibitor, mifepristone, miltefosine, mirimostim, mismatched double stranded RNA, mitoguazone, mitolactol, mitomycin analogues, mitonafide, mitotoxin fibroblast growth factor-saporin, mitoxantrone, mofarotene, molgramostim, monoclonal antibody, human chorionic gonadotrophin, monophosphoryl lipid A+myobacterium cell wall sk, mopidamol, multiple drug resistance gene inhibitor, multiple tumor suppressor 1-based therapy, mustard anticancer agent, mycaperoxide B, mycobacterial cell wall extract, myriaporone, N-acetyldinaline, N-substituted benzamides, nafarelin, nagrestip, naloxone+pentazocine, napavin, naphterpin, nartograstim, nedaplatin, nemorubicin, neridronic acid, neutral endopeptidase, nilutamide, nisamycin, nitric oxide modulators, nitroxide antioxidant, nitrullyn, O6-benzylguanine, octreotide, okicenone, oligonucleotides, onapristone, ondansetron, ondansetron, oracin, oral cytokine inducer, ormaplatin, osaterone, oxaliplatin, oxaunomycin, paclitaxel, paclitaxel analogues, paclitaxel derivatives, palauamine, palmitoylrhizoxin, pamidronic acid, panaxytriol, panomifene, parabactin, pazelliptine, pegaspargase, peldesine, pentosan polysulfate sodium, pentostatin, pentrozole, perflubron, perfosfamide, perillyl alcohol, phenazinomycin, phenylacetate, phosphatase inhibitors, picibanil, pilocarpine hydrochloride, pirarubicin, piritrexim, placetin A, placetin B, plasminogen activator inhibitor, platinum complex, platinum compounds, platinum-triamine complex, porfimer sodium, porfiromycin, prednisone, propyl bis-acridone, prostaglandin J2, proteasome inhibitors, protein A-based immune modulator, protein kinase C inhibitor, protein kinase C inhibitors, microalgal, protein tyrosine phosphatase inhibitors, purine nucleoside phosphorylase inhibitors, purpurins, pyrazoloacridine, pyridoxylated hemoglobin polyoxyethylene conjugate, raf antagonists, raltitrexed, ramosetron, ras farnesyl protein transferase inhibitors, ras inhibitors, ras-GAP inhibitor, retelliptine demethylated, rhenium Re 186 etidronate, rhizoxin, ribozymes, RII retinamide, rogletimide, rohitukine, romurtide, roquinimex, rubiginone B1, ruboxyl, safingol, saintopin, SarCNU, sarcophytol A, sargramostim, Sdi 1 mimetics, semustine, senescence derived inhibitor 1, sense oligonucleotides, signal transduction inhibitors, signal transduction modulators, single chain antigen binding protein, sizofiran, sobuzoxane, sodium borocaptate, sodium phenylacetate, solverol, somatomedin binding protein, sonermin, sparfosic acid, spicamycin D, spiromustine, splenopentin, spongistatin 1, squalamine, stem cell inhibitor, stem-cell division inhibitors, stipiamide, stromelysin inhibitors, sulfmosine, superactive vasoactive intestinal peptide antagonist, suradista, suramin, swainsonine, synthetic glycosaminoglycans, tallimustine, tamoxifen methiodide, tauromustine, taxol, tazarotene, tecogalan sodium, tegafur, tellurapyrylium, telomerase inhibitors, temoporfin, temozolomide, teniposide, tetrachlorodecaoxide, tetrazomine, thaliblastine, thalidomide, thiocoraline, thioguanine, thrombopoietin, thrombopoietin mimetic, thymalfasin, thymopoietin receptor agonist, thymotrinan, thyroid stimulating hormone, tin ethyl etiopurpurin, tirapazamine, titanocene bichloride, topsentin, toremifene, totipotent stem cell factor, translation inhibitors, tretinoin, triacetyluridine, triciribine, trimetrexate, triptorelin, tropisetron, turosteride, tyrosine kinase inhibitors, tyrphostins, UBC inhibitors, ubenimex, urogenital sinus-derived growth inhibitory factor, urokinase receptor antagonists, vapreotide, variolin B, vector system, erythrocyte gene therapy, velaresol, veramine, verdins, verteporfin, vinorelbine, vinxaltine, vitaxin, vorozole, zanoterone, zeniplatin, zilascorb, and zinostatin stimalamer. Preferred additional anti-cancer drugs are 5-fluorouracil and leucovorin.

In more particular embodiments, the present invention also comprises the administration of one or more monoclonal antibodies of the invention in combination with the administration of one or more therapies such as, but not limited to anti-cancer agents such as those disclosed in Table 2, preferably for the treatment of breast, ovary, melanoma, prostate, colon and lung cancers as described above.

**TABLE 2**

| Therapeutic Agent | Administration | Dose | Intervals |
|---|---|---|---|
| doxorubicin hydrochloride (Adriamycin RDF® and Adriamycin PFS®) | Intravenous | 60-75 mg/m$^2$ on Day 1 | 21 day intervals |
| epirubicin hydrochloride (Ellence™) | Intravenous | 100-120 mg/m$^2$ on Day 1 of each cycle or divided equally and given on Days 1-8 of the cycle | 3-4 week cycles |
| fluorousacil | Intravenous | How supplied: 5 ml and 10 ml vials (containing 250 and 500 mg flourouracil respectively) | |
| docetaxel (Taxotere®) | Intravenous | 60- 100 mg/m$^2$ over 1 hour | Once every 3 weeks |
| paclitaxel (Taxol®) | Intravenous | 175 mg/m$^2$ over 3 hours | Every 3 weeks for 4 courses (administered sequentially to doxorubicin-containing combination chemotherapy) |
| tamoxifen citrate (Nolvadex®) | Oral (tablet) | 20-40 mg Dosages greater than 20 mg should be given in divided doses (morning and evening) | Daily |
| leucovorin calcium for injection | Intravenous or intramuscular injection | How supplied: 350 mg vial | Dosage is unclear from text. PDR 3610 |
| luprolide acetate (Lupron®) | Single subcutaneous injection | 1 mg (0.2 ml or 20 unit mark) | Once a day |
| flutamide (Eulexin®) | Oral (capsule) | 250 mg (capsules contain 125 mg flutamide each) | 3 times a day at 8 hour intervals (total daily dosage 750 mg) |
| nilutamide (Nilandron®) | Oral (tablet) | 300 mg or 150 mg (tablets contain 50 or 150 mg nilutamide each) | 300 mg once a day for 30 days followed by 150 mg once a day |
| bicalutamide (Casodex®) | Oral (tablet) | 50 mg (tablets contain 50 mg bicalutamide each) | Once a day |
| progesterone | Injection | USP in sesame oil 50 mg/ml | |
| ketoconazole (Nizoral®) | Cream | 2% cream applied once or twice daily depending on symptoms | |

(continued)

| Therapeutic Agent | Administration | Dose | Intervals |
|---|---|---|---|
| prednisone | Oral (tablet) | Initial dosage may vary from 5 mg to 60 mg per day depending on the specific disease entity being treated. | |
| estramustine phosphate sodium (Emcyt®) | Oral (capsule) | 14 mg/ kg of body weight (*i.e.* one 140 mg capsule for each 10 kg or 22 1b of body weight) | Daily given in 3 or 4 divided doses |
| etoposide or VP-16 | Intravenous | 5 ml of 20 mg/ ml solution (100 mg) | |
| dacarbazine (DTIC-Dome®) | Intravenous | 2-4.5 mg/kg | Once a day for 10 days. May be repeated at 4 week intervals |
| polifeprosan 20 with carmustine implant (BCNU) (nitrosourea) (Gliadel®) | wafer placed in resection cavity | 8 wafers, each containing 7.7 mg of carmustine, for a total of 61.6 mg, if size and shape of resection cavity allows | |
| cisplatin | Injection | How supplied: solution of 1 mg/ml in multi-dose vials of 50mL and 100mL | |
| mitomycin | Injection | supplied in 5 mg and 20 mg vials (containing 5 mg and 20 mg mitomycin) | |
| gemcitabine HCl (Gemzar®) | Intravenous | For NSCLC- 2 schedules have been investigated and the optimum schedule has not been determined 4 week schedule-administration intravenously at 1000 $mg/m^2$ over 30 minutes on 3 week schedule-Gemzar administered intravenously at 1250 $mg/m^2$ over 30 minutes | 4 week schedule-Days 1,8 and 15 of each 28-day cycle. Cisplatin intravenously at 100 $mg/m^2$ on day 1 after the infusion of Gemzar. 3 week schedule-Days 1 and 8 of each 21 day cycle. Cisplatin at dosage of 100 $mg/m^2$ administered intravenously after administration of Gemzar on day 1. |
| carboplatin (Paraplatin®) | Intravenous | Single agent therapy: 360 $mg/m^2$ I.V. on day 1 (infusion lasting 15 minutes or longer) Other dosage calculations: Combination therapy with cyclophosphamide, Dose adjustment recommendations, Formula dosing, etc. | Every 4 weeks |

(continued)

| Therapeutic Agent | Administration | Dose | Intervals |
|---|---|---|---|
| ifosamide (Ifex®) | Intravenous | 1.2 $g/m^2$ daily | 5 consecutive days Repeat every 3 weeks or after recovery from hematologic toxicity |
| topotecan hydrochloride (Hycamtin®) | Intravenous | 1.5 $mg/m^2$ by intravenous infusion over 30 minutes daily | 5 consecutive days, starting on day 1 of 21 day course |

The invention also encompasses administration of the EphA2 antibodies of the invention in combination with radiation therapy comprising the use of x-rays, gamma rays and other sources of radiation to destroy the cancer cells. In preferred embodiments, the radiation treatment is administered as external beam radiation or teletherapy wherein the radiation is directed from a remote source. In other preferred embodiments, the radiation treatment is administered as internal therapy or brachytherapy wherein a radioactive source is placed inside the body close to cancer cells or a tumor mass.
Cancer therapies and their dosages, routes of administration and recommended usage are known in the art and have been described in such literature as the Physician's Desk Reference (56th ed., 2002).

## 5.3 Identification of Antibodies of the Invention

### 5.3.1 Agonistic Antibodies

Antibodies of the invention may preferably agonize (*i.e.*, elicit EphA2 phosphorylation) as well as immunospecifically bind to the EphA2 receptor. When agonized, EphA2 becomes phosphorylated and then subsequently degraded. Any method known in the art to assay either the level of EphA2 phosphorylation, activity, or expression can be used to assay candidate EphA2 antibodies to determine their agonistic activity (see, *e.g.*, Section 6.2 *infra).*

### 5.3.2 Antibodies That Preferentially Bind EphA2 Epitopes Exposed on Cancer Cells

Antibodies of the invention may preferably bind to EphA2 epitopes exposed on cancer cells (*e.g.*, cells overexpressing EphA2 and/or cells with substantial EphA2 that is not bound to ligand) but not non-cancer cells or cell where EphA2 is bound to ligand. In this embodiment, antibodies of the invention are antibodies directed to an EphA2 epitope not exposed on non-cancer cells but exposed on cancer cells (see, *e.g.*, Section 6.8 *infra).* Differences in EphA2 membrane distribution between non-cancer cells and cancer cells expose certain epitopes on cancer cells that are not exposed on non-cancer cells. For example, normally EphA2 is bound to its ligand, EphrinA1 and localizes at areas of cell-cell contacts. However, cancer cells generally display decreased cell-cell contacts as well as overexpress EphA2 in excess of its ligand. Thus, in cancer cells, there is an increased amount of unbound EphA2 that is not localized to cell-cell contacts. As such, in one embodiment, an antibody that preferentially binds unbound, unlocalized EphA2 is an antibody of the invention.
Any method known in the art to determine candidate EphA2 antibody binding/localization on a cell can be used to screen candidate antibodies for desirable binding properties. In a one embodiment, immunofluorescence microscopy is used to determine the binding characteristics of an antibody. Standard techniques can be used to compare the binding of an antibody binding to cells grown *in vitro.* In a specific embodiment, antibody binding to cancer cells is compared to antibody binding to non-cancer cells. An exposed EphA2 epitope antibody binds poorly to non-cancer cells but binds well to cancer cells. In another specific embodiment, antibody binding to non-cancer dissociated cells (*e.g.*, treated with a calcium chelator such as EGTA) is compared to antibody binding to non-cancer cells that have not been dissociated. An exposed EphA2 epitope antibody binds poorly non-cancer cells that have not been dissociated but binds well to dissociated non-cancer cells.
In another embodiment, flow cytometry is used to determine the binding characteristics of an antibody. In this embodiment, EphA2 may or may not be crosslinked to its ligand, Ephrin A1. An exposed EphA2 epitope antibody binds poorly crosslinked EphA2 but binds well to uncrosslinked EphA2.
In another embodiment, cell-based or immunoassays are used to determine the binding characteristics of an antibody. In this embodiment, antibodies that can compete with an EphA2 ligand (*e.g.*, Ephrin A1) for binding to EphA2 displace Ephrin A1 from EphA2. The EphA2 ligand used in this assay can be soluble protein (*e.g.*, recombinantly expressed) or expressed on a cell so that it is anchored to the cell.

### 5.3.3 Cancer Cell Phenotype Inhibiting Antibodies

Antibodies of the invention may preferably inhibit (and preferably reduce) cancer cell colony formation in, for example, soft agar, or tubular network formation in a three-dimensional basement membrane or extracellular matrix preparation as well as immunospecifically bind to the EphA2 receptor. One of skill in the art can assay candidate EphA2 antibodies for their ability to inhibit such behavior (see, *e.g.*, Section 6.2 *infra).* Metastatic tumor cells suspended in soft agar form colonies while benign tumors cells do not. Colony formation in soft agar can be assayed as described in Zelinski et al. (2001, Cancer Res. 61:2301-6). Antibodies to be assayed for agonistic activity can be included in bottom and top agar solutions. Metastatic tumor cells can be suspended in soft agar and allowed to grow. EphA2 cancer cell phenotype inhibiting antibodies will inhibit colony formation.

Another behavior specific to metastatic cells that can be used to identify cancer cell phenotype inhibiting antibodies is tubular network formation within a three-dimensional microenvironment, such as MATRIGEL™. Normally, cancer cells quickly assemble into tubular networks that progressively invade all throughout the MATRIGEL™. In the presence of an EphA2 cancer cell phenotype inhibiting antibody, cancer cells assemble into spherical structures that resemble the behavior of differentiated, non-cancerous cells. Accordingly, EphA2 cancer cell phenotype inhibiting antibodies can be identified by their ability to inhibit tubular network formation of cancer cells.

Any other method that detects an increase in contact inhibition of cell proliferation (*e.g.*, reduction of colony formation in a monolayer cell culture) may also be used to identify cancer cell phenotype inhibiting antibodies.

In addition to inhibiting cancer cell colony formation, cancer cell phenotype inhibiting antibodies may also cause a reduction or elimination of colonies when added to already established colonies of cancer cells by cell killing, e.g., by necrosis or apoptosis. Methods for assaying for necrosis and apoptosis are well known in the art.

### 5.3.4 Antibodies with Low $K_{off}$ Rates

The binding affinity of a monoclonal antibody of the invention to EphA2 or a fragment thereof and the off- rate of a monoclonal antibody-EphA2 interaction can be determined by competitive binding assays. One example of a competitive binding assay is a radioimmunoassay comprising the incubation of labeled EphA2 (*e.g.*, $^3H$ or $^{125}I$) with the monoclonal antibody of interest in the presence of increasing amounts ofunlabeled EphA2, and the detection of the monoclonal antibody bound to the labeled EphA2. The affinity of a monoclonal antibody for an EphA22 and the binding off-rates can be determine from the data by scatchard plot analysis. Competition with a second monoclonal antibody can also be determined using radioimmunoassays. In this case, EphA2 is incubated with a monoclonal antibody conjugated to a labeled compound (*e.g.*, $^3H$ or $^{125}I$) in the presence of increasing amounts of a second unlabeled Monoclonal antibody.

In a preferred embodiment, a candidate EphA2 antibody may be assayed using any surface plasmon resonance based assays known in the art for characterizing the kinetic parameters of the EphA2-EphA2 antibody interaction. Any SPR instrument commercially available including, but not limited to, BIACORE Instruments, available from Biacore AB (Uppsala, Sweden); IAsys instruments available form Affinity Sensors (Franklin, MA.); IBIS system available from Windsor Scientific Limited (Berks, UK), SPR-CELLIA systems available from Nippon Laser and Electronics Lab (Hokkaido, Japan), and SPR Detector Spreeta available from Texas Instruments (Dallas, TX) can be used in the instant invention. For a review of SPR-based technology see Mullet et al., 2000, Methods 22: 77-91; Dong et al., 2002, Review in Mol. Biotech., 82: 303-23; Fivash et al., 1998, Current Opinion in Biotechnology 9: 97-101; Rich et al., 2000, Current Opinion in Biotechnology 11: 54-61; all of which are incorporated herein by reference in their entirety. Additionally, any or the SPR instruments and SPR based methods for measuring protein-protein interactions described in U.S. Patent Nos. 6,373,577; 6,289,286; 5,322,798; 5,341,215; 6,268,125 are contemplated in the methods of the invention.

Briefly, SPR based assays involve immobilizing a member of a binding pair on a surface, and monitoring its interaction with the other member of the binding pair in solution. SPR is based on measuring the change in refractive index of the solvent near the surface that occurs upon complex formation or dissociation. The surface onto which the immobilization occur is the sensor chip, which is at the heart of the SPR technology; it consists of a glass surface coated with a thin layer of gold and forms the basis for a range of specialized surfaces designed to optimize the binding of a molecule to the surface. A variety of sensor chips are commercially available especially from the companies listed *supra,* all of which may be used in the methods of the invention. Examples of sensor chips include those available from BIAcore AB, Inc., *e.g.*, Sensor Chip CMS, SA, NTA, and HPA. A molecule of the invention may be immobilized onto the surface of a sensor chip using any of the immobilization methods and chemistries known in the art, including but not limited to direct covalent coupling via amine groups, direct covalent coupling via sulfhydryl groups, biotin attachment to avidin coated surface, aldehyde coupling to carbohydrate groups and attachment through the histidine tag with NTA chips.

In a more preferred embodiment, BIACORE™ kinetic analysis is used to determine the binding on and off rates of monoclonal antibodies to EphA2 (see, *e.g.*, Section 6.7 *infra).* BIACORE™ kinetic analysis comprises analyzing the binding and dissociation of a monoclonal antibody from chips with immobilized EphA2 or fragment thereof on their surface.

Once an entire data set is collected, the resulting binding curves are globally fitted using computer aigorithms supplied by the manufacturer, BIAcore, Inc. (Piscataway, NJ). These algorithms calculate both the $K_{on}$ and $K_{off}$, from which the apparent equilibrium binding constant, $K_D$ is deduced as the ratio of the two rate constants (*i.e.*, $K_{off}/K_{on}$). More detailed treatments of how the individual rate constants are derived can be found in the BIAevaluaion Software Handbook (BIAcore, Inc., Piscataway, NJ). The analysis of the generated data may be done using any method known in the art. For a review of the various methods of interpretation of the kinetic data generated *see* Myszka, 1997, Current Opinion in Biotechnology 8: 50-7; Fisher et al.,1994, Current Opinion in Biotechnology 5: 389-95; O'Shannessy, 1994, Current Opinion in Biotechnology, 5:65-71; Chaiken et al., 1992, Analytical Biochemistry, 201: 197-210; Morton et al., 1995, Analytical Biochemistry 227: 176-85; O'Shannessy et al., 1996, Analytical Biochemistry 236: 275-83.

The invention encompasses antibodies that immunospecifically bind to EphA2 and have a **$K_{off}$ rate (antibody (Ab) + antigen (Ag) $\xleftarrow{K_{off}}$ Ab-Ag)** of less than 3 X $10^{-3}$ $s^{-1}$, more preferably less than 1 X $10^{-3}s^{-1}$. In other embodiments, the antibodies of the invention immunospecifically bind to EphA2 and have a $K_{off}$ of less than 5 X $10^{-3}$ $s^{-1}$, less than $10^{-3}$ $s^{-1}$, less than 8 X $10^{-4}$ $s^{-1}$, less than 5 X $10^{-4}$ $s^{-1}$, less than $10^{-4}$ $s^{-1}$, less than 9 X $10^{-5}$ $s^{-1}$, less than 5 X $10^{-5}$ $s^{-1}$, less than $10^{-5}$ $s^{-1}$, less than 5 X $10^{-6}$ $s^{-1}$, less than $10^{-6}$ $s^{-1}$, less than 5 X $10^{-7}$ $s^{-1}$, less than $10^{-7}s^{-1}$, less than 5 X $10^{-8}$ $s^{-1}$, less than $10^{-8}$ $s^{-1}$, less than 5 X $10^{-9}$ $s^{-1}$ less than $10^{-9}$ $s^{-1}$, or less than $10^{-10}$ $s^{-1}$.

Thus, the invention describes methods of assaying and screening for EphA2 antibodies of the invention by incubating antibodies that specifically bind EphA2, particularly that bind the extracellular domain of EphA2, with cells that express EphA2, particularly cancer cells, preferably metastatic cancer cells, that overexpress EphA2 (relative to non-cancer cells of the same cell type) and then assaying for an increase in EphA2 phosphorylation and/or EphA2 degradation (for agonistic antibodies), or reduction in colony formation in soft agar or tubular network formation in three-dimensional basement membrane or extracellular matrix preparation (for cancer cell phenotype inhibiting antibodies), or increased antibody binding to cancer cells as compared to non-cancer cells by *e.g.*, immunofluorescence (for exposed EphA2 epitope antibodies) thereby identifying an EphA2 antibody of the invention.

### 5.5 Characterization And Demonstration Of Therapeutic Or Prophylactic Utility

Toxicity and efficacy of the prophylactic and/or therapeutic protocols of the instant invention can be determined by standard pharmaceutical procedures in cell cultures or experimental animals, *e.g.*, for determining the $LD_{50}$ (the dose lethal to 50% of the population) and the $ED_{50}$ (the dose therapeutically effective in 50% of the population). The dose ratio between toxic and therapeutic effects is the therapeutic index and it can be expressed as the ratio $LD_{50}/ED_{50}$. Prophylactic and/or therapeutic agents that exhibit large therapeutic indices are preferred. While prophylactic and/or therapeutic agents that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage of the prophylactic and/or therapeutic agents for use in humans. The dosage of such agents lies preferably within a range of circulating concentrations that include the $ED_{50}$ with little or no toxicity. The dosage may vary within this range depending upon the dosage form employed and the route of administration utilized. For any agent used in the method of the invention, the therapeutically effective dose can be estimated initially from cell culture assays. A dose may be formulated in animal models to achieve a circulating plasma concentration range that includes the $IC_{50}$ (*i.e.*, the concentration of the test compound that achieves a half-maximal inhibition of symptoms) as determined in cell culture. Such information can be used to more accurately determine useful doses in humans. Levels in plasma may be measured, for example, by high performance liquid chromatography.

The anti-cancer activity of the therapies used in accordance with the present invention also can be determined by using various experimental animal models for the study of cancer such as the SCID mouse model or transgenic mice where a mouse EphA2 is replaced with the human EphA2, nude mice with human xenografts, animal models described in Section 6 *infra,* or any animal model (including hamsters, rabbits, etc.) known in the art and described in Relevance of Tumor Models for Anticancer Drug Development (1999, eds. Fiebig and Burger); Contributions to Oncology (1999, Karger); The Nude Mouse in Ontology Research (1991, eds. Boven and Winograd); and Anticancer Drug Development Guide (1997 ed. Teicher).

#### 5.5.1 Demonstration of Therapeutic or Prophylactic Utility

The protocols and compositions of the invention are preferably tested *in vitro,* and then *in vivo,* for the desired therapeutic or prophylactic activity, prior to use in humans. For example, *in vitro* assays which can be used to determine whether administration of a specific therapeutic protocol is indicated, include *in vitro* cell culture assays in which a patient tissue sample is grown in culture, and exposed to or otherwise administered a protocol, and the effect of such protocol upon the tissue sample is observed, *e.g.*, increased phosphorylation/degradation of EphA2, inhibition of or decrease in growth and/or colony formation in soft agar or tubular network formation in three-dimensional basement membrane or extracellular matrix preparations. A lower level of proliferation or survival of the contacted cells indicates that the therapeutic agent is effective to treat the condition in the patient Alternatively, instead of culturing cells from a patient, therapeutic agents and methods may be screened using cells of a tumor or malignant cell line. Many assays standard in the art can be used to assess such survival and/or growth; for example, cell proliferation can be assayed by measuring $^3$H-thymidine incorporation, by direct cell count, by detecting changes in transcriptional activity of known genes such as proto-oncogenes (*e.g.*, fos, myc) or cell cycle markers; cell viability can be assessed by trypan blue staining, differentiation can be assessed visually based on changes in morphology, increased phosphorylation/degradation of EphA2, decreased growth and/or colony formation in soft agar or tubular network formation in three-dimensional basement membrane or extracellular matrix preparation, etc.

Compounds for use in therapy can be tested in suitable animal model systems prior to testing in humans, including but not limited to in rats, mice, chicken, cows, monkeys, rabbits, hamsters, etc., for example, the animal models described above. The compounds can then be used in the appropriate clinical trials.

Further, any assays known to those skilled in the art can be used to evaluate the prophylactic and/or therapeutic utility of the combinatorial therapies disclosed herein for treatment or prevention of cancer.

## 5.6 Pharmaceutical Compositions

The compositions of the invention include bulk drug compositions useful in the manufacture of pharmaceutical compositions (*e.g.*, impure or non-sterile compositions) and pharmaceutical compositions (*i.e.*, compositions that are suitable for administration to a subject or patient) which can be used in the preparation of unit dosage forms. Such compositions comprise a prophylactically or therapeutically effective amount of a prophylactic and/or therapeutic agent disclosed herein or a combination of those agents and a pharmaceutically acceptable carrier. Preferably, compositions of the invention comprise a prophylactically or therapeutically effective amount of one or more EphA2 antibodies of the invention and a pharmaceutically acceptable carrier or an agent that reduces EphA2 expression (*e.g.*, antisense oligonucleotides) and a pharmaceutically acceptable carrier. In a further embodiment, the composition of the invention further comprises an additional therapeutic, *e.g.*, anti-cancer, agent.

In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant (*e.g.*, Freund's adjuvant (complete and incomplete) or, more preferably, MF59C.1 adjuvant available from Chiron, Emeryville, CA), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like.

Generally, the ingredients of compositions of the invention are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compositions of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

Various delivery systems are known and can be used to administer an agonistic monoclonal antibody of the invention or the combination of an agonistic monoclonal antibody of the invention and a prophylactic agent or therapeutic agent useful for preventing or treating cancer, *e.g.*, encapsulation in liposomes, microparticles, microcapsules, recombinant cells capable of expressing the antibody or antibody fragment, receptor-mediated endocytosis (see, *e.g.,* Wu and Wu, 1987, J. Biol. Chem. 262:4429-4432), construction of a nucleic acid as part of a retroviral or other vector, etc.

Methods of administering a prophylactic or therapeutic agent of the invention include, but are not limited to, parenteral administration (*e.g.*, intradermal, intramuscular, intraperitoneal, intravenous and subcutaneous), epidural, and mucosal (*e.g.*, intranasal, inhaled, and oral routes). In a specific embodiment, prophylactic or therapeutic agents of the invention are administered intramuscularly, intravenously, or subcutaneously. The prophylactic or therapeutic agents may be administered by any convenient route, for example by infusion or bolus injection, by absorption through epithelial or mucocutaneous linings (*e.g.*, oral mucosa, rectal and intestinal mucosa, etc.) and may be administered together with other biologically active agents. Administration can be systemic or local.

In a specific embodiment, it may be desirable to administer the prophylactic or therapeutic agents of the invention locally to the area in need of treatment; this may be achieved by, for example, and not by way of limitation, local infusion, by injection, or by means of an implant, said implant being of a porous, non-porous, or gelatinous material, including membranes, such as sialastic membranes, or fibers.

In yet another embodiment, the prophylactic or therapeutic agent can be delivered in a controlled release or sustained release system. In one embodiment, a pump may be used to achieve controlled or sustained release (see Langer, *supra;* Sefton, 1987, CRC Crit. Ref. Biomed. Eng. 14:20; Buchwald et al., 1980, Surgery 88:507; Saudek et al., 1989, N. Engl. J. Med. 321:574). In another embodiment, polymeric materials can be used to achieve controlled or sustained release of the antibodies of the invention or fragments thereof (see *e.g.*, Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, 1983, J. Macromol. Sci. Rev. Macromol. Chem. 23:61; see also Levy et al., 1985, Science 228:190; During et al., 1989, Ann. Neural. 25: 351; Howard et al., 1989, J. Neurosurg. 7 1:105); U.S. Patent Nos. 5,679,377; 5,916,597; 5,912,015; 5,989,463; 5,128,326; International Publication Nos. WO 99/15154 and WO 99/20253. Examples of polymers used in sustained release formulations include, but are not limited to, poly(2-hydroxy ethyl methacrylate), poly(methyl methacrylate), poly (acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In a preferred embodiment, the polymer used in a sustained release formulation is inert, free of leachable impurities, stable on storage, sterile, and biodegradable. In yet another embodiment, a controlled or sustained release system can be placed in proximity of the prophylactic or therapeutic target, thus requiring only a fraction of the systemic dose (see, *e.g.*, Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more therapeutic agents of the invention. See, *e.g.*, U.S. Patent No. 4,526,938; International Publication Nos. WO 91/05548 and WO 96/20698; Ning et al., 1996, Radiotherapy & Oncology 39:179-189; Song et al., 1995, PEA Journal of Pharmaceutical Science & Technology 50:372-397; Cleek et al., 1997, Pyro. Int'l. Symp. Control. Rel. Bioact. Mater. 24:853-854; and Lam et al., 1997, Proc. Int'l. Symp. Control Rel. Bioact. Mater. 24:759-760.

### 5.6.2 Formulations

Pharmaceutical compositions for use in accordance with the present invention may be formulated in conventional manner using one or more physiologically acceptable carriers or excipients.

Thus, the EphA2 agonistic antibodies of the invention or other anti-EphA2 agents (*e.g.*, antisense and other nucleic acids) and their physiologically acceptable salts and solvates may be formulated for administration by inhalation or sedation (either through the mouth or the nose) or oral, parenteral or mucosal (such as buccal, vaginal, rectal, sublingual) administration. In a preferred embodiment, local or systemic parenteral administration is used.

For oral administration, the pharmaceutical compositions may take the form of, for example, tablets or capsules prepared by conventional means with pharmaceutically acceptable excipients such as binding agents (*e.g.*, pregelatinised maize starch, polyvinylpyrrolidone or hydroxypropyl methylcellulose); fillers (*e.g.*, lactose, microcrystalline cellulose or calcium hydrogen phosphate); lubricants (*e.g.*, magnesium stearate, talc or silica); disintegrants (*e.g.*, potato starch or sodium starch glycolate); or wetting agents (*e.g.*, sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g.*, sorbitol syrup, cellulose derivatives or hydrogenated edible fats); emulsifying agents (*e.g.*, lecithin or acacia); non-aqueous vehicles (*e.g.*, almond oil, oily esters, ethyl alcohol or fractionated vegetable oils); and preservatives (*e.g.*, methyl or propyl-p-hydroxybenzoates or sonic acid). The preparations may also contain buffer salts, flavoring, coloring and sweetening agents as appropriate.

Preparations for oral administration may be suitably formulated to give controlled release of the active compound.

For buccal administration the compositions may take the form of tablets or lozenges formulated in conventional manner.

For administration by inhalation, the prophylactic or therapeutic agents for use according to the present invention are conveniently delivered in the form of an aerosol spray presentation from pressurized packs or a nebulizer, with the use of a suitable propellant, *e.g.*, dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g.*, gelatin for use in an inhaler or insufflator may be formulated containing a powder mix of the compound and a suitable powder base such as lactose or starch.

The prophylactic or therapeutic agents may be formulated for parenteral administration by injection, *e.g.*, by bolus injection or continuous infusion. Formulations for injection may be presented in unit dosage form, *e.g.*, in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g.*, sterile pyrogen-free water, before use.

The prophylactic or therapeutic agents may also be formulated in rectal compositions such as suppositories or retention enemas, *e.g.*, containing conventional suppository bases such as cocoa butter or other glycerides.

In addition to the formulations described previously, the prophylactic or therapeutic agents may also be formulated as a depot preparation. Such long acting formulations may be administered by implantation (for example subcutaneously or intramuscularly) or by intramuscular injection. Thus, for example, the prophylactic or therapeutic agents may be formulated with suitable polymeric or hydrophobic materials (for example as an emulsion in an acceptable oil) or ion exchange resins, or as sparingly soluble derivatives, for example, as a sparingly soluble salt.

The invention also provides that a prophylactic or therapeutic agent is packaged in a hermetically sealed container such as an ampoule or sachette indicating the quantity. In one embodiment, the prophylactic or therapeutic agent is supplied as a dry sterilized lyophilized powder or water free concentrate in a hermetically sealed container and can be reconstituted, *e.g.*, with water or saline to the appropriate concentration for administration to a subject.

In a preferred embodiment of the invention, the formulation and administration of various chemotherapeutic, biological/immunotherapeutic and hormonal therapeutic agents are known in the art and often described in the Physician's Desk Reference, 56th ed. (2002). For instance, in certain specific embodiments of the invention, the therapeutic agents of the invention can be formulated and supplied as provided in Table 2.

In other embodiments of the invention, radiation therapy agents such as radioactive isotopes can be given orally as liquids in capsules or as a drink. Radioactive isotopes can also be formulated for intravenous injections. The skilled oncologist can determine the preferred formulation and route of administration.

In certain embodiments the agonistic monoclonal antibodies of the invention, are formulated at 1 mg/ml, 5 mg/ml, 10 mg/ml, and 25 mg/ml for intravenous injections and at 5 mg/ml, 10 mg/ml, and 80 mg/ml for repeated subcutaneous administration and intramuscular injection.

The compositions may, if desired, be presented in a pack or dispenser device that may contain one or more unit dosage forms containing the active ingredient. The pack may for example comprise metal or plastic foil, such as a blister pack. The pack or dispenser device may be accompanied by instructions for administration.

### 5.6.3 Dosages

The amount of the composition of the invention which will be effective in the treatment, prevention or management of cancer can be determined by standard research techniques. For example, the dosage of the composition which will be effective in the treatment, prevention or management of cancer can be determined by administering the composition to an animal model such as, *e.g.*, the animal models disclosed herein or known to those skilled in the art. In addition, *in vitro* assays may optionally be employed to help identify optimal dosage ranges.

Selection of the preferred effective dose can be determined (*e.g.*, via clinical trials) by a skilled artisan based upon the consideration of several factors which will be known to one of ordinary skill in the art. Such factors include the disease to be treated or prevented, the symptoms involved, the patient's body mass, the patient's immune status and other factors known by the skilled artisan to reflect the accuracy of administered pharmaceutical compositions.

The precise dose to be employed in the formulation will also depend on the route of administration, and the seriousness of the cancer, and should be decided according to the judgment of the practitioner and each patient's circumstances. Effective doses may be extrapolated from dose-response curves derived from *in vitro* or animal model test systems.

For antibodies, the dosage administered to a patient is typically 0.1 mg/kg to 100 mg/kg of the patient's body weight. Preferably, the dosage administered to a patient is between 0.1 mg/kg and 20 mg/kg of the patient's body weight, more preferably 1 mg/kg to 10 mg/kg of the patient's body weight. Generally, human and humanized antibodies have a longer half-life within the human body than antibodies from other species due to the immune response to the foreign polypeptides. Thus, lower dosages of human antibodies and less frequent administration is often possible.

For other cancer therapeutic agents administered to a patient, the typical doses of various cancer therapeutics known in the art are provided in Table 2. Given the invention, certain preferred embodiments will encompass the administration of lower dosages in combination treatment regimens than dosages recommended for the administration of single agents.

The invention provides for any method of administrating lower doses of known prophylactic or therapeutic agents than previously thought to be effective for the prevention, treatment, management or amelioration of cancer. Preferably, lower doses of known anti-cancer therapies are administered in combination with lower doses of agonistic monoclonal antibodies of the invention.

## 5.7 Kits

The invention provides a pharmaceutical pack or kit comprising one or more containers filled with an monoclonal antibody of the invention. Additionally, one or more other prophylactic or therapeutic agents useful for the treatment of a cancer can also be included in the pharmaceutical pack or kit. The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The present invention provides kits that can be used in the above methods. In one embodiment, a kit comprises one or more a monoclonal antibodies of the invention. In another embodiment, a kit further comprises one or more other prophylactic or therapeutic agents useful for the treatment of cancer, in one or more containers. Preferably the monoclonal antibody of the invention is Eph099B-102.147, Eph099B-208.261, Eph099B-210.248, Eph099B-233.152, or any of the antibodies listed in Table 6. In certain embodiments, the other prophylactic or therapeutic agent is a chemotherapeutic. In other embodiments, the prophylactic or therapeutic agent is a biological or hormonal therapeutic.

# 6. EXAMPLES

## 6.1 Preparation of Monoclonal Antibodies

### Immunization and Fusion

Monoclonal antibodies against the extracellular domain of EphA2 were generated using the fusion protein EphA2-Fc. This fusion protein consisted of the extracellular domain of human EphA2 linked to human immunoglobulin to facilitate secretion of the fusion protein.

Two groups of 5 mice each (either Balb/c mice (group A) or SJL mice (group B)) were injected with 5 μg of EphA2-Fc in TiterMax Adjuvant (total volume 100μl) in the left metatarsal region at days 0 and 7. Mice were injected with 10 μg of EphA2-Fc in PBS (total volume 100μl) in the left metatarsal region at days 12 and 14. On day 15, the popliteal and inguinal lymph node cells from the left leg and groin were removed and somatically fused (using PEG) with P3XBc1-2-13 cells.

Hybridomas producing Eph099B-102.147, Eph099B-208.261, Eph099B-210.248, and Eph099B-233.152 antibodies were isolated from fusions of lymph nodes from immunized SJL mice.

### Antibody Screening

Supernatants from bulk culture hybridomas were screened for immunoreactivity against EphA2 (Table 6, column 4) using standard molecular biological techniques (*e.g.*, ELISA immunoassay). Supernatants were further screened for the ability to inhibit an EphA2 monoclonal antibody (EA2; ATCC deposit no. PTA-4380; see copending US Patent Application Serial No. _, entitled "EphA2 Agonistic Monoclonal Antibodies and Methods of Use Thereof filed May 12, 2003 as Attorney Docket No. 10271-107-999) from binding to EphA2. Briefly, the ability of labeled EA2 to bind EphA2-Fc was assayed by competitive ELISA in presence of either unlabeled EA2 or unlabeled Eph099B-208.261 (FIG. 1). Both antibodies could decrease the amount of labeled EA2 binding to EphA2-Fc with increasing concentrations of unlabeled antibody added. Additionally, many of the other antibodies could inhibit EA2 binding to EphA2 as well (Table 6, column 3).

### 6.2 EphA2 Monoclonal Antibodies Decrease Metastatic Properties of Tumor Cells

#### 6.2.1 EphA2 Phosphorylation and Degradation

EphA2 antibodies promoted tyrosine phosphorylation and degradation of EphA2 in MDA-MB-231 cells. Monolayers of cells were incubated in the presence of EphA2 agonistic antibodies or control at 37°C. Cell lysates were then immunoprecipitated with an EphA2-specific antibody (D7, purchased from Upstate Biologicals, Inc., Lake Placid, NY and deposited with the American Type Tissue Collection on December 8, 2000, and assigned ATCC number PTA 2755), resolved by SDS-PAGE and subjected to western blot analysis with a phosphotyrosine-specific antibody (PY20 or 4G10, purchased from Upstate Biologicals, Inc., Lake Placid, NY). Eph099B-208.261, EA2 (FIGS. 2A-2B), and Eph099B-233.152 (FIG. 4A) increased EphA2 phosphorylation. Some membranes were stripped and re-probed with the EphA2-specific antibody used in the immunoprecipitation (D7) as a loading control (FIGS. 2C-2D). Additionally, other EphA2 antibodies of the invention were also found to increase EphA2 phosphorylation (Table 6, column 5) including Eph099B-102.147 and Eph099B-210.248 (data not shown).

Monolayers of MDA-MB-231 cells were incubated in the presence of EphA2 agonistic antibodies at 37°C. Cell lysates were then resolved by SDS-PAGE and subjected to western blot analysis with an EphA2-specific antibody (D7). Eph099B-208.261, EA2 (FIGS. 3A-3B), and Eph099B-233.152 (FIG. 4B) decreased EphA2 protein level. Some membranes were stripped and re-probed with a β-catenin-specific antibody as a loading control (FIGS. 3C-3D). Additionally, other EphA2 antibodies of the invention were also found to decrease EphA2 protein levels 4 hours and/or 24 hours after antibody treatment (Table 6, columns 6 and 7) including Eph099B-102.147 and Eph099B-210.248 (data not shown). Decreased EphA2 expression is due, in part, to deceased mRNA expression levels in response to EphA2 protein degradation caused by agonistic antibody binding (data not shown).

Western blot analyses and immunoprecipitations were performed as described previously (Zantek et al., 1999, Cell Growth Diff. 10:629-38, which is incorporated by reference in its entirety). Briefly, detergent extracts of cell monolayers were extracted in Tris-buffered saline containing 1% Triton X-100 (Sigma, St. Louis, MO). After measuring protein concentrations (BioRad, Hercules, CA), 1.5 mg of cell lysate was immunoprecipitated, resolved by SDS-PAGE and transferred to nitrocellulose (PROTRAN™, Schleicher and Schuell, Keene, NH). Antibody binding was detected by enhanced chemiluminescence (Pierce, Rockford, IL) and autoradiography (Kodak X-OMAT; Rochester, NY).

#### 6.2.2 Growth in Soft Agar

The ability of the antibodies of the invention to inhibit cancer cell formation in soft agar was assayed as described in Zelinski et al. (2001, Cancer Res. 61:2301-6). Briefly, cells were suspended in soft agar for 7 days at 37°C in the presence of purified antibody or control solution (PBS). Antibodies were administered at the time of suspension in both bottom and top agar solutions. Colony formation was scored microscopically using an Olympus CK-3 inverted phase-contrast microscope outfitted with a 40x objective. Clusters containing at least three cells were scored as a positive. Both Eph099B-208.261 and EA2 inhibited colony growth in soft agar (FIG. 5). Additionally, other antibodies of the invention can inhibit colony formation in soft agar (Table 6, column 9) including Eph099B-102.147 and Eph099B-210.248 (data not shown).

The ability of the antibodies of the invention to eliminate cancer cell colonies already formed in soft agar was assayed. Assay methods were similar to those described above except that antibodies were not added to the cancer cells until the third day of growth in soft agar. Some of the antibodies of the invention can kill cancer cells already growing in colonies in soft agar while other antibodies can slow or reduce cancer cell colony growth in soft agar (Table 6, column 10) including Eph099B-102.147, Eph099B-208.261, Eph099B-210.248, and Eph099B-233.152 (data not shown).

#### 6.2.3 Tubular Network Formation in MATRIGEL™

Tumor cell behavior within a three-dimensional microenvironment, such as MATRIGEL™, can reliably predict the differentiation state and aggressiveness of breast epithelial cells. Monolayer cultures of benign (MCF-10A) or malignant (MDA-MB-231) breast epithelial cells are incubated on MATRIGEL™ in the presence of EphA2 antibodies (10 μg/ml) or control solution (PBS). The behavior of cells on MATRIGEL™ is analyzed as described in Zelinski et al. (2001, Cancer Res. 61:2301-6). Briefly, tissue culture dishes are coated with MATRIGEL™ (Collaborative Biomedical Products, Bedford, MA) at 37°C before adding 1 x $10^5$ MDA-MB-231 or MCF-10A cells previously incubated on ice for 1 hour with the EphA2 antibody or control solution (PBS). Cells are incubated on MATRIGEL™ for 24 hours at 37°C, and cell behavior is assessed using an Olympus IX-70 inverted light microscope. All images are recorded onto 35 mm film (T-Max-400. Kodak, Rochester, NY).

### 6.2.4 Growth *in vivo*

The ability of the antibodies of the invention to inhibit tumor cancer growth *in vivo* was assayed. Eph099B-233.152 can inhibit tumor cell growth *in vivo* and extend survival time of tumor-bearing mice. Briefly, $5x10^6$ MDA-MB-231 breast cancer cells were implanted subcutaneously into athymic mice. After the tumors had grown to an average volume of $100mm^3$, mice were administered 6mg/ml Eph099B-233.152 or PBS control intraperitoneally twice a week for 3 weeks. Tumor growth was assessed and expressed as a ratio of the tumor volume divided by initial tumor volume (100 $mm^3$). After 30 days, mice administered Eph099B-233.152 had smaller tumors than mice administered PBS (FIG. 6A). Tumor growth was allowed to proceed until tumor volume reached $1000mm^3$. Survival of the mice was assessed by scoring the percent of mice living each day post treatment. A greater percentage of mice survived at each time point examined in the group administered Eph099B-233.152 (FIG. 6B). By day 36, all of the mice in the control group had died in contrast with only 70% of the mice admixture Eph099B-233.152.

Additionally, EA2 and Eph099B-208.261 can also inhibit tumor cell growth *in vivo.* $5x10^6$ MDA-MB-231 breast cancer cells were implanted orthotopically or subcutaneously and $5x10^6$ A549 lung cancer cells were implanted subcutaneously into athymic mice. After the tumors had grown to an average volume of $100mm^3$, mice were administered 6mg/kg of an EphA2 agonistic antibody or negative control (PBS or 1A7 antibody) intraperitoneally twice a week for 3 weeks. Animals were generally sacrificed at least two weeks after the last treatment or when tumors exceeded 2000 $mm^3$. Tumor growth was assessed and expressed either as a ratio of the tumor volume divided by initial tumor volume (100 $mm^3$) or as total tumor volume. Growth of MDA-MB-231 cells implanted orthotopically was inhibited by EA2 (FIG. 7A). Growth of MDA-MB-231 cells implanted subcutaneously was inhibited by EA2, Eph099B-208.261, and Eph099B-233.152 (FIG. 7B, D). Growth of A549 cells implanted subcutaneously was inhibited by EA2, or Eph099B-208.261 (FIG. 7C).

### 6.3 Estrogen Dependence in Breast Cancer Cells

Estrogen-sensitive breast cancer cells, MCF-7 cells, were transfected with and stably overexpressed human EphA2 (MCF-$7^{EphaA2}$) (pNeoMSV-EphA2 provided by Dr. T. Hunter, Scripps Institute). Western blot analyses confirmed the ectopic overexpression of EphA2 in transfected cells relative to matched controls (data not shown).

EphA2 overexpression increased malignant growth (FIGS. 8A-8B). Growth assays were conducted as follows. MCF-$7^{neo}$ (control cells) or $MCF7^{EphA2}$ cells were seeded in 96-well plates. Cell growth was measured with Alamar blue (Biosource International, Camarillo, CA) following the manufacture's suggestion. Colony formation in soft agar was performed as previously described (Zelinski et al., 2001, Cancer Res. 61:2301-6) and scored microscopically, defining clusters of at least three cells as a positive. The data represent the average of ten separate high-power microscopic fields from each sample and representative of at least three separate experiments. Error bars represent the standard error of the mean of at least three different experiments as determined using Microsoft Excel software.

Although MCF-7 control cells were largely unable to colonize soft agar (an average of 0.1 colony/field), MCF-$7^{EphA2}$ cells formed larger and more numerous colonies (4.7 colonies/field; $P < 0.01$) that persisted for at least three weeks (FIG. 8A and data not shown). Despite increased colonization of soft agar, the growth of MCF-$7^{EphA2}$ cells in monolayer culture did not differ from matched controls (FIG. 8B), thus indicating that the growth promoting activities of EphA2 were most apparent using experimental conditions that model anchorage-independent (malignant) cell growth.

Consistent with increased soft agar colonization, orthotopically implanted MCF-7 $^{EphA2}$ cells formed larger, more rapidly growing tumors *in vivo.* Six to eight week-old athymic (*nu*/*nu*) mice were purchased from Harlan Sprague Dawley (Indianapolis, IN). When indicated, a controlled release estradiol pellet (0.72 mg 17β-estradiol, 60-day formulation) was injected subcutaneously via a sterile 14-gauge trocar 24 hours prior to tumor implantation and pellets were replaced every 60 days for those experiments spanning > 60 days in duration. $1x10^6$ MCF-$7^{neo}$ or $MCF7^{EphA2}$ cells were injected into the mammary fat pad under direct visualization. When indicated, tamoxifen (1 mg) was administered by oral gavage 6 days per week.

In the presence of supplemental estrogen (17β-estradiol purchased from Stigma), the MCF-$7^{EphA2}$ cells demonstrated a two-fold increase in tumor volume relative to matched controls (FIG. 9A). EphA2-overexpressing tumors differed phenotypically from control tumors in that they were more vascular and locally invasive at the time of resection (data not shown). To confirm that these tumors expressed EphA2, whole cell lysates of resected tumors were subjected to western blot analyses with EphA2-specific antibodies (FIG. 9B). The membranes were then stripped and reprobed with β-catenin antibodies to verify equal sample loading. The relative amount of EphA2 was higher in tumor samples than in the input cells (prior to implantation), suggesting that tumors arose from cells with high levels of EphA2. Comparable findings with *in vitro* and *in vivo* models indicate that EphA2 overexpression results in a more aggressive phenotype.

Parallel studies were performed in the absence of exogenous estrogen. Experimental deprivation of estrogen amplified differences between the cellular behaviors of control and MCF -$7^{EphA2}$ cells. While MCF-$7^{EphA2}$ cells continued to colonize soft agar more efficiently than matched controls (FIG. 10A), these cells did grow in the absence of exogenous estrogen (FIG. 10B). In contrast, supplemental estrogen was required for monolayer growth of control cells (FIG. 10B). Additionally, MCF-$7^{EphA2}$ cells retained tumorigenic potential in the absence of supplemental estrogen. While control MCF-7 cells rarely formed palpable tumors, the MCF-$7^{EphA2}$ cells formed tumors that persisted for over 12 weeks (FIG. 10C and data not shown). Thus, both *in vitro* and *in vivo* assay systems confirm that EphA2 overexpression decreases the need for exogenous estrogen.

Sensitivity of MCF -$7^{EphA2}$ cells to tamoxifen was measured. Tamoxifen (4-hydroxy tamoxifen purchased from Sigma) reduced soft agar colonization of control MCF-7 cells by at least 60%. The inhibitory actions of tamoxifen on MCF-$7^{EphA2}$ cells were less pronounced (25% inhibition, FIG. 11A). Notably, excess estradiol overcame the inhibitory effects of tamoxifen, which provided additional evidence for the specificity of this finding (FIG. 11A). Similarly, the tumorigenic potential ofMCF-$7^{EphA2}$ cells was less sensitive to tamoxifen as compared with control (MCF-$7^{neo}$) cells (FIG. 11B).

Since tamoxifen sensitivity often relates to estrogen receptor expression, estrogen receptor expression and activity was assayed in MCF-$7^{EphA2}$. Western blot analyses revealed comparable levels of ER$\alpha$ and ER$\beta$ in control and MCF-$7^{EphA2}$ cells (FIGS. 12A-12B) (ER$\alpha$ and ER$\beta$ antibodies were purchased from Chemicon, Temecula, CA). Moreover, comparable levels of estrogen receptor activity were detected in control and MCF-7 $^{EphA}$2 cells and this enzymatic activity remained sensitive to tamoxifen (FIGS. 12E-12F). Estrogen receptor activity was measured using ERE-TK-CAT vector (which encodes a single ERE; a generous gift from Dr. Nakshatri, Indiana University School of Medicine) in the unstimulated state, after estradiol ($10^{-8}$ M) stimulation and tamoxifen ($10^{-6}$ M) inhibition. Cells were plated in phenol red free, charcoal stripped sera for 2 days and transfected with ERE-TK-CAT (5 $\mu$g) using calcium phosphate method. The $\beta$ galactosidase expression vector RSV/$\beta$-galactosidase (2 $\mu$g, Dr. Nakshatri's gift) was cotransfected as a control. Fresh media including the appropriate selection drugs were added 24 hours after transfection. Cells were harvested after 24 hours and CAT activity was evaluated as described (Nakshatri et al., 1997, Mol. Cell. Biol. 17:3629-39). These results indicate that the estrogen receptor in MCF-$7^{EphA2}$ cells is expressed and remains sensitive to tamoxifen, thus suggesting that the defect which renders MCF-$7^{EPA2}$ less dependent on estrogen lies downstream of estrogen signaling.

Growth MCF-$7^{EphA2}$ cells which had decreased EphA2 expression levels was assayed in soft agar. The EphA2 monoclonal antibody EA2 induced EphA2 activation and subsequent degradation. Decreased levels of EphA2 expression were observed within two hours of EA2 treatment and EphA2 remained undetectable for at least the following 24 hours (FIG. 13A). The soft agar colonization of control MCF-7 cells was sensitive to tamoxifen (FIG. 13C) and EA2 did not further alter this response (since these cells lack of endogenous EphA2). The MCF-$7^{EphA2}$ cells were less sensitive to tamoxifen (25% inhibition by tamoxifen) as compared to the matched controls (75% inhibition by tamoxifen). Whereas EA2 decreased soft agar colonization (by 19%), the combination of EA2 and tamoxifen caused a much more dramatic (>80%) decrease in soft agar colonization. Thus, EA2 treatment restored a phenotype that was comparable to control MCF-7 cells. These findings suggest that antibody targeting of EphA2 can serve to re-sensitize the breast tumor cells to tamoxifen.

All statistical analyses were performed using Student's t-test using Microsoft Excel (Seattle, WA), defining $P \leq 0.05$ as significant. *In vivo* tumor growth analyses were performed using GraphPad Software (San Diego, CA).

### 6.4 Expression of EphA2 in Prostatic Intraepithelial Neoplasia

EphA2 immunoreactivity distinguished neoplastic prostatic epithelial cells from their non-neoplastic counterparts. Ninety-three cases of radical retropubic prostatectomy were obtained from the surgical pathology files of Indiana University Medical Center. Patients ranged in age from 44 to 77 years (mean = 63 years). Grading of the primary tumor from radical prostatectomy specimens was performed according to the Gleason system (Bostwick "Neoplasms of the prostate" in Bostwick and Eble, eds., 1997, Urologic Surgical Pathology St. Louis:Mosby page 343-422; Gleson and Mellinger, 1974, J. Urol. 111:58-64). The Gleason grade ranged from 4 to 10. Pathological stage was evaluated according to the 1997 TNM (tumor, lymph nodes, and metastasis) standard (Fleming et al., 1997, AJCC Cancer Staging Manual. Philadelphia:Raven and Lippincott). Pathological stages were T2a (n= 9 patients), T2b (n= 43), T3a (n= 27), T3b (n=14). Thirteen patients had lymph node metastasis at the time of surgery.

Serial 5 $\mu$m-thick sections of formalin-fixed slices of radical prostatectomy specimens were used for immunofluorescent staining. Tissue blocks that contained the maximum amount of tumor and highest Gleason grade were selected. One representative slide from each case was analyzed. Slides were deparaffinized in xylene twice for 5 minutes and rehydrated through graded ethanols to distilled water. Antigen retrieval was carried out by heating sections in EDTA (pH 8.0) for 30 minutes. Endogenous peroxidase activity was inactivated by incubation in 3% $H_2O_2$ for 15 minutes. Non-specific binding sites were blocked using Protein Block (DAKO) for 20 minutes. Tissue sections were then incubated with a mouse monoclonal antibody against human EphA2 (IgG1, 1:100 dilution) overnight at room temperature, followed by biotinylated secondary antibody (DAKO corporation, Carpintera, CA) and peroxidase-labeled streptavidin, and 3,3-diaminobenzidine was used as the chromogen in the presence of hydrogen peroxide. Positive and negative controls were run in parallel with each batch.

The extent and intensity of staining were evaluated in benign epithelium, high-grade prostatic intraepithelial neoplasia (PIN) and adenocarcinoma from the same slide for each case. Microscopic fields with highest degree of immunoreactivity were chosen for analysis. At least 1000 cells were analyzed in each case. The percentage of cells exhibiting staining in each case was evaluated semiquantitatively on a 5% incremental scale ranging from 0 to 95%. A numeric intensity score is set from 0 to 3 (0, no staining; 1 weak staining; 2 moderate staining; and 3, strong staining) (Jiang et al., 2002, Am. J. Pathol. 160:667-71; Cheng et al., 1996, Am J. Pathol. 148:1375-80).

The mean percentage of immunoreactive cells in benign epithelium, high-grade PIN and adenocarcinoma were compared using the Wilcoxon paired signed rank test. The intensity of staining for EphA2 in benign epithelium, high-grade PIN, and adenocarcinoma was compared using Cochran-Mantel-Haenszel tests for correlated ordered categorical data. Pairwise comparisons were made if the ANOVA revealed significant differences. A p-value<0.05 was considered significant, and all p-values were two-sided.

EphA2 immunoreactivity was observed in all cases of high-grade prostatic intraepithelial neoplasia (PIN) and cancers but not in benign epithelial cells. For example, EphA2 expression (both the mean percentage of immunoreactive cells and staining intensity) was increased in both high-grade PIN and cancers relative to benign epithelial cells (Tables 3 and 4). Similarly, EphA2 immunoreactivity (both the mean percentage of immunoreactive cells and staining intensity) was increased in prostatic carcinomas compared with high-grade PIN (Tables 3 and 4). This immunoreactivity was evident at the membrane and cytoplasm of the neoplastic epithelial cells (data not shown). In contrast, no EphA2 immunoreactivity was observed in tumor-proximal stromal cells. In the high-grade PIN group, 22% showed grade 1 staining intensity, 73% showed grade 2 staining intensity, and 5% showed grade 3 staining intensity (Table 3). In the adenocarcinoma group, 13% of cases showed grade 1 staining intensity, 50% showed grade 2 staining intensity, and 37% showed grade 3 staining intensity. In contrast, the normal epithelium group showed grade 1 stain in 66% of the cases, the remaining cases showed no immunoreactivity for EphA2 protein (grade 0 staining intensity) (Table3). The mean percentage of EphA2 immunoreactive cells was 12% in the normal epithelial cells, 67% in the high-grade PIN, and 85% in the prostatic adenocarcinoma (Table 4).

Although high levels of EphA2 could distinguish neoplastic from benign prostatic epithelial cells, EphA2 did not correlate with other histologic and pathologic parameters of disease severity. For example, high levels of EphA2 were observed in most prostatic carcinomas and did not relate to Gleason grade, pathologic stage, lymph node metastasis, extraprostatic extension, surgical margins, vascular invasion, perineural invasion or the presence of other areas of the prostate with high-grade PIN (Table 5).

**TABLE 3**

| Cell Type | Staining Intensity Grade | | | |
|---|---|---|---|---|
| | 0 | 1 | 2 | 3 |
| Benign epithelium | 31(33%) | 61(66%) | 1 (1%) | 0 (0%) |
| High-grade PIN[a] | 0 (0%) | 20 (22%) | 68 (73%) | 5 (5%) |
| Adenocarcinoma[a,b] | 0 (0%) | 12 (13%) | 47(50%). | 34 (37%) |

[a]Indicates percentage of staining intensity was statistically lower compared to that of the normal cells with a P-value = 0.0001 using a Wilcoxon paired signed rank test.
The staining intensity was significantly higher compared to high-grade PIN (P<0.01, Cochran-Mantel-Henszel test).

**TABLE 4**

| Cell Type | Mean % of Cells Staining ± SD | Range (%) |
|---|---|---|
| Normal Cells | 12 ± 17 | 0 - 90 |
| High-grade PIN | 67 ± 18[a] | 5 - 95 |

(continued)

| Cell Type | Mean % of Cells Staining ± SD | Range (%) |
|---|---|---|
| Adenocarcinoma | 85 ± 12[a,b] | 30 - 95 |
| [a]Indicates percentage of staining statistically lower compared to that of the normal cells with a P-value = 0.0001 using a Wilcoxon paired signed rank test. [b]The percentage of staining was statistically higher compared to high-grade PIN (P<0.01, ANOVA). | | |

**TABLE 5**

| Patient Characteristic | | % of Total Patients (n=93) | Mean % of Cells Staining w/EphA2 Antibody (±SD) | Mean EphA2 Antibody Staining Intensity (±SD) |
|---|---|---|---|---|
| Primary Gleason Grade | | | | |
| | 2 | 12 | 83±2 | 2.0±0.6 |
| | 3 | 43 | 86±10 | 2.3±0.7 |
| | 4 | 23 | 84±16 | 2.3±0.7 |
| | 5 | 15 | 86±11 | 2.3±0.6 |
| Secondary Gleason Grade | | | | |
| | 2 | 15 | 82±16 | 2.3±0.5 |
| | 3 | 29 | 85±15 | 2.1±0.6 |
| | 4 | 35 | 85±9 | 2.3±0.7 |
| | 5 | 14 | 88±8 | 2.4±0.8 |
| Gleason Sum | <7 | 28 | 83±12 | 2.2±0.6 |
| | 7 | 35 | 85±14 | 2.2±0.7 |
| | >7 | 30 | 87±10 | 2.4±0.7 |
| T Classification | T2a | 9 | 89±6 | 2.3±0.5 |
| | T2b | 43 | 84±12 | 2.2±0.7 |
| | T3a | 27 | 84±15 | 2.2±0.7 |
| | T3b | 14 | 63±10 | 2.4±0.6 |
| Lymph Node Metastasis | | | | |
| | Positive | 13 | 88±9 | 2.3±0.6 |
| | Negative | 80 | 84±13 | 2.2±0.7 |
| Extraprostatic Extension | | | | |
| | Positive | 53 | 86±11 | 2.3±0.7 |
| | Negative | 40 | 84±14 | 2.2±0.7 |
| Surgical Margin | Positive | 50 | 86±11 | 2.1±0.6 |
| | Negative | 43 | 84±13 | 2.4±0.7 |
| Vascular Invasion | Positive | 30 | 85±11 | 2.1±0.8 |
| | Negative | 63 | 86±13 | 2.3±0.6 |
| Perineural Invasion | Positive | 82 | 82±15 | 2.4±0.5 |
| | Negative | 11 | 85±12 | 2.2±0.7 |
| High-grade PIN | Positive | 89 | 85±12 | 2.3±0.7 |
| | Negative | 4 | 85±9 | 2.0±0.8 |

**6.5 Treatment Of Patients With Metastatic Cancer**

A study is designed to assess pharmacokinetics and safety of monoclonal antibodies of the invention in patients with metastatic breast cancer. Cancer patients currently receive Taxol or Taxotere. Patients currently receiving treatment are permitted to continue these medications.

Patients are administered a single IV dose of a monoclonal antibody of the invention and then, beginning 4 weeks later, are analyzed following administration of repeated weekly IV doses at the same dose over a period of 12 weeks. The safety of treatment with the monoclonal antibody of the invention is assessed as well as potential changes in disease activity over 26 weeks of IV dosing. Different groups of patients are treated and evaluated similarly but receive doses of 1 mg/kg, 2 mg/kg, 4 mg/kg, or 8 mg/kg.

Monoclonal antibodies of the invention are formulated at 5 mg/ml and 10 mg/ml for IV injection. A formulation of 80 mg/ml is required for repeated subcutaneous administration. The monoclonal antibodies of the invention are also formulated at 100 mg/ml for administration for the purposes of the study.

Changes are measured or determined by the progression of tumor growth.

### 6.6 Decreased EuhA2 Levels Using EphA2 Antisense Oligonucleotides

An antisense oligonucleotide-based approach that decreased EphA2 expression in tumor cells independent of EphA2 activation was developed. To decrease EphA2 protein levels, MDA-MB-231 breast carcinoma cells were transiently transfected with phosphorothioate-modified antisense oligonucleotides that corresponded to a sequence that was found to be unique to EphA2 as determined using a sequence evaluation of GenBank (5'-CCAGCAGTACCGCTTCCTTGCCCTGCGGCCG-3'; SEQ ID NO:49). Inverted antisense oligonucleotides (5'-GCCGCGTCCCGTTCCTTCACCATGACGACC-3'; SEQ ID NO:50) provided a control. The cells were transfected with oligonucleotides (2 μg/ml) using Lipofectamine PLUS Reagent (Life Technologies, Inc.) according to the manufacturer's protocol. Twenty-four hours post-transfection, the cells were divided. Half of the cells were seeded into soft agar, and the remaining cells were extracted and subjected to western blot analysis.

Western blot analyses and immunoprecipitations were performed as described previously (Zantek et al., 1999, Cell Growth Diff. 10:629-38). Briefly, detergent extracts of cell monolayers were extracted in Tris-buffered saline containing 1% Triton X-100 (Sigma, St. Louis, MO). After measuring protein concentrations (BioRad, Hercules, CA), 1.5 mg of cell lysate was immunoprecipitated, resolved by SDS-PAGE and transferred to nitrocellulose (PROTRAN™, Schleicher and Schuell, Keene, NH). EphA2 was detected with an EphA2-specific antibody (D7, purchased from Upstate Biologicals, Inc., Lake Placid, NY). To control for sample loading, the membranes were stripped and re-probed with paxillin antibodies (a gift from Dr. K. Burridge at the University of North Carolina). Antibody binding was detected by enhanced chemiluminescence (Pierce, Rockford, IL) and autoradiography (Kodak X-OMAT; Rochester, NY).

Western blot analyses confirmed that antisense oligonucleotides selectively decreased EphA2 expression in MDA-MB-231 cells whereas an inverted antisense control (IAS) did not (FIGS. 14A-14B).

MDA-MB-231 cells were suspended in soft agar. Colony formation in soft agar was performed as described in Zelinski et al. (2001, Cancer Res. 61:2301-6, which is incorporated by reference in its entirety). Antibodies or a control solution (PBS) was included in bottom and top agar solutions. Colony formation was scored microscopically using an Olympus CK-3 inverted phase-contrast microscope outfitted with a 40x objective. Clusters containing at least three cells were scored as a positive. The average number of colonies per high-powered field is shown. Ten separate high-power microscopic fields were averaged in each experiment, and the results shown are representative of at least three separate experiments.

EphA2 antisense oligonucleotides decreased soft agar colonization by at least 60% as compared to matched controls (FIG. 14C). Consistent results with EphA2 antibodies and antisense oligonucleotides thus indicate that decreased EphA2 expression is sufficient to decrease tumor cell growth.

### 6.7 Kinetic Analysis of EphA2 Antibodies

The surface plasmon resonance-based BIACORE™ assay was used to measure the $K_{off}$ rates of the monoclonal antibodies of the invention. IgG present in the hybridoma supernatant was used for measurement. Antibodies with $K_{off}$ rates of approximately less than 3 X $10^{-3}$ $s^{-1}$ have slow $K_{off}$ rates. Antibodies with $K_{off}$ rates of approximately 8 X $10^{-4}$ $s^{-1}$ or less have very slow $K_{off}$ rates. Antibodies with $K_{off}$ rates of approximately 9 X $10^{-5}s^{-1}$ or less have ultra slow $K_{off}$ rates.

Immobilization of EphA2

EphA2-Fc was immobilize to a surface on a CM5 sensorchip using a standard amine (70μl of a 1:1 mix of NHS/EDC) coupling chemistry. Briefly, a 400nM solution of EphA2-Fc in 10mM NaOAc, pH4, was then injected over the activated surface to a density of 1000-1100 RU's. Unused reactive esters were subsequently "capped" with a 70μl injection of 1M Et-NH2. Similarly, an activated and "capped" control surface was prepared on the same sensor chip without protein to serve as a reference surface.

Binding Experiments

A 250 μl injection of each of the EphA2 hybridoma supernatants was made over both the EphA2-Fc and control surfaces, and the binding responses were recorded. These supernatants were used undiluted. Following each injection, at least 10 min. of dissociation phase data was collected. Purified EphA2 monoclonal antibody EA2 was prepared to serve as a positive control (at 1μg, 5μg and 25μg per 250μl of growth medium). A negative control monoclonal antibody that does not bind EphA2 was also prepared at 5μg/250μl growth medium. Control injections of growth medium across these surfaces were also made. Following each binding cycle, the EphA2-Fc surface was regenerated with a single 1 min. pulse (injection) of 1M NaCl-50mM NaOH.

Data Evaluation

The binding data was corrected by subtracting out both artifactual noise (blank medium injections) and non-specific binding (control surface), in a technique known as "double-referencing." Thus the sensorgram overlays represent "net" binding curves. Eph099B-208.261 and Eph099B-233.152 (see Table 6) have slower $K_{off}$ rates than EA2 (FIG. 15). Additionally, other antibodies of the invention have slow $K_{off}$ rates (Table 6, column 8) including Eph099B-102.147 and Eph099B-210.248 (data not shown).

Table 6 summarizes the characterization of EphA2 monoclonal antibodies as described herein.

**Table 6**

| ***Clone*** | ***Subclone*** | ***Specificity*** | | ***EphA2 Phosphorylation*** | ***EphA2 Degradation 4hrs Soft*** | ***EphA2 Degradation24 hrs*** | ***Off Rate*** | ***Colony Inhibition in*** **Soft Agar** ***Agar*** | ***Colony Elimination in*** **Soft Agar** ***Binds*** |
|---|---|---|---|---|---|---|---|---|---|
| | | **Inhibits EA2 Binding** | **Binds EphA2** | | | | | | |
| ***A-Group*** | | | | | | | | | |
| **101** | | yes | yes | nd | moderate | nd | very slow | nd | nd |
| **102** | | yes | yes | nd | low-mod | nd | very slow | nd | nd |
| **201** | | yes | yes | nd | no | nd | slow | nd | nd |
| | | | | | | | | | |
| ***B-Group*** | | | | | | | | | |
| **101** | | nd | yes | weak | moderate | no | nd | strong | nd |
| **102** | | yes | yes | yes | Strong | strong | ultra slow | strong | mod-strong |
| **103** | | yes | yes | weak | Strong | strong | nd | moderate-strong | nd |
| **108** | | nd | yes | nd | low-mod | nd | nd | strong | nd |
| **201** | | yes | yes | nd | no | nd | very slow | strong | low |
| **203** | | yes | yes | nd | low-mod | nd | nd | strong | nd |
| **204** | | yes | yes | strong | strong | strong | nd | none | moderate |
| **208** | | yes | yes | yes | strong | nd | nd | moderate | moderate |
| | **103** | | | nd | strong | strong | nd | nd | strong |
| | **108** | | | nd | strong | nd | nd | nd | nd |
| | **117** | | | nd | strong | strong | nd | nd | very strong |
| | **177** | | | nd | strong | nd | nd | nd | nd |
| | **205** | | | nd | strong | no | nd | nd | nd |
| | **222** | | | nd | strong | nd | nd | nd | nd |

(continued)

| *Clone* | *Subclone* | *Specificity* | | *EphA2 Phosphorylation* | *EphA2 Degradation 4hrs Soft* | *EphA2 Degradation24 hrs* | *Off Rate* | *Colony Inhibition in* Soft Agar *Agar* | *Colony Elimination in* Soft Agar *Binds* |
|---|---|---|---|---|---|---|---|---|---|
| | | **Inhibits EA2 Binding** | **Binds EphA2** | | | | | | |
| | **234** | | | nd | strong | nd | nd | nd | nd |
| | **235** | | | nd | strong | moderate | nd | nd | nd |
| | **238** | | | nd | strong | nd | nd | nd | nd |
| **209** | | nd | yes | nd | low | nd | nd | strong | nd |
| **210** | | yes | yes | yes | strong | no | nd | strong | moderate |
| **211** | | no | yes | nd | no | nd | moderate | strong | nd |
| **219** | | yes | yes | nd | low | nd | slow | strong | nd |
| **220** | | yes | yes | nd | no | nd | ultra slow | strong | very strong |
| **221** | | yes | yes | nd | no | nd | ultra slow | strong | very strong |
| **223** | | yes | yes | strong | strong | moderate | slow | none | moderate |
| **229** | | yes | yes | nd | no | nd | very slow | strong | nd |
| **230** | | yes | yes | nd | no | nd | very slow | strong | nd |
| **231** | | yes | yes | yes | strong | no | very slow | strong | moderate |
| **233** | | yes | yes | weak | strong | strong | very slow | none | moderate |
| **301** | | no | yes | nd | no | nd | very slow | strong | none |
| **302** | | no | yes | nd | low | nd | nd | strong | nd |
| **307** | | no | yes | weak | moderate | no | slow | strong | nd |
| **308** | | no | yes | nd | low | nd | nd | strong | nd |
| **309** | | yes | yes | nd | no | nd | ultra slow | strong | very strong |
| **310** | | nd | yes | nd | no | nd | nd | strong | nd |
| **311** | | yes | yes | nd | low | nd | very slow | strong | nd |

(continued)

| *Clone* | *Subclone* | *Specificity* | | *EphA2 Phosphorylation* | *EphA2 Degradation 4hrs Soft* | *EphA2 Degradation24 hrs* | *Off Rate* | *Colony Inhibition in* **Soft Agar** *Agar* | *Colony Elimination in* **Soft Agar** *Binds* |
|---|---|---|---|---|---|---|---|---|---|
| | | **Inhibits EA2 Binding** | **Binds EphA2** | | | | | | |
| **312** | | no | yes | nd | low-moderate | nd | nd | strong | nd |
| **313** | | yes | yes | nd | low | nd | very slow | strong | nd |
| **314** | | yes | yes | nd | low | nd | ultra slow | strong | moderate |
| **315** | | yes | yes | nd | low | nd | ultra slow | strong | moderate |
| **316** | | yes | yes | nd | no | nd | very slow | strong | nd |
| **317** | | yes | yes | nd | no | nd | slow | strong | nd |
| **401** | | no | yes | nd | no | nd | nd | strong | nd |
| **402** | | nd | yes | nd | low | nd | nd | strong | nd |
| **404** | | nd | yes | yes | moderate | no | nd | nd | nd |
| **406** | | no | yes | nd | no | nd | nd | nd | nd |
| **407** | | no | yes | nd | no | nd | slow | nd | nd |
| **408** | | no | yes | nd | no | nd | slow | nd | nd |
| **409** | | nd | yes | nd | no | nd | nd | nd | nd |
| **410** | | no | yes | strong | moderate | no | fast | nd | nd |

### 6.8 Epitope Analysis of EphA2 Antibodies

The epitopes of EphA2 antibodies were characterized. Non-transformed MCF-10A cells or transformed MDA-MB-231 cells were incubated with 10 μg/ml Eph099B-233.152 or EA2 at 4°C for 30 min. prior to fixation in a 3% formalin solution and immunolabeling with fluorophore-conjugated anti-mouse IgG. EA2 preferentially binds EphA2 on transformed cells (FIG. 16D). In contrast, Eph099B-233.152 binds EphA2 expressed on both transformed and non-transformed cells (FIGS. 16A-16B). Treatment of non-transformed MCF-10A cells with 4mM EGTA for 20 min. dissociated the cells. EA2 bound EphA2 on the EGTA dissociated cells but not the untreated cells (FIGS. 17A-17B).

An equivalent experiment was performed using MCF-10A or MDA-MB-231 cells. The amount of EA2 binding to EphA2 was measured using flow cytometry (FIGS. 17C-17D). Cells were either treated by incubation in 4 mM EGTA for 10-15 minutes on ice (top panel) or were not treated with EGTA (middle panel) before incubation with 10 μg/ml EA2. Cells were then fixed with 3% formalin and labeled with fluorophore-labeled donkey anti-mouse IgG. Control cells were incubated only with secondary antibody (fluorophore-labeled donkey anti-mouse IgG) in the absence of primary antibody (EA2) (bottom panel). The samples were then evaluated using flow cytometry (Becton Dickinson FACStar Plus). EGTA treatment did not affect EA2 binding to transformed cells (FIG. 17D, top and middle panels). In contrast, EA2 binding to non-transformed cells was increased by incubation in EGTA (FIG. 17C, top and middle panels).

A microtiter plate was coated with 10 mg/ml Ephrin A1-$F_c$ overnight at 4°C. A fusion protein consisting of the extracellular domain of EphA2 linked to human $IgG_1$ constant region (EphA2-$F_c$) was incubated with and bound to the immobilized Ephrin A1-$F_c$. Biotinylated Ephrin A1-$F_{c,}$ EA2, or Eph099B-233.152 was incubated with the EphA2-Ephrin A1-$F_c$ complex and amount of binding was measured. Very little additional Ephrin Al-$F_c$ bound the EphA2-Ephrin A1-$F_c$ complex while, in contrast, considerable levels of EA2 and Eph099B-233.152 bound the EphA2-EphrinA1- $F_c$ complex (FIG. 18A).

The EphA2-Ephrin A1-$F_c$ complex was prepared as described above. Biotinylated EA2 (10 μg/ml) was then incubated with the complex for 30 min. Unlabeled competitor was incubated with EphA2-Ephrin A1-$F_c$-EA2 complex in the indicated amount. Unlabeled EA2 could displace the labeled EA2 at concentrations of 100 ng/ml or greater. Unlabeled Eph099B-233.152 and Ephrin A1-$F_c$ were similar in their ability to displace labeled EA2 (FIG 18B).

## 7. EQUIVALENTS

Those skilled in the art will recognize, or be able to ascertain using no more than routine experimentation, many equivalents to the specific embodiments of the invention described herein.

## Claims

**1.** An isolated antibody that specifically binds EphA2 with a $k_{off}$ of less than 3 x $10^{-3}$ $s^{-1}$ and induces phosphorylation of the EphA2, wherein:

a. the antibody competes for binding to EphA2 with the Eph099B-102.147 antibody produced by the hybridoma deposited with American Type Culture Collection (ATCC) and assigned accession no. PTA-4572;
b. the antibody competes for binding to EphA2 with the Eph099B-208.261 antibody produced by the hybridoma deposited with ATCC and assigned accession no. PTA-4573;
c. the antibody competes for binding to EphA2 with the Eph099B-210.248 antibody produced by the hybridoma deposited with ATCC and assigned accession no. PTA-4574;
d. the antibody competes for binding to EphA2 with the Eph099B.233.152 antibody produced by the hybridoma deposited with ATCC and assigned accession no. PTA-5194;
e. the antibody is produced by the hybridoma deposited with the ATCC and assigned accession no. PTA-4572;
f. the antibody is produced by the hybridoma deposited with the ATCC and assigned accession no. PTA-4573;
g. the antibody is produced by the hybridoma deposited with the ATCC and assigned accession no. PTA-4574;

h. the antibody is produced by the hybridoma deposited with the ATCC and assigned accession no. PTA-5194;
i. the antibody comprises a VH chain comprising the amino acid sequence of SEQ ID NO:5 or 21 and a VL chain comprising the amino acid sequence of SEQ ID NO:1 or 17
j. the antibody comprises a VL CDR1 having the amino acid sequence of SEQ ID NO:2 or 18, a VL CDR2 having the amino acid sequence of SEQ ID NO:3 or 19, a VL CDR3 having the amino acid sequence of SEQ ID NO: 4 or 20, a VH CDR having the amino acid of SEQ ID NO: 6 or 22, a VH CDR2 having the amino acid sequence of SEQ ID NO:7 or 23, and a VH CDR3 having the amino acid sequence of SEQ ID NO:8 or 24; or
k. the antibody comprises a VL CDR1 having the amino acid sequence of SEQ ID NO:2 or 18, a VL CDR2 having the amino acid sequence of SEQ ID NO:3 or 19, a VL CDR3 having the amino acid sequence of SEQ ID NO:4 or 20, a VH CDR1 having the amino acid of SEQ ID NO: 6 or 22, a VH CDR2 having the amino acid sequence of SEQ ID NO:7 or 23, and a VH CDR3 having the amino acid sequence of SEQ ID NO:8 or 24, wherein one or more of the CDRs has one, two, or three mutations.

**2.** The antibody of claim 1, wherein the $k_{off}$ is less than 1 x $10^{-3}$ $S^{-1}$.

**3.** The antibody of claim 1 which is produced by a hybridoma deposited with the ATCC having accession number PTA-4572.

**4.** The antibody of claim 1 which is produced by a hybridoma deposited with the ATCC having accession number PTA-4573.

**5.** The antibody of claim 1 which is produced by a hybridoma deposited with ATCC having accession number PTA-4574.

**6.** The antibody of claim 1 which is produced by a hybridoma deposited with ATCC having accession number PTA-5194.

**7.** The antibody of claim 1, wherein the antibody competes for binding to EphA2 with the Eph099B-102.147 antibody produced by the hybridoma deposited with the ATCC and assigned accession no. PTA-457Z.

**8.** The antibody of claim 1 or 2, wherein the antibody competes for binding to EphA2 with the Eph099B-208.261 antibody produced by the hybridoma deposited with the ATCC and assigned accession no. PTA-4573.

**9.** The antibody of claim 1 or 2, wherein the antibody competes for binding to EphA2 with the Eph099B-210.248 antibody produced by the hybridoma deposited with the ATCC and assigned accession no. PTA-4574.

**10.** The antibody of claim 1 or 2, wherein the antibody competes for binding to EphA2 with the Eph099B-233.152 antibody produced by the hybridoma deposited with the ATCC and assigned accession no. PTA-5194.

**11.** The antibody of claim 1, wherein the antibody comprises a variable heavy (VH) chain having the amino acid sequence of SEQ ID NO:5 or 21.

**12.** The antibody of claim 1, wherein the antibody comprises a variable light (VL) chain having the amino acid sequence of SEQ ID NO:1 or 17.

**13.** The antibody of claim 1, wherein the antibody comprises a VH chain comprising the amino acid sequence of SEQ ID NO:5 and a VL chain comprising the amino acid sequence of SEQ ID NO:1.

**14.** The antibody of claim 1, wherein the antibody comprises a VH chain comprising the amino acid of SEQ ID NO:21 and a VL chain comprising the amino acid sequence of SEQ ID NO:17.

**15.** The antibody of claim 1, wherein the antibody comprises a VL CDR1 having the amino acid sequence of SEQ ID NO:2, a VL CDR2 having the amino acid sequence of SEQ ID NO:3, and a VL CDR3 having the amino acid sequence of SEQ ID NO:4.

**16.** The antibody of claim 1, wherein the antibody comprises a VH CDR1 having the amino acid sequence of SEQ ID NO:6, a VH CDR2 having the amino acid sequence of SEQ ID NO:7, and a VH CDR3 having the amino acid sequence of SEQ ID NO:8.

**17.** The antibody of claim 1, wherein the antibody comprises a VL CDR1 having the amino acid sequence of SEQ ID NO:18, a VL CDR2 having the amino acid sequence of SEQ ID NO:19, and a VL CDR3 having the amino acid sequence of SEQ ID NO:20.

**18.** The antibody of claim 1, wherein the antibody comprises a VH CDR1 having the amino acid sequence of SEQ ID NO:22, a VH CDR2 having the amino acid sequence of SEQ ID NO:23, and a VH CDR3 having the amino acid sequence of SEQ ID NO:24.

**19.** The antibody of claim 1, wherein the antibody comprises a VL CDR1 having the amino acid sequence af SEQ ID NO:2, a VL CDR2 having the amino acid sequence of SEQ ID NO:3, a VL CDR3 having the amino acid sequence of SEQ ID NO:4, a VH CDR1 having the amino acid sequence of SEQ ID NO:6, a VH CDR2 having the amino acid sequence of SEQ ID NO:7, and a VH CDR3 having the amino acid sequence of SEQ ID NO:8.

**20.** The antibody of claim 1, wherein the antibody comprises a VL CDR1 having the amino acid sequence of SEQ ID NO:18, a VL CDR2 having the amino acid sequence of SEQ ID NO:9, a VL CDR3 having the amino acid sequence of SEQ ID NO:20, a VH CDR1 having the amino acid sequence of SEQ ID NO:22, a VH CDR2 having the amino acid sequence of SEQ ID NO:23, and a VH CDR3 having the amino acid sequence af SEQ ID NO:24.

**21.** The antibody of any one of claims 1 or 7 to 20, wherein the antibody is a humanized antibody, a Fab fragment, a chimeric antibody, a single chain antibody, a F(ab') fragment, or a disulfide-linked Fv.

**22.** The antibody of claim 1 or 7 to 20, wherein the antibody is a monoclonal antibody.

**23.** An antibody conjugate comprising the antibody of any one of the preceding claims conjugated or fused to a heterologous agent.

**24.** The antibody conjugate of claim 23, wherein the heterologous agent is diagnostic or detectable agent.

**25.** The antibody conjugate of claim 23, wherein the heterologous agent is a cytotoxin.

**26.** The antibody conjugate of claim 23, wherein the heterologous agent is a therapeutic agent or drug moiety.

**27.** An isolated nucleic acid comprising a nucleotide sequence encoding the antibody of any one of claims 1, 3 to 6, 13, 14, 19, or 20.

**28.** A vector comprising the nucleic acid of claim 27.

**29.** A host cell comprising the vector of claim 28.

**30.** A host cell engineered to contain or express the nucleic acid of claim 27.

**31.** A pharmaceutical composition comprising the antibody of any one of claims 1 to 26 and a pharmaceutically acceptable carrier.

**32.** Use of the pharmaceutical composition of claim 31 in the preparation of a medicament for treating cancer in a patient, wherein the cancer is associated with overexpression of EphA2.

**33.** A pharmaceutical composition comprising the antibody conjugate of claim 23, 24, 25, or 26.

**34.** Use of the pharmaceutical composition of claim 33 in the preparation of a medicament for treating cancer in a patient, wherein the cancer is associated with overexpression of EphA2.

**35.** Use of the antibody of any one of claims 1 to 22 in the preparation of a medicament for treating cancer in a patient, wherein the cancer is associated with overexpression of EphA2.

**36.** Use of the antibody conjugate of claim 23, 25, or 26 in the preparation of a medicament for treating cancer in a patient, wherein the cancer is associated with overexpression of EphA2.

**37.** Use of claim 35 or 36, wherein the composition further comprises additional therapy.

**38.** An in vitro method for diagnosing cancer in a patient, comprising:

a. contacting cells from said patient with the antibody of any one of claims 1 to 22 under conditions appropriate for antibody-EphA2 binding; and
b. measuring EphA2 antibody binding to said cells, wherein a higher EphA2 antibody binding level than a control indicates that the patient has cancer.

**39.** Use of the antibody of any one of claims 1 to 22 in the preparation of a composition for diagnosing, prognosing or monitoring the efficacy of therapy for cancer in a patient, wherein the cancer is associated with overexpression of EphA2.

**40.** Use of the antibody conjugate of claim 24 in the preparation of a composition for diagnosing, prognosing or monitoring the efficacy of therapy for cancer in a patient, wherein the cancer is associated with overexpression of EphA2.

**41.** Use of any one of claims 34 to 37, 39, or 40, wherein the cancer is of an epithelial cell origin.

**42.** Use of any one of claims 34 to 37, 39, or 40, wherein the cancer is a metastatic cancer.

**43.** Use of any one of claims 34 to 37 or 39 to 42, wherein the cancer is breast cancer, colon cancer, prostate cancer, melanoma, ovarian cancer, lung cancer, skin cancer, bladder cancer, pancreatic cancer, or a renal cell carcinoma.

**44.** The method of claim 38, wherein the cancer is breast cancer, colon cancer, prostate cancer, melanoma, ovarian cancer, lung cancer, skin cancer, bladder cancer, pancreatic cancer, or a renal cell carcinoma.

**45.** The method of claim 38, wherein the cancer is of an epithelial cell origin.

**46.** The method of claim 38, wherein the cancer is a metastatic cancer.

**47.** The method of claim 38, wherein the cells are from whole blood, sputum, urine, serum, or fine needle aspirates of tumor cell tissue.

**48.** Use of any one of claims 34 to 37, 39, or 40, wherein the patient is a human.

**49.** Use of claim 48, wherein the human is refractory to a first therapy.

**50.** The method of claim 38, wherein the patient is a human.

**51.** A hybridoma deposited with the ATCC having accession number PTA-4572.

**52.** A hybridoma deposited with the ATCC having accession number PTA-4573.

**53.** A hybridoma deposited with the ATCC having accession number PTA-4574.

**54.** A hybridoma deposited with the ATCC having accession number PTA-5194.

**Patentansprüche**

**1.** Isolierter Antikörper, der EphA2 spezifisch mit einem $k_{off}$ von weniger als $3 \times 10^{-3} s^{-1}$ bindet und die Phosphorylierung des EphA2 induziert, wobei:

a. der Antikörper um die Bindung an EphA2 mit dem Eph099B-102.147-Antikörper konkurriert, der durch das Hybridom, das bei der American Type Culture Collection (ATCC) hinterlegt und die Zugangsnummer PTA-4572 zugewiesen bekommen hat, produziert wird;
b. der Antikörper um die Bindung an EphA2 mit dem Eph099B-208.261-Antikörper konkurriert, der durch das Hybridom, das bei der ATCC hinterlegt und die Zugangsnummer PTA-4573 zugewiesen bekommen hat, produziert wird;
c. der Antikörper um die Bindung an EphA2 mit dem Eph099B-210.248-Antikörper konkurriert, der durch das Hybridom, das bei der ATCC hinterlegt und die Zugangsnummer PTA-4574 zugewiesen bekommen hat, produziert wird;
d. der Antikörper um die Bindung an EphA2 mit dem Eph099B-233.152-Antikörper konkurriert, der durch das Hybridom, das bei der ATCC hinterlegt und die Zugangsnummer PTA-5194 zugewiesen bekommen hat, produziert wird;
e. der Antikörper durch das Hybridom, das bei der ATCC hinterlegt und die Zugangsnummer PTA-4572 zugewiesen bekommen hat, produziert wird;
f. der Antikörper durch das Hybridom, das bei der ATCC hinterlegt und die Zugangsnummer PTA-4573 zugewiesen bekommen hat, produziert wird;
g. der Antikörper durch das Hybridom, das bei der ATCC hinterlegt und die Zugangsnummer PTA-4574 zugewiesen bekommen hat, produziert wird;
h. der Antikörper durch das Hybridom, das bei der ATCC hinterlegt und die Zugangsnummer PTA-5194 zugewiesen bekommen hat, produziert wird;
i. der Antikörper eine VH-Kette, die die Aminosäuresequenz von SEQ ID NO: 5 oder 21 umfasst, oder eine VL-Kette, die die Aminosäuresequenz von SEQ ID NO: 1 oder 17 umfasst, umfasst;
j. der Antikörper ein VL CDR1, das die Aminosäuresequenz von SEQ ID NO: 2 oder 18 aufweist, ein VL CDR2, das die Aminosäuresequenz von SEQ ID NO: 3 oder 19 aufweist, ein VL CDR3, das die Aminosäuresequenz von SEQ ID NO: 4 oder 20 aufweist, ein VH CDR1, das die Aminosäuresequenz von SEQ ID NO: 6 oder 22 aufweist, ein VH CDR2, das die Aminosäuresequenz von SEQ ID NO: 7 oder 23 aufweist und ein VH CDR3, das die Aminosäuresequenz von SEQ ID NO: 8 oder 24 aufweist, umfasst; oder
k. der Antikörper ein VL CDR1, das die Aminosäuresequenz von SEQ ID NO: 2 oder 18 aufweist, ein VL CDR2, das die Aminosäuresequenz von SEQ ID NO: 3 oder 19 aufweist, ein VL CDR3, das die Aminosäuresequenz von SEQ ID NO: 4 oder 20 aufweist, ein VH CDR1, das die Aminosäuresequenz von SEQ ID NO: 6 oder 22 aufweist, ein VH CDR2, das die Aminosäuresequenz von SEQ ID NO: 7 oder 23 aufweist und ein VH CDR3, das die Aminosäuresequenz von SEQ ID NO: 8 oder 24 aufweist, umfasst, wobei eines oder mehrere der CDR eine, zwei oder drei Mutationen aufweist/aufweisen.

**2.** Antikörper nach Anspruch 1, wobei das $K_{off}$ weniger als 1 x $10^{-3}s^{-1}$ beträgt.

**3.** Antikörper nach Anspruch 1, der durch ein Hybridom, das bei der ATCC hinterlegt und die Zugangsnummer PTA-4572 zugewiesen bekommen hat, produziert wird.

**4.** Antikörper nach Anspruch 1, der durch ein Hybridom, das bei der ATCC hinterlegt und die Zugangsnummer PTA-4573 zugewiesen bekommen hat, produziert wird.

**5.** Antikörper nach Anspruch 1, der durch ein Hybridom, das bei der ATCC hinterlegt und die Zugangsnummer PTA-4574 zugewiesen bekommen hat, produziert wird.

**6.** Antikörper nach Anspruch 1, der durch ein Hybridom, das bei der ATCC hinterlegt und die Zugangsnummer PTA-5194 zugewiesen bekommen hat, produziert wird.

**7.** Antikörper nach Anspruch 1, wobei der Antikörper um die Bindung an EphA2 mit dem Eph099B-102.147-Antikörper konkurriert, der durch das Hybridom, das bei der ATCC hinterlegt und die Zugangsnummer PTA-4572 zugewiesen bekommen hat, produziert wird.

**8.** Antikörper nach Anspruch 1 oder 2, wobei der Antikörper um die Bindung an EphA2 mit dem Eph099B-208.261-Antikörper konkurriert, der durch das Hybridom, das bei der ATCC hinterlegt und die Zugangsnummer PTA-4573 zugewiesen bekommen hat, produziert wird.

**9.** Antikörper nach Anspruch 1 oder 2, wobei der Antikörper um die Bindung an EphA2 mit dem Eph099B-210.248-Antikörper konkurriert, der durch das Hybridom, das bei der ATCC hinterlegt und die Zugangsnummer PTA-4574 zugewiesen bekommen hat, produziert wird.

**10.** Antikörper nach Anspruch 1 oder 2, wobei der Antikörper um die Bindung an EphA2 mit dem Eph099B-233.152-Antikörper konkurriert, der durch das Hybridom, das bei der ATCC hinterlegt und die Zugangsnummer PTA-5194 zugewiesen bekommen hat, produziert wird;

**11.** Antikörper nach Anspruch 1, wobei der Antikörper eine variable schwere (VH-) Kette umfasst, die die Aminosäuresequenz SEQ ID NO: 5 oder 21 aufweist.

**12.** Antikörper nach Anspruch 1, wobei der Antikörper eine variable leichte (VL-) Kette umfasst, die die Aminosäuresequenz SEQ ID NO: 1 oder 17 aufweist.

**13.** Antikörper nach Anspruch 1, wobei der Antikörper eine VH-Kette, die die Aminosäuresequenz von SEQ ID NO: 5 und eine VL-Kette, die die Aminosäuresequenz von SEQ ID NO: 1 umfasst, umfasst.

**14.** Antikörper nach Anspruch 1, wobei der Antikörper eine VH-Kette, die die Aminosäuresequenz von SEQ ID NO: 21 und eine VL-Kette, die die Aminosäuresequenz von SEQ ID NO: 17 umfasst, umfasst.

**15.** Antikörper nach Anspruch 1, wobei der Antikörper ein VL CDR1, das die Aminosäuresequenz von SEQ ID NO: 2 aufweist, ein VL CDR2, das die Aminosäuresequenz von SEQ ID NO: 3 aufweist und ein VL CDR3, das die Aminosäuresequenz von SEQ ID NO: 4 aufweist, umfasst.

**16.** Antikörper nach Anspruch 1, wobei der Antikörper ein VH CDR1, das die Aminosäuresequenz von SEQ ID NO: 6 aufweist, ein VH CDR2, das die Aminosäuresequenz von SEQ ID NO: 7 aufweist und ein VH CDR3, das die Aminosäuresequenz von SEQ ID NO: 8 aufweist, umfasst.

**17.** Antikörper nach Anspruch 1, wobei der Antikörper ein VL CDR1, das die Aminosäuresequenz von SEQ ID NO: 18 aufweist, ein VL CDR2, das die Aminosäuresequenz von SEQ ID NO: 19 aufweist, und ein VL CDR3, das die Aminosäuresequenz von SEQ ID NO: 20 aufweist, umfasst.

**18.** Antikörper nach Anspruch 1, wobei der Antikörper ein VH CDR1, das die Aminosäuresequenz von SEQ ID NO: 22 aufweist, ein VH CDR2, das die Aminosäuresequenz von SEQ ID NO: 23 aufweist, und ein VH CDR3, das die Aminosäuresequenz von SEQ ID NO: 24 aufweist, umfasst.

**19.** Antikörper nach Anspruch 1, wobei der Antikörper ein VL CDR1, das die Aminosäuresequenz von SEQ ID NO: 2 aufweist, ein VL CDR2, das die Aminosäuresequenz von SEQ ID NO: 3 aufweist, ein VL CDR3, das die Aminosäuresequenz von SEQ ID NO: 4 aufweist, ein VH CDR1, das die Aminosäuresequenz von SEQ ID NO: 6 aufweist, ein VHL CDR2, das die Aminosäuresequenz von SEQ ID NO: 7 aufweist, und ein VH CDR3, das die Aminosäuresequenz von SEQ ID NO: 8 aufweist, umfasst.

**20.** Antikörper nach Anspruch 1, wobei der Antikörper ein VL CDR1, das die Aminosäuresequenz von SEQ ID NO: 18 aufweist, ein VL CDR2, das die Aminosäuresequenz von SEQ ID NO: 19 aufweist, ein VL CDR3, das die Aminosäuresequenz von SEQ ID NO: 20 aufweist, ein VH CDR1, das die Aminosäuresequenz von SEQ ID NO: 22 aufweist, ein VH CDR2, das die Aminosäuresequenz von SEQ ID NO: 23 aufweist, und ein VH CDR3, das die Aminosäuresequenz von SEQ ID NO: 24 aufweist, umfasst.

**21.** Antikörper nach einem der Ansprüche 1 oder 7 bis 20, wobei der Antikörper ein humanisierter Antikörper, ein Fab-Fragment, ein chimärer Antikörper, ein Einkettenantikörper, ein F(ab')-Fragment oder ein Disulfid-verknüpfter Fv ist.

**22.** Antikörper nach Anspruch 1 oder 7 bis 20, wobei der Antikörper ein monoklonaler Antikörper ist.

**23.** Antikörperkonjugat umfassend den Antikörper nach einem der vorhergehenden Ansprüche, der an ein heterologes Agens konjugiert oder fusioniert ist.

**24.** Antikörperkonjugat nach Anspruch 23, wobei das heterologe Agens ein diagnostisches oder erfassbares Agens ist.

**25.** Antikörperkonjugat nach Anspruch 23, wobei das heterologe Agens ein Cytotoxin ist.

**26.** Antikörperkonjugat nach Anspruch 23, wobei das heterologe Agens ein therapeutisches Mittel oder ein Arzneimittelanteil ist.

**27.** Isolierte Nukleinsäure umfassend eine Nukleotidsequenz, die für den Antikörper nach einem der Ansprüche 1, 3 bis 6, 13, 14, 19 oder 20 kodiert.

**28.** Vektor, der die Nukleinsäure nach Anspruch 27 umfasst.

**29.** Wirtszelle, die den Vektor nach Anspruch 28 umfasst.

**30.** Wirtszelle, die so manipuliert ist, dass sie die Nukleinsäure nach Anspruch 27 enthält oder exprimiert.

**31.** Pharmazeutische Zusammensetzung, die den Antikörper nach einem der Ansprüche 1 bis 26 und einen pharmazeutisch akzeptablen Träger umfasst.

**32.** Verwendung der pharmazeutischen Zusammensetzung nach Anspruch 31 bei der Herstellung eines Medikaments für die Behandlung von Krebs bei einem Patienten, wobei der Krebs mit der Überexpression von EphA2 verbunden ist.

**33.** Pharmazeutische Zusammensetzung, die das Antikörperkonjugat nach Anspruch 23, 24, 25 oder 26 umfasst.

**34.** Verwendung der pharmazeutischen Zusammensetzung nach Anspruch 33 bei der Herstellung eines Medikaments für die Behandlung von Krebs bei einem Patienten, wobei der Krebs mit der Überexpression von EphA2 verbunden ist.

**35.** Verwendung des Antikörpers nach einem der Ansprüche 1 bis 22 bei der Herstellung eines Medikaments für die Behandlung von Krebs bei einem Patienten, wobei der Krebs mit der Überexpression von EphA2 verbunden ist.

**36.** Verwendung des Antikörpers nach Anspruch 23, 25 oder 26 bei der Herstellung eines Medikaments für die Behandlung von Krebs bei einem Patienten, wobei der Krebs mit der Überexpression von EphA2 verbunden ist.

**37.** Verwendung nach Anspruch 35 oder 36, wobei die Zusammensetzung des Weiteren zusätzliche Therapie umfasst.

**38.** In vitro-Verfahren zum Diagnostizieren von Krebs bei einem Patienten, umfassend:

a. das Kontaktieren von Zellen des Patienten mit dem Antikörper nach einem der Ansprüche 1 bis 22 unter Bedingungen, die für die Antikörper-EphA2-Bindung geeignet sind; und
b. das Messen der EphA2-Antikörperbindung an die Zellen, wobei ein höheres EphA2- Antikörper-Bindungsniveau als bei einer Kontrolle anzeigt, dass der Patient an Krebs leidet.

**39.** Verwendung des Antikörpers nach einem der Ansprüche 1 bis 22 bei der Herstellung einer Zusammensetzung zum Diagnostizieren, Prognostizieren oder Überwachen der Wirksamkeit von Therapie gegen Krebs bei einem Patienten, wobei der Krebs mit der Überexpression von EphA2 verbunden ist.

**40.** Verwendung des Antikörperkonjugats nach Anspruch 24 bei der Herstellung einer Zusammensetzung zum Diagnostizieren, Prognostizieren oder Überwachen der Wirksamkeit von Therapie gegen Krebs bei einem Patienten, wobei der Krebs mit der Überexpression von EphA2 verbunden ist.

**41.** Verwendung nach einem der Ansprüche 34 bis 37, 39 oder 40, wobei der Krebs aus einem Epithelzellenursprung stammt.

**42.** Verwendung nach einem der Ansprüche 34 bis 37, 39 oder 40, wobei der Krebs ein metastatischer Krebs ist.

**43.** Verwendung nach einem der Ansprüche 34 bis 37 oder 39 bis 42, wobei der Krebs Brustkrebs, Kolonkrebs, Prostatakrebs, Melanom, Eierstockkrebs, Lungenkrebs, Hautkrebs, Blasenkrebs, Bauchspeicheldrüsenkrebs oder Nierenzellkarzinom ist.

**44.** Verfahren nach Anspruch 38, wobei der Krebs Brustkrebs, Kolonkrebs, Prostatakrebs, Melanom, Eierstockkrebs, Lungenkrebs, Hautkrebs, Blasenkrebs, Bauchspeicheldrüsenkrebs oder Nierenzellkarzinom ist.

**45.** Verfahren nach Anspruch 38, wobei der Krebs aus einem Epithelzellenursprung stammt.

**46.** Verfahren nach Anspruch 38, wobei der Krebs ein metastatischer Krebs ist.

**47.** Verfahren nach Anspruch 38, wobei die Zellen aus Vollblut, Speichel, Urin, Serum oder Feinnadelaspiraten von Tumorzellengewebe stammen.

**48.** Verwendung nach einem der Ansprüche 34 bis 37, 39 oder 40, wobei der Patient ein Mensch ist.

**49.** Verwendung nach Anspruch 48, wobei der Mensch einer ersten Therapie unzugänglich ist.

**50.** Verfahren nach Anspruch 38, wobei der Patient ein Mensch ist.

**51.** Hybridom, das bei der ATCC hinterlegt ist und die Zugangsnummer PTA-4572 besitzt.

**52.** Hybridom, das bei der ATCC hinterlegt ist und die Zugangsnummer PTA-4573 besitzt.

**53.** Hybridom, das bei der ATCC hinterlegt ist und die Zugangsnummer PTA-4574 besitzt.

**54.** Hybridom, das bei der ATCC hinterlegt ist und die Zugangsnummer PTA-5194 besitzt.

**Revendications**

**1.** Anticorps isolé qui se lie spécifiquement à EphA2 avec une valeur de $k_{off}$ de moins de 3 x $10^{-3}$ $s^{-1}$ et induit une phosphorylation de EphA2, dans lequel:

a. l'anticorps est en concurrence pour la liaison à EphA2 avec l'anticorps Eph099B-102.147 produit par l'hybridome déposé à la collection de cultures types américaine (ATCC) et avec le no. d'entrée attribué PTA-4572;
b. l'anticorps est en concurrence pour la liaison à EphA2 avec l'anticorps Eph099B-208.261 produit par l'hybridome déposé à l'ATCC et avec le no. d'entrée attribué PTA-4573;
c. l'anticorps est en concurrence pour la liaison à EphA2 avec l'anticorps Eph099B-210.248 produit par l'hybridome déposé à l'ATCC et avec le no. d'entrée attribué PTA-4574;
d. l'anticorps est en concurrence pour la liaison à EphA2 avec l'anticorps Eph099B-233.152 produit par l'hybridome déposé à l'ATCC et avec le no, d'entrée attribué PTA-5194;
e. l'anticorps est produit par l'hybridome déposé à l'ATCC et avec le no. d'entrée attribué PTA-4572;
f. l'anticorps est produit par l'hybridome déposé à l'ATCC et avec le no. d'entrée attribué PTA-4573;
g. l'anticorps est produit par l'hybridome déposé à l'ATCC et avec le no. d'entrée attribué PTA-4574;
h. l'anticorps est produit par l'hybridome déposé à l'ATCC et avec le no. d'entrée attribué PTA-5194;
i. l'anticorps comprend une chaîne VH comprenant la séquence d'acides aminés de la SEQ ID NO:5 ou 21 et une chaîne VL comprenant la séquence d'acides aminés de la SEQ ID NO:1 1 ou 17;
j. l'anticorps comprend une CDR1 de VL possédant la séquence d'acides aminés de la SEQ ID NO:2 ou 18, une CDR2 de VL possédant la séquence d'acides aminés de la SEQ ID NO:3 ou 19, une CDR3 de VL possédant la séquence d'acides aminés de la SEQ ID NO:4 ou 20, une CDR1 de VII possédant la séquence d'acides aminés de la SEQ ID NO:6 ou 22, une CDR2 de VH possédant la séquence d'acides aminés de la SEQ ID NO: 7 ou 23 et une CDR3 de VH possédant la séquence d'acides aminés de la SEQ ID NO:8 ou 24; ou
k. l'anticorps comprend une CDR1 de VL possédant la séquence d'acides aminés de la SEQ ID NO:2 ou 18, une CDR2 de VL possédant la séquence d'acides aminés de la SEQ ID NO:3 ou 19, une CDR3 de VL possédant la séquence d'acides aminés de la SEQ ID NO:4 ou 20, une CDR1 de VH possédant la séquence d'acides aminés de la SEQ ID NO:6 ou 22, une CDR2 de VH possédant la séquence d'acides aminés de la SEQ ID NO: 7 ou 23 et une CDR3 de VH possédant la séquence d'acides aminés de la SEQ ID NO:8 ou 24, où une ou plusieurs des CDRs présentent une, deux ou trois mutations.

**2.** Anticorps selon la revendication 1, dans lequel la valeur de $k_{off}$ est de moins de 1 x $10^{-3}$ $s^{-1}$.

**3.** Anticorps selon la revendication 1, qui est produit par un hybridome déposé à l'ATCC avec le numéro d'entrée PTA-4572.

**4.** Anticorps selon la revendication 1, qui est produit par un hybridome déposé à l'ATCC avec le numéro d'entrée PTA-4573.

**5.** Anticorps selon la revendication 1, qui est produit par un hybridome déposé à l'ATCC avec le numéro d'entrée PTA-4574.

**6.** Anticorps selon la revendication 1, qui est produit par un hybridome déposé à l'ATCC avec le numéro d'entrée PTA-5194.

7. Anticorps selon la revendication 1, où l'anticorps est en concurrence pour la liaison à EphA2 avec l'anticorps Eph099B-102.147 produit par l'hybridome déposé à l'ATCC et avec le no. d'entrée attribué PTA-4572.

8. Anticorps selon la revendication 1 ou 2, où l'anticorps est en concurrence pour la liaison à EphA2 avec l'anticorps Eph099B-208.261 produit par l'hybridome déposé à l'ATCC et avec le no. d'entrée attribué PTA-4573.

9. Anticorps selon la revendication 1 ou 2, où l'anticorps est en concurrence pour la liaison à EphA2 avec l'anticorps Eph099B-210.248 produit par l'hybridome déposé à l'ATCC et avec le no. d'entrée attribué PTA-4574.

10. Anticorps selon la revendication 1 ou 2, où l'anticorps est en concurrence pour la liaison à EphA2 avec l'anticorps Eph099B-233.152 produit par l'hybridome déposé à l'ATCC et avec le no. d'entrée attribué PTA-5194.

11. Anticorps selon la revendication 1, où l'anticorps comprend une chaîne lourde variable (VH) possédant la séquence d'acides aminés de la SEQ ID NO:5 ou 21.

12. Anticorps selon la revendication 1, où l'anticorps comprend une chaîne légère variable (VL) possédant la séquence d'acides aminés de la SEQ ID NO:1 ou 17.

13. Anticorps selon la revendication 1, où l'anticorps comprend une chaîne VH comprenant la séquence d'acides aminés de la SEQ ID NO:5 et une chaîne VL comprenant la séquence d'acides aminés de la SEQ ID NO:1.

14. Anticorps selon la revendication 1, où l'anticorps comprend une chaîne VH comprenant la séquence d'acides aminés de la SEQ ID NO:21 et une chaîne VL comprenant la séquence d'acides aminés de la SEQ ID NO:17.

15. Anticorps selon la revendication 1, où l'anticorps comprend une CDR1 de VL possédant la séquence d'acides aminés de la SEQ ID NO:2, une CDR2 de VL possédant la séquence d'acides aminés de la SEQ ID NO:3 et une CDR3 de VL possédant la séquence d'acides aminés de la SEQ ID NO:4.

16. Anticorps selon la revendication 1, où l'anticorps comprend une CDR1 de VH possédant la séquence d'acides aminés de la SEQ ID NO:6, une CDR2 de VH possédant la séquence d'acides aminés de la SEQ ID NO:7 et une CDR3 de VH possédant la séquence d'acides aminés de la SEQ ID NO:8 .

17. Anticorps selon la revendication 1, où l'anticorps comprend une CDR1 de VL possédant la séquence d'acides aminés de la SEQ ID NO:18, une CDR2 de VL possédant la séquence d'acides aminés de la SEQ ID NO:19 et une CDR3 de VL possédant la séquence d'acides aminés de la SEQ ID NO:20.

18. Anticorps selon la revendication 1, où l'anticorps comprend une CDR1 de VH possédant la séquence d'acides aminés de la SEQ ID NO:22, une CDR2 de VH possédant la séquence d'acides aminés de la SEQ ID NO:23 et une CDR3 de VH possédant la séquence d'acides aminés de la SEQ ID NO:24.

19. Anticorps selon la revendication 1, où l'anticorps comprend une CDR1 de VL possédant la séquence d'acides aminés de la SEQ ID NO:2, une CDR2 de VL possédant la séquence d'acides aminés de la SEQ ID NO:3, une CDR3 de VL possédant la séquence d'acides aminés de la SEQ ID NO:4, une CDR1 de VH possédant la séquence d'acides aminés de la SEQ ID NO:6, une CDR2 de VH possédant la séquence d'acides aminés de la SEQ ID NO: 7 et une CDR3 de VH possédant la séquence d'acides aminés de la SEQ ID NO:8.

20. Anticorps selon la revendication 1, où l'anticorps comprend une CDR1 de VL possédant la séquence d'acides aminés de la SEQ ID NO:18, une CDR2 de VL possédant la séquence d'acides aminés de la SEQ ID NO:19, une CDR3 de VL possédant la séquence d'acides aminés de la SEQ ID NO:20, une CDR1 de VH possédant la séquence d'acides aminés de la SEQ ID NO:22, une CDR2 de VH possédant la séquence d'acides aminés de la SEQ ID NO: 23 et une CDR3 de VH possédant la séquence d'acides aminés de la SEQ ID NO:24.

21. Anticorps selon l'une quelconque des revendications 1 ou 7 à 20, où l'anticorps est un anticorps humanisé, un fragment Fab, un anticorps chimérique, un anticorps à une seule chaîne, un fragment F(ab') ou un Fv lié à un disulfure.

22. Anticorps selon la revendication 1 ou 7 à 20, où l'anticorps est un anticorps monoclonal.

23. Conjugué d'anticorps comprenant l'anticorps selon l'une quelconque des revendications précédentes conjugué ou fusionné à un agent hétérologue.

**24.** Conjugué d'anticorps selon la revendication 23, dans lequel l'agent hétérologue est un agent de diagnostic ou détectable.

**25.** Conjugué d'anticorps selon la revendication 23, dans lequel l'agent hétérologue est une cytotoxine.

**26.** Conjugué d'anticorps selon la revendication 23, dans lequel l'agent hétérologue est un agent thérapeutique ou une fraction de médicament.

**27.** Acide nucléique isolé comprenant une séquence de nucléotides codant pour l'anticorps selon l'une quelconque des revendications 1, 3 à 6, 13, 14, 19 ou 20.

**28.** Vecteur comprenant l'acide nucléique selon la revendication 27.

**29.** Cellule hôte comprenant le vecteur selon la revendication 28.

**30.** Cellule hôte mise au point pour contenir ou exprimer l'acide nucléique selon la revendication 27.

**31.** Composition pharmaceutique comprenant l'anticorps selon l'une quelconque des revendications 1 à 26 et un support pharmaceutiquement acceptable.

**32.** Utilisation de la composition pharmaceutique selon la revendication 31 dans la préparation d'un médicament pour le traitement d'un cancer chez un patient, dans laquelle le cancer est associé à une surexpression de EphA2.

**33.** Composition pharmaceutique comprenant le conjugué d'anticorps selon la revendication 23, 24, 25 ou 26.

**34.** Utilisation de la composition pharmaceutique selon la revendication 33 dans la préparation d'un médicament pour le traitement d'un cancer chez un patient, dans laquelle le cancer est associé à une surexpression de EphA2.

**35.** Utilisation de l'anticorps selon l'une quelconque des revendications 1 à 22 dans la préparation d'un médicament pour le traitement d'un cancer chez un patient, dans laquelle le cancer est associé à une surexpression de EphA2.

**36.** Utilisation du conjugué d'anticorps selon la revendication 23, 25 ou 26 dans la préparation d'un médicament pour le traitement d'un cancer chez un patient, dans laquelle le cancer est associé à une surexpression de EphA2.

**37.** Utilisation selon la revendication 35 ou 36, dans laquelle la composition comprend en outre une thérapie supplémentaire.

**38.** Méthode in vitro pour diagnostiquer un cancer chez un patient, comprenant:

a. la mise en contact de cellules provenant dudit patient avec l'anticorps selon l'une quelconque des revendications 1 à 22 dans des conditions appropriées pour une liaison anticorps-EphA2; et
b. la mesure de la liaison de l'anticorps contre EphA2 avec lesdites cellules, dans laquelle un niveau de liaison de l'anticorps contre EphA2 supérieur à un témoin indique que le patient a un cancer.

**39.** Utilisation de l'anticorps selon l'une quelconque des revendications 1 à 22 dans la préparation d'une composition pour diagnostiquer, prévoir ou surveiller l'efficacité d'une thérapie pour un cancer chez un patient, dans laquelle le cancer est associé à une surexpression de EphA2.

**40.** Utilisation du conjugué d'anticorps selon la revendication 24 dans la préparation d'une composition pour diagnostiquer, prévoir ou surveiller l'efficacité d'une thérapie pour un cancer chez un patient, dans laquelle le cancer est associé à une surexpression de EphA2.

**41.** Utilisation selon l'une quelconque des revendications 34 à 37, 39 ou 40, dans laquelle le cancer est d'une origine de cellules épithéliales.

**42.** Utilisation selon l'une quelconque des revendications 34 à 37, 39 ou 40, dans laquelle le cancer est un cancer métastatique.

**43.** Utilisation selon l'une quelconque des revendications 34 à 37 ou 39 à 42, dans laquelle le cancer est un cancer du sein, un cancer du côlon, un cancer de la prostate, un mélanome, un cancer de l'ovaire, un cancer du poumon, un cancer de la peau, un cancer de la vessie, un cancer du pancréas ou un carcinome des cellules rénales.

**44.** Méthode selon la revendication 38, dans laquelle le cancer est un cancer du sein, un cancer du côlon, un cancer de la prostate, un mélanome, un cancer de l'ovaire, un cancer du poumon, un cancer de la peau, un cancer de la vessie, un cancer du pancréas ou un carcinome des cellules rénales.

**45.** Méthode selon la revendication 38, dans laquelle le cancer est d'une origine de cellules épithéliales.

**46.** Méthode selon la revendication 38, dans laquelle le cancer est un cancer métastatique.

**47.** Méthode selon la revendication 38, dans laquelle les cellules proviennent de sang entier, d'expectoration, d'urine, de sérum ou d'aspirations à l'aiguille fine d'un tissu de cellules tumorales.

**48.** Utilisation selon l'une quelconque des revendications 34 à 37, 39 ou 40, dans laquelle le patient est un être humain.

**49.** Utilisation selon la revendication 48, dans laquelle l'être humain est réfractaire à une première thérapie.

**50.** Méthode selon la revendication 38, dans laquelle le patient est un être humain.

**51.** Hybridome déposé à l'ATCC avec le numéro d'entrée PTA-4572.

**52.** Hybridome déposé à l'ATCC avec le numéro d'entrée PTA-4573.

**53.** Hybridome déposé à l'ATCC avec le numéro d'entrée PTA-4574.

**54.** Hybridome déposé à l'ATCC avec le numéro d'entrée PTA-5194.

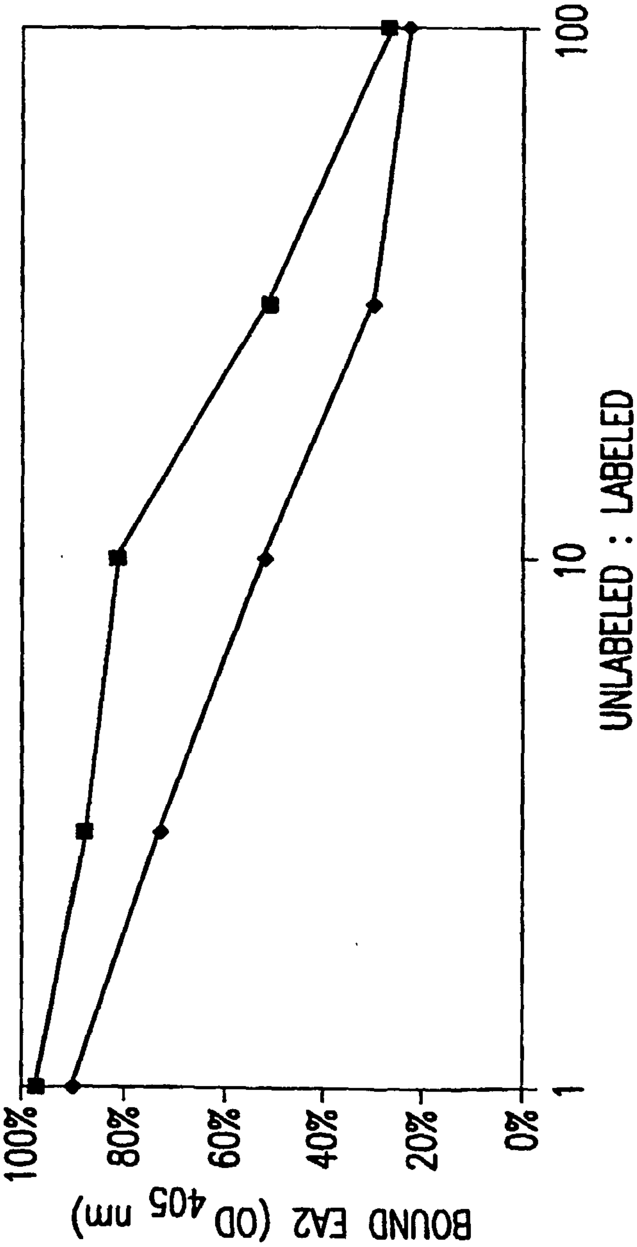


FIG. 1

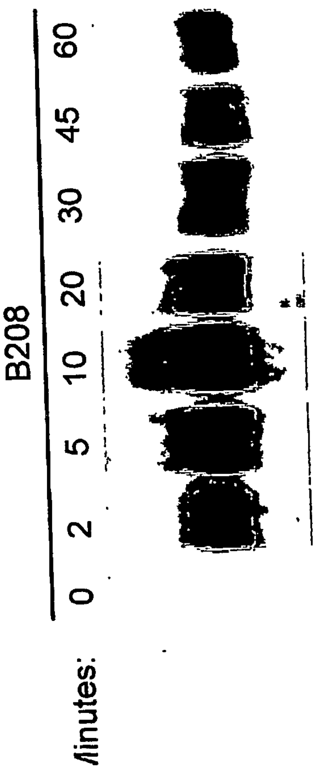


FIG.2A

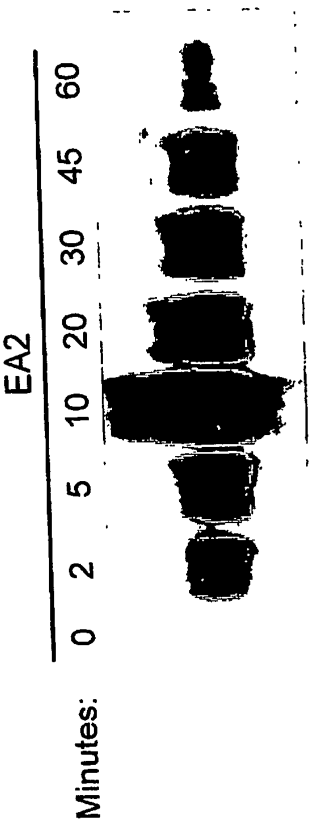


FIG.2B

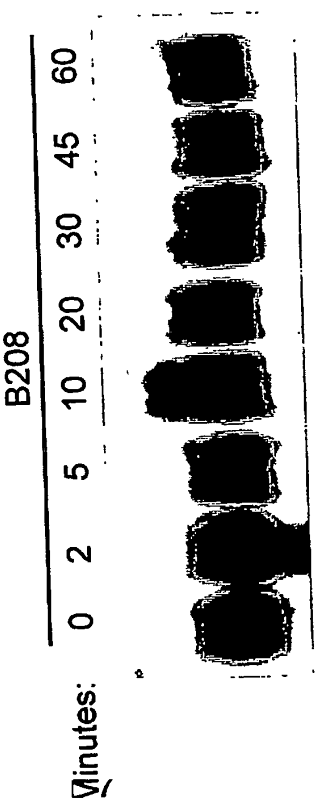


FIG.2C

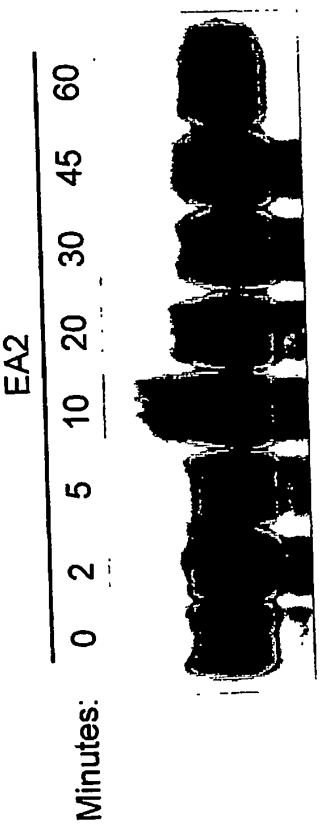


FIG.2D

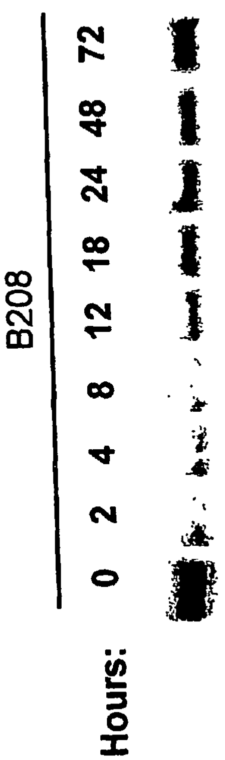


FIG.3A

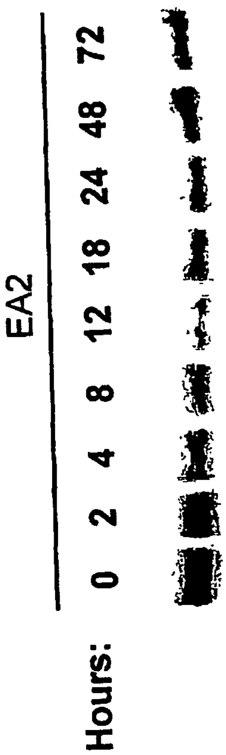


FIG.3B

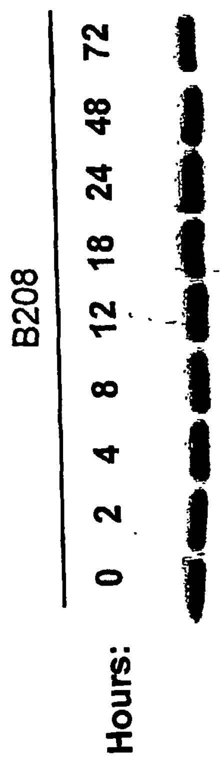


FIG.3D

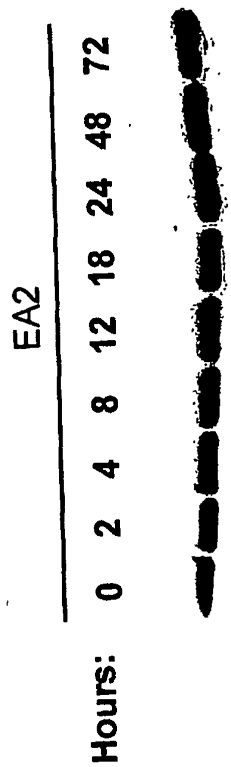


FIG.3D

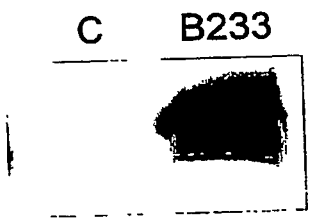


FIG.4A

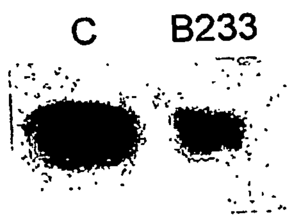


FIG.4B

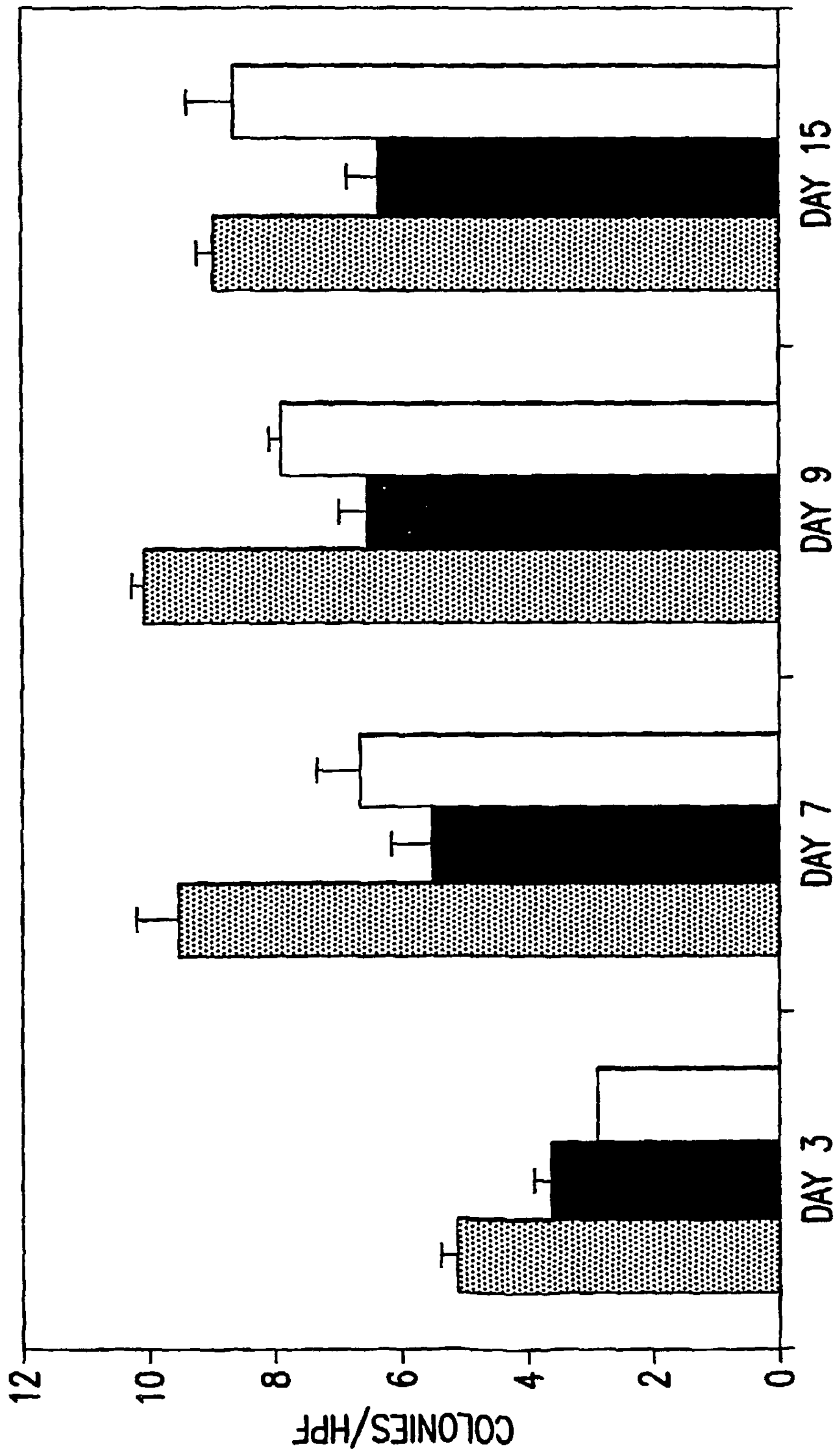


FIG.5

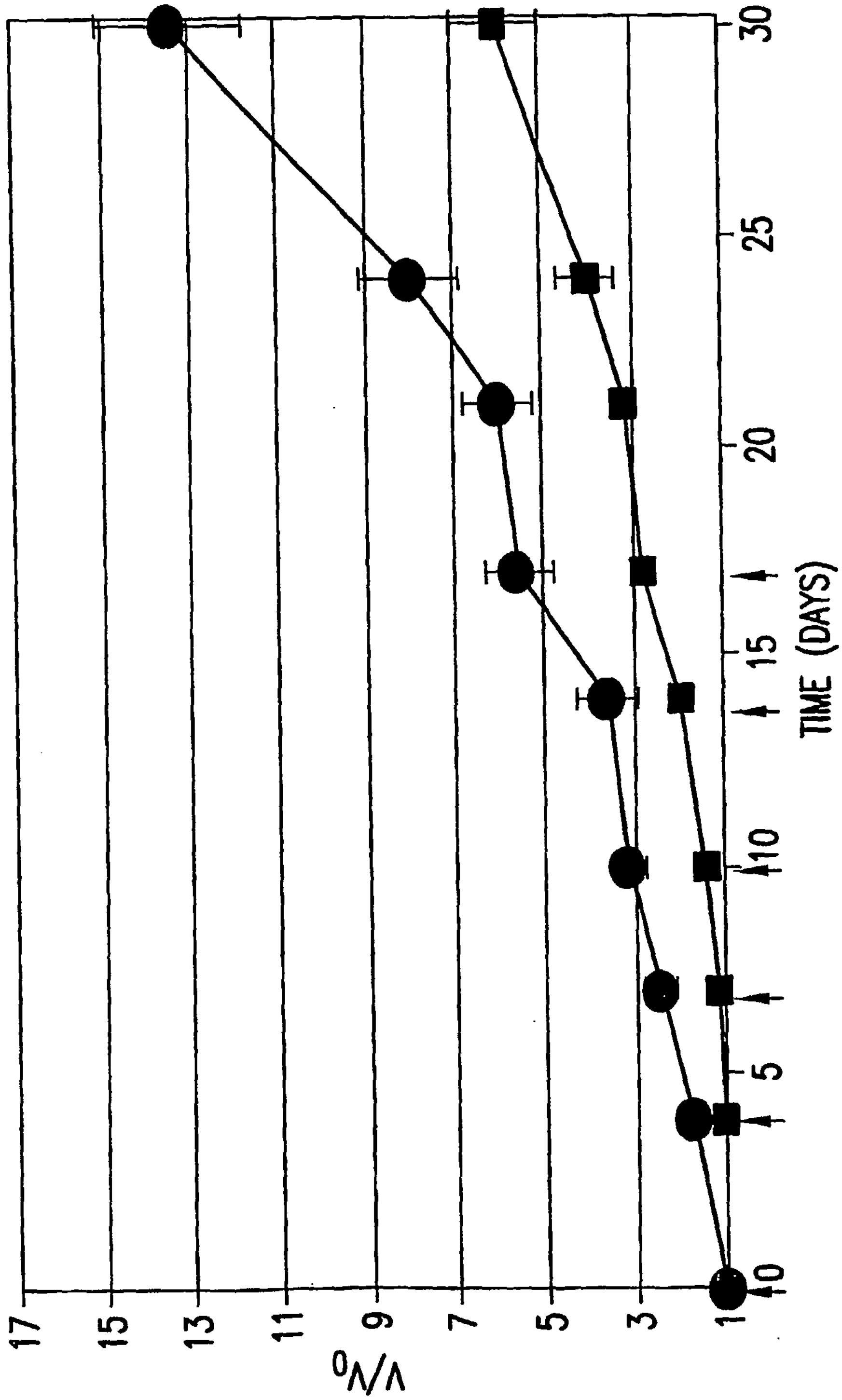


FIG.6A

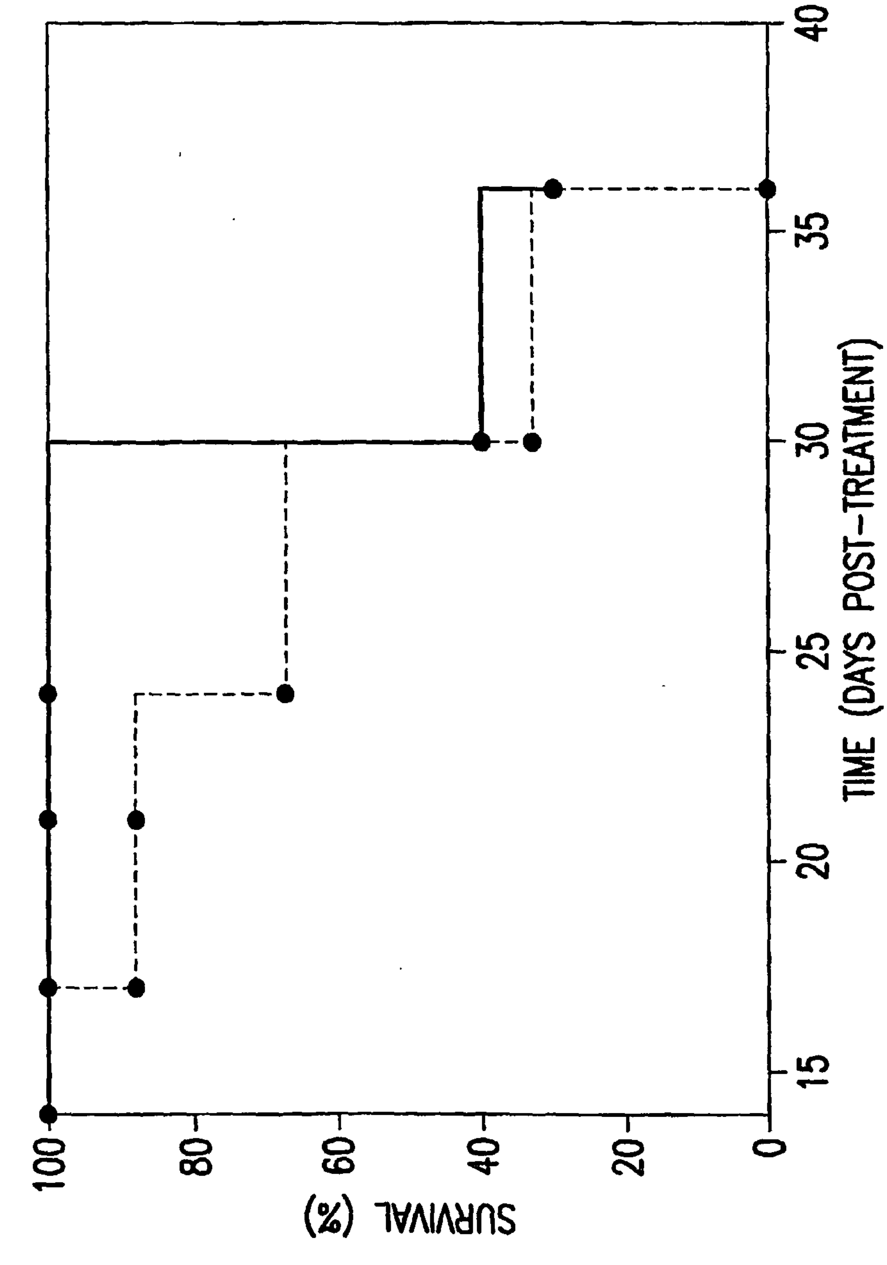
SURVIVAL (%)
100
80
60
40
20
0
15
20
25
30
35
40
TIME (DAYS POST-TREATMENT)

FIG.6B

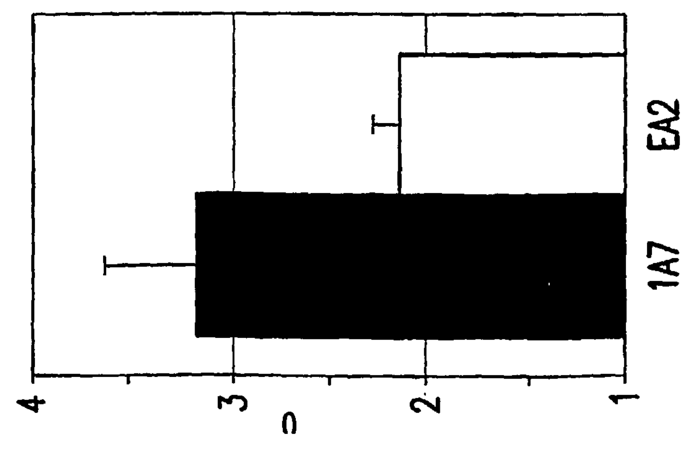


FIG.7A

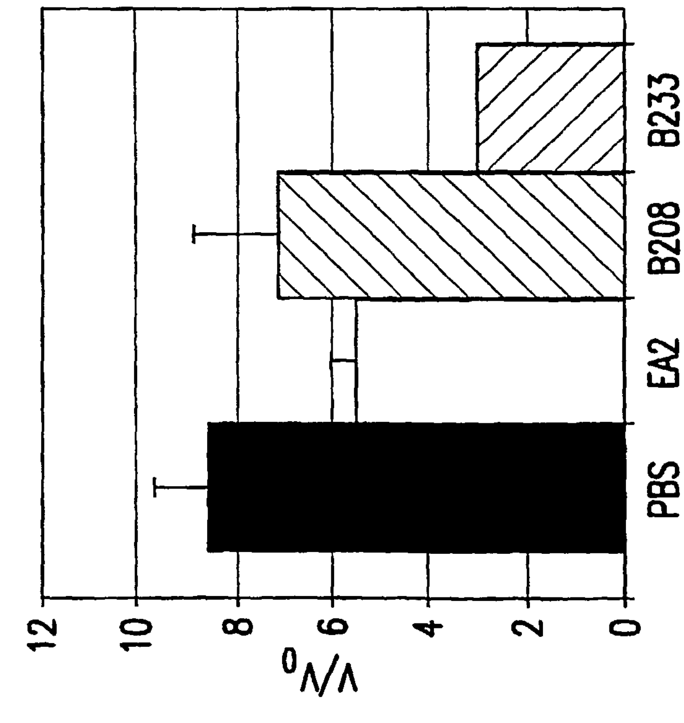


FIG.7B

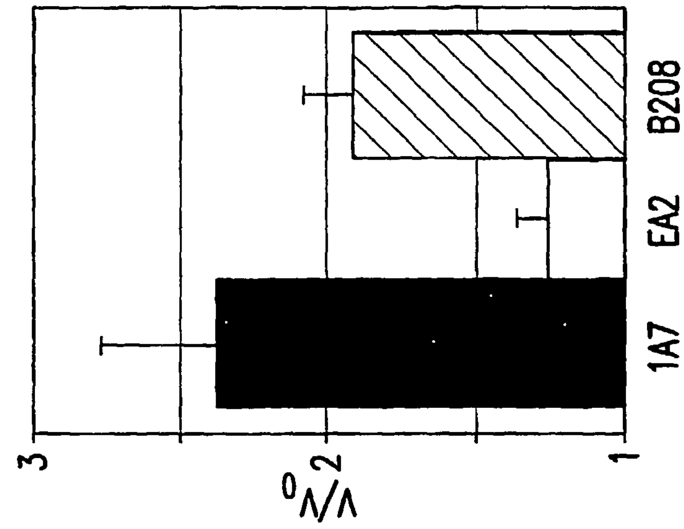


FIG.7C

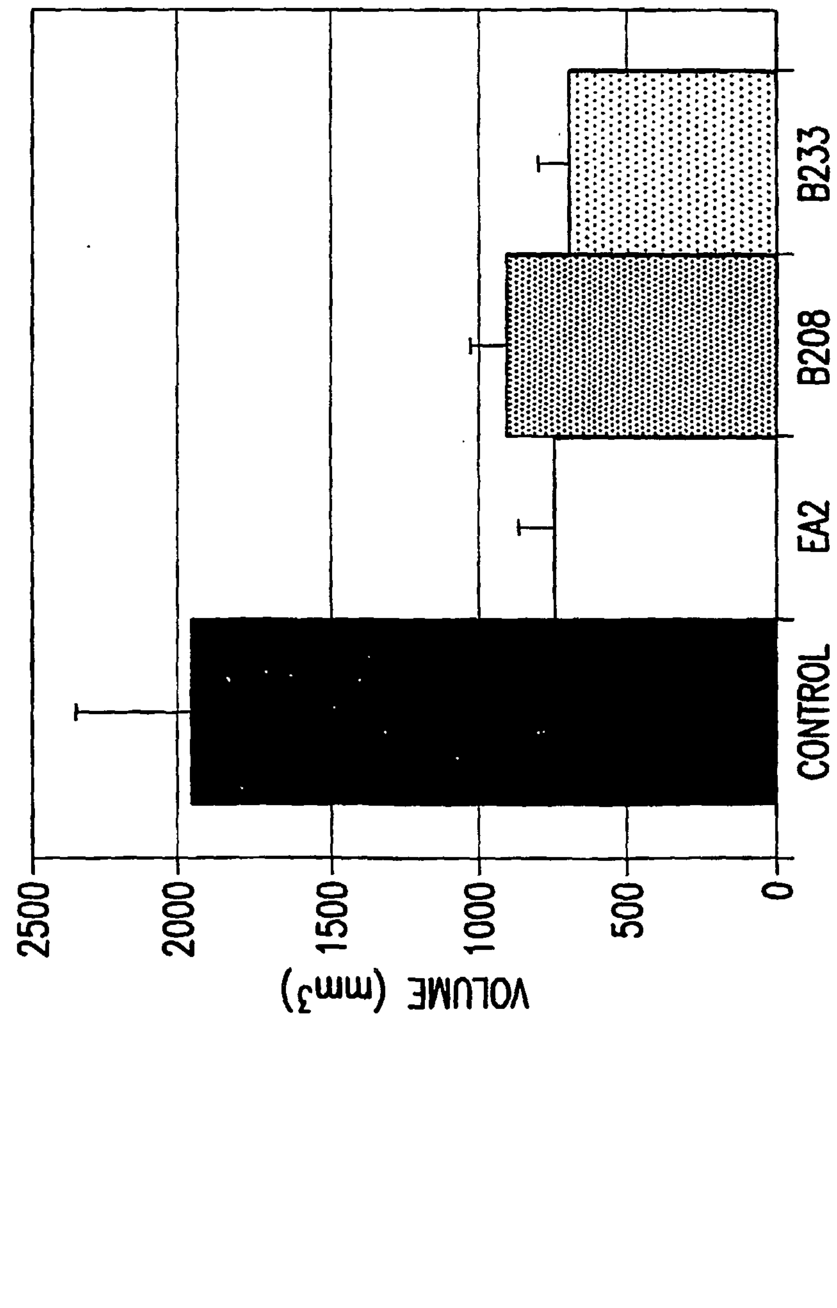


FIG.7D

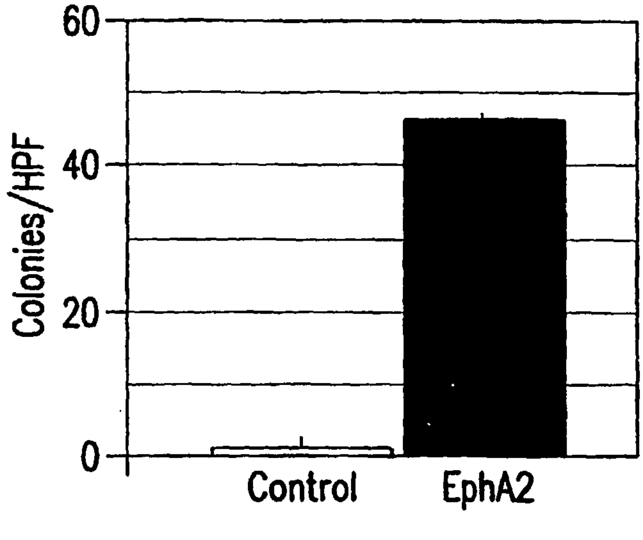
60
40
20
0
Colonies/HPF
Control
EphA2

FIG.8A

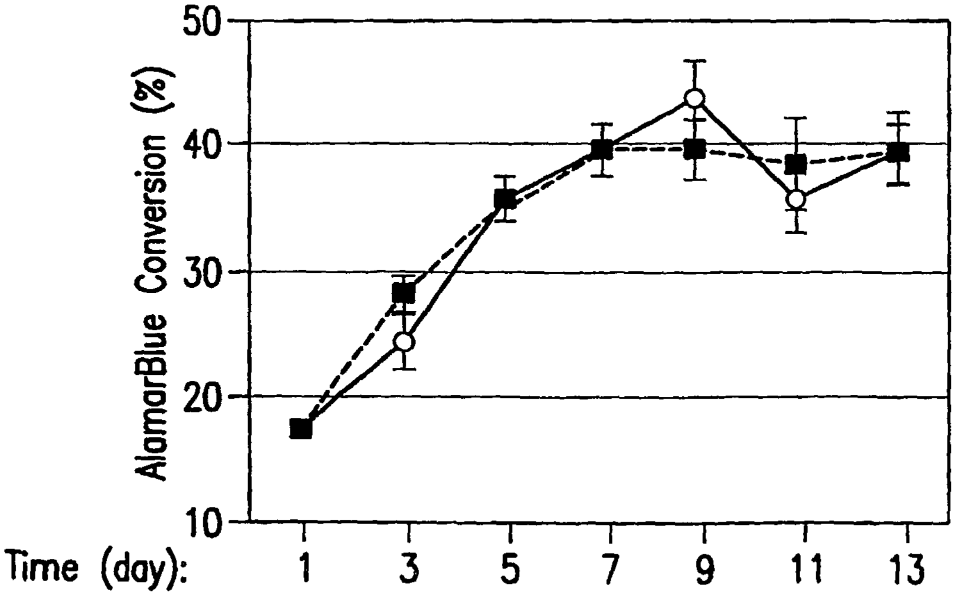
50
40
30
20
10
AlamarBlue Conversion (%)
Time (day):
1
3
5
7
9
11
13

FIG.8B

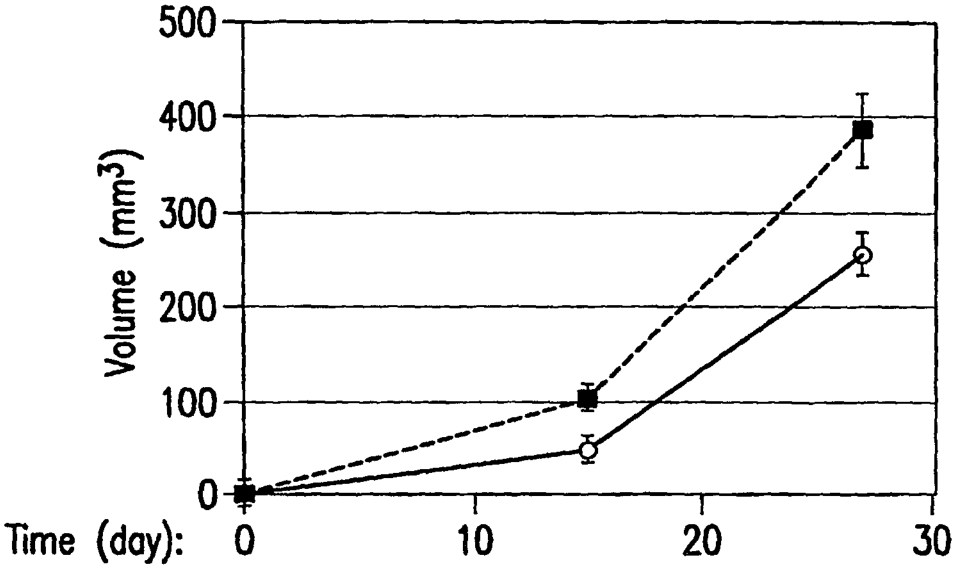


FIG.9A

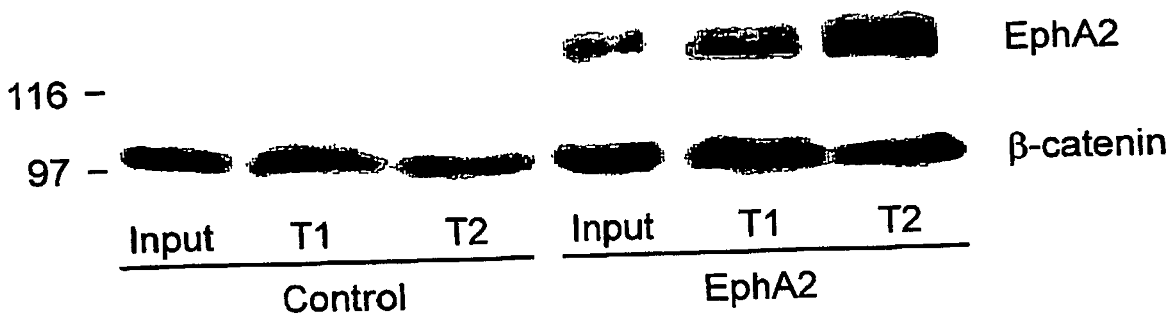


FIG.9B

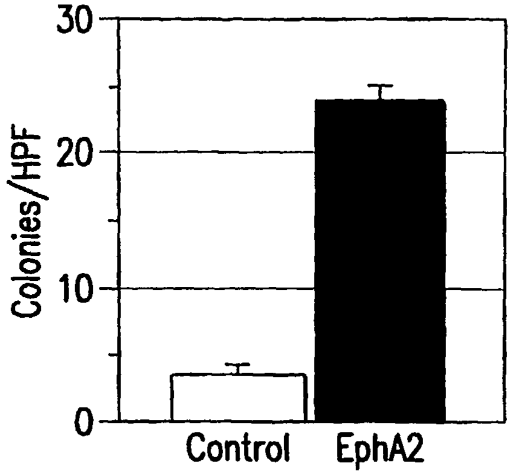


FIG.10A

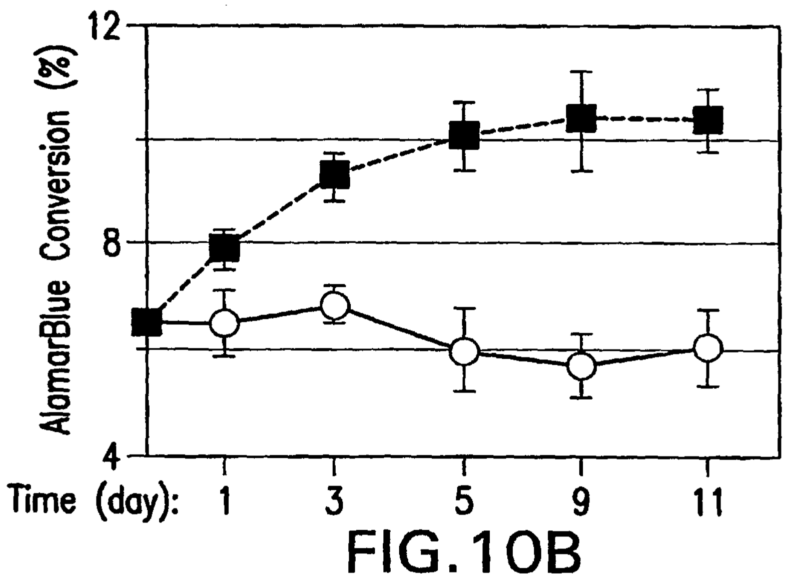


FIG.10B

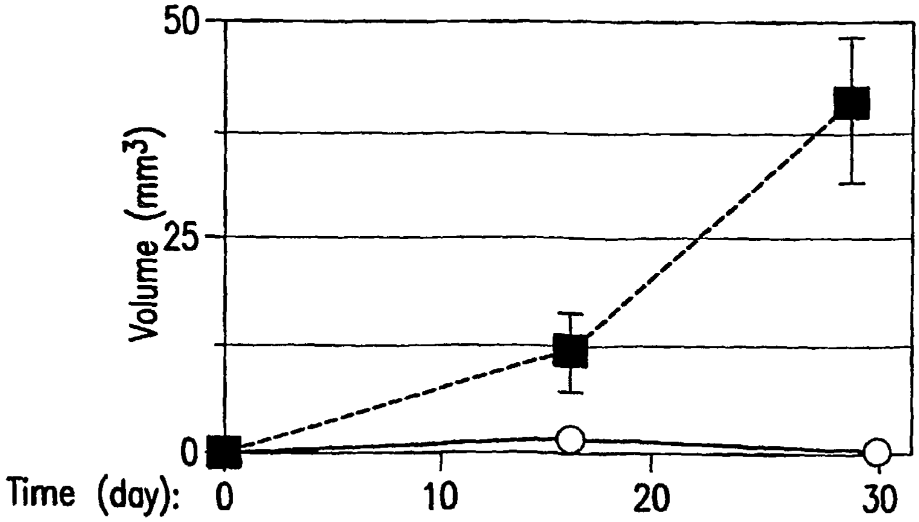


FIG.10C

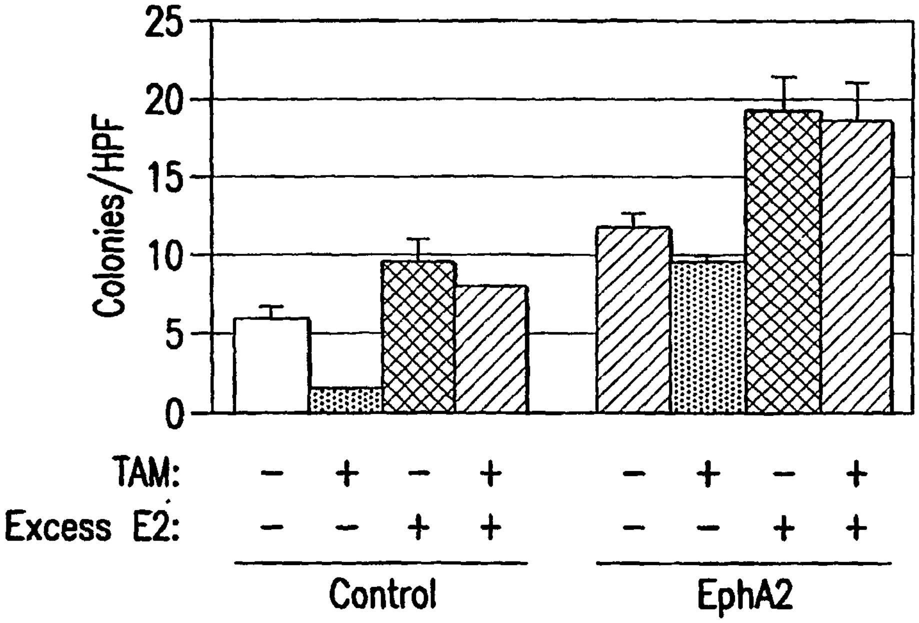


FIG.11A

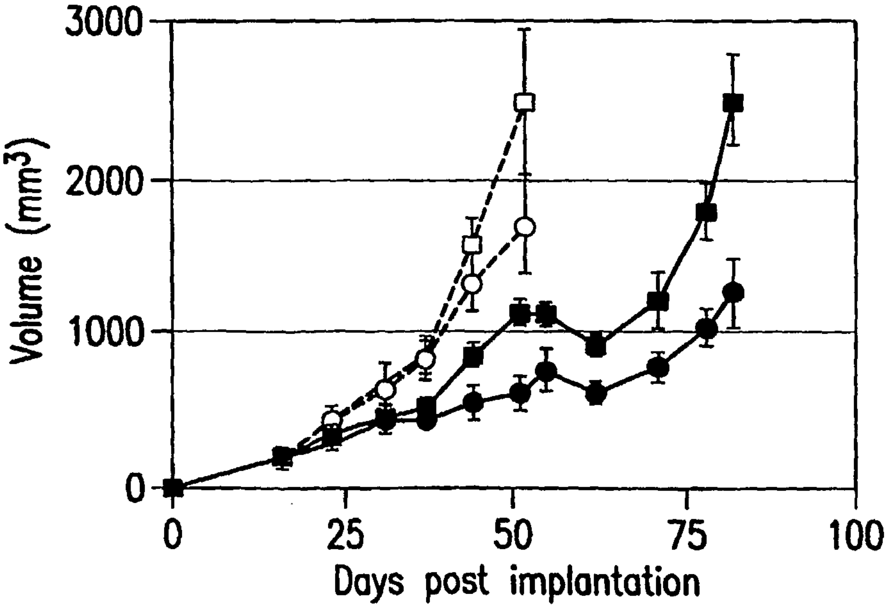


FIG.11B

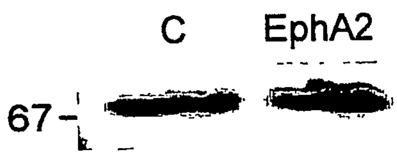


FIG.12A

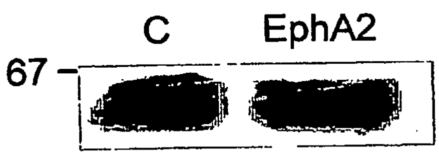


FIG.12B

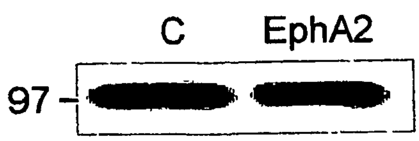


FIG.12C

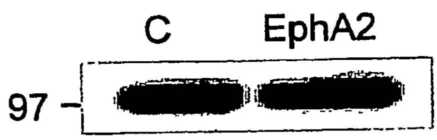


FIG.12D

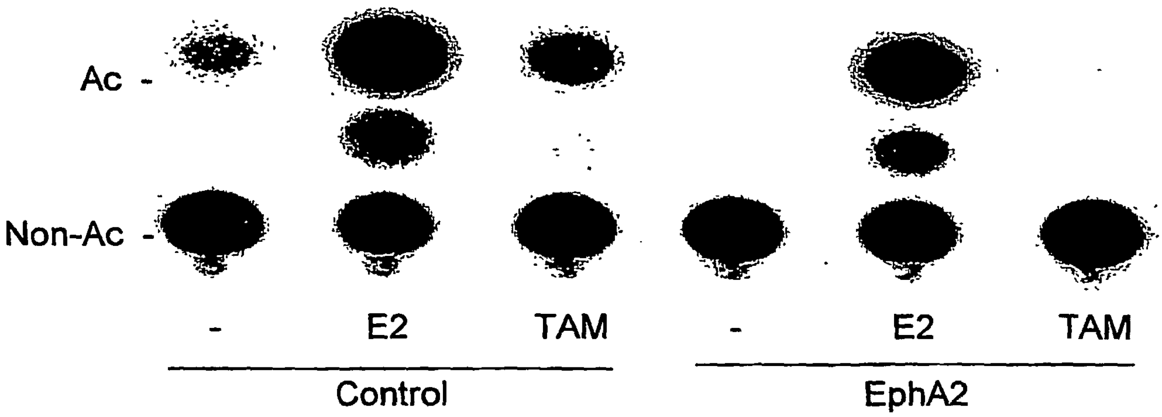


FIG.12E

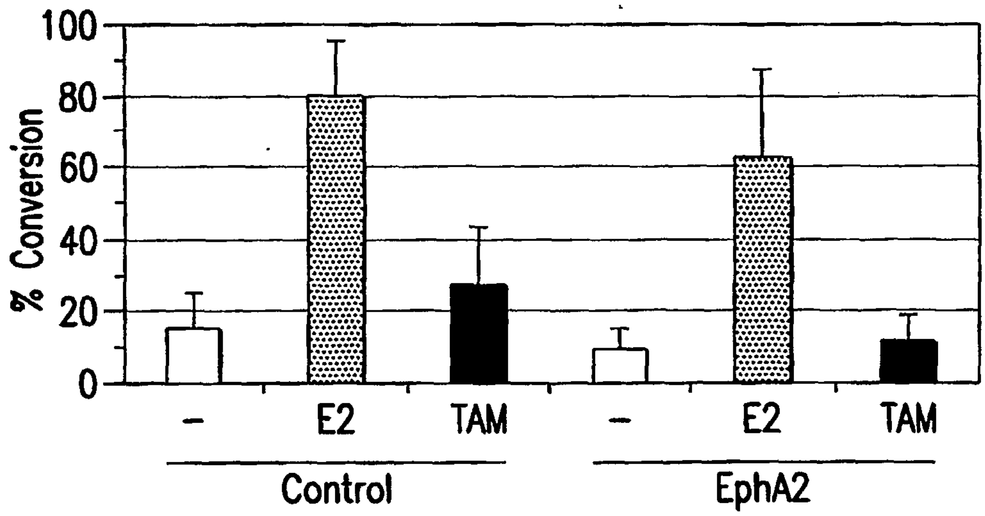


FIG.12F

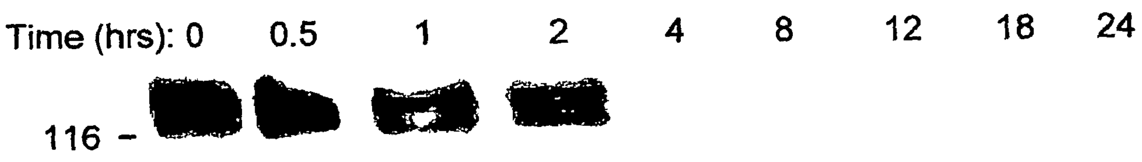


FIG.13A

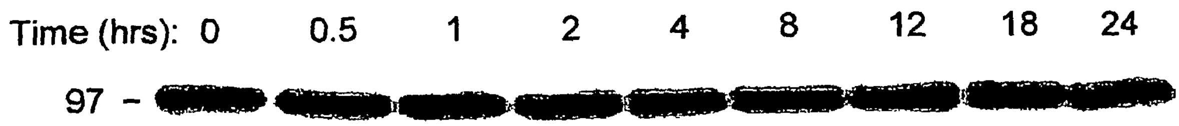


FIG.13B

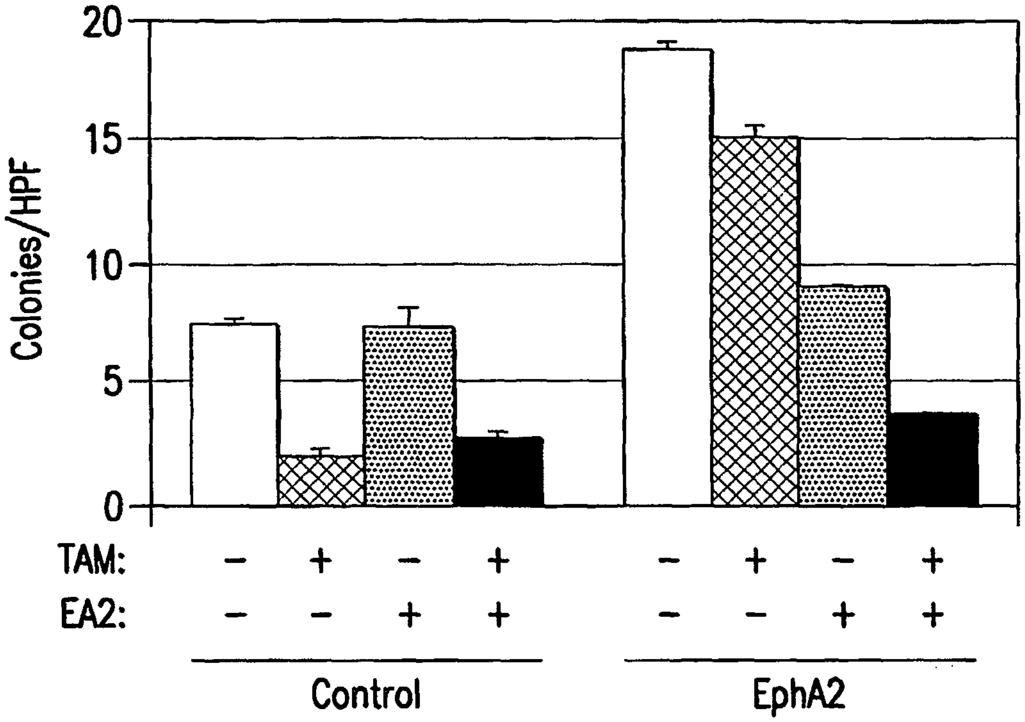


FIG.13C

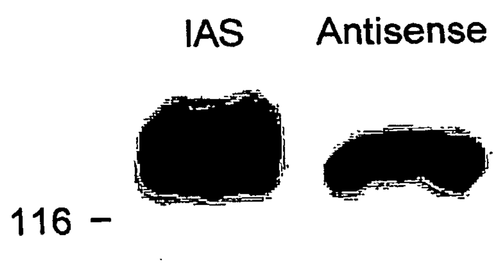


FIG.14A

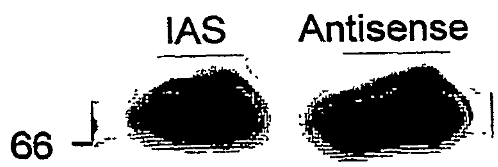


FIG.14B

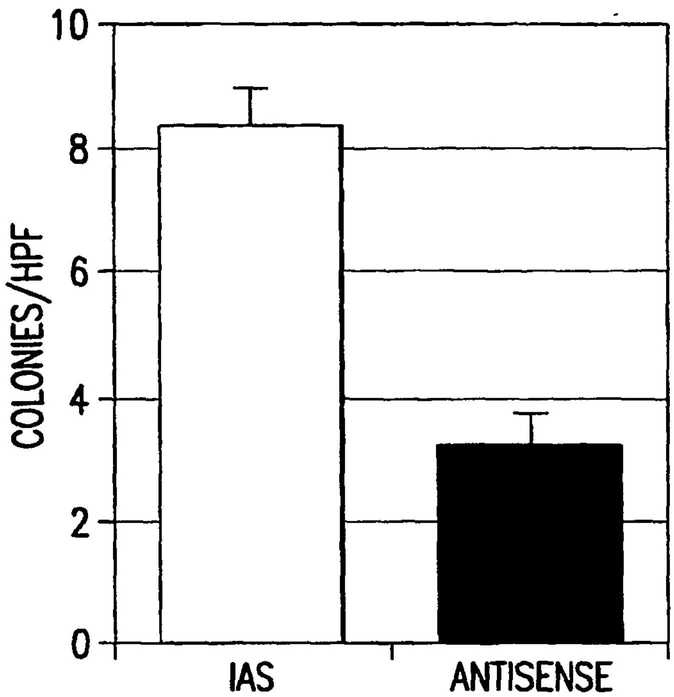


FIG.14C

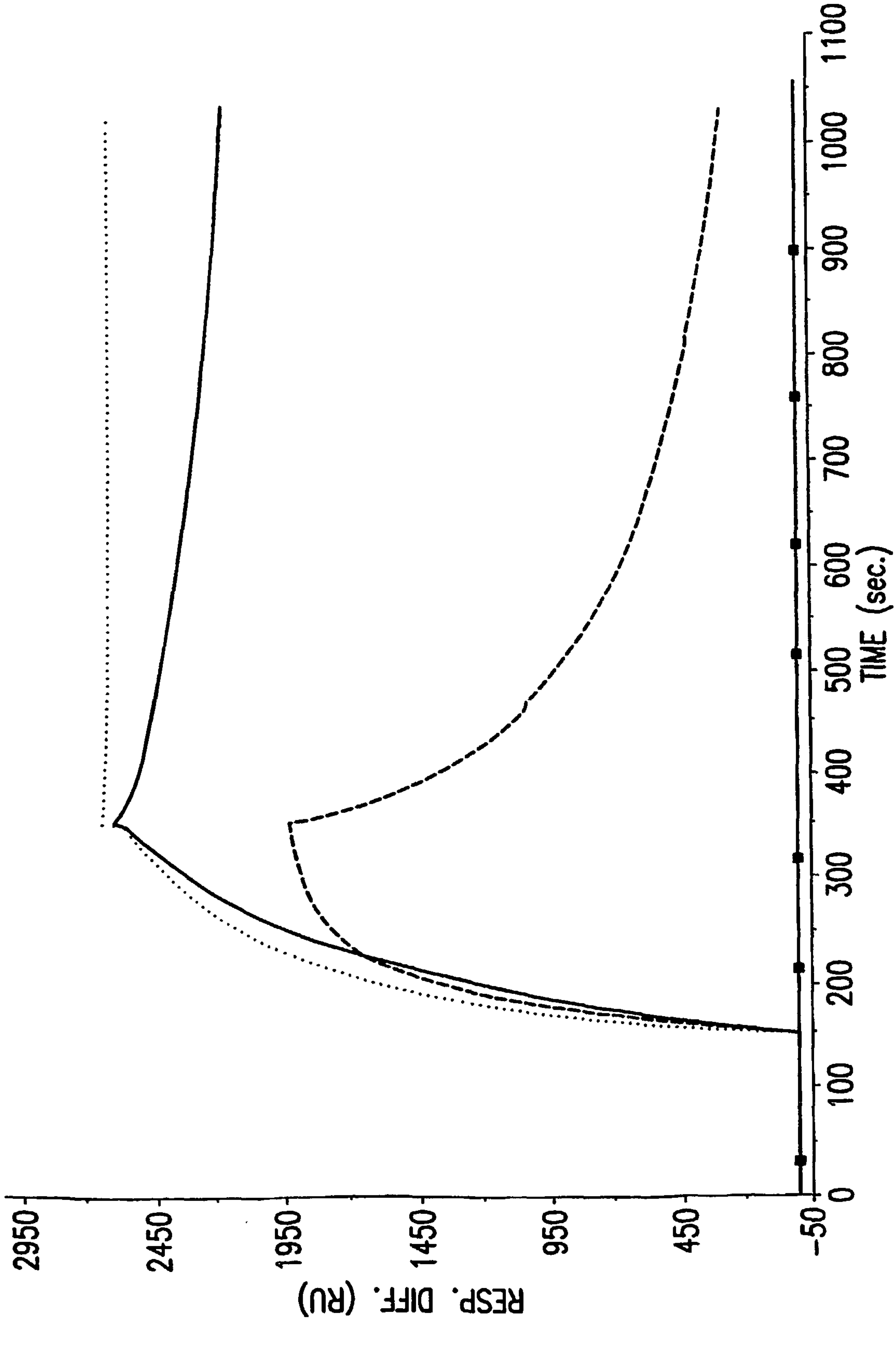
RESP. DIFF. (RU)
2950
2450
1950
1450
950
450
-50
0
100
200
300
400
500
600
700
800
900
1000
1100
TIME (sec.)

FIG.15

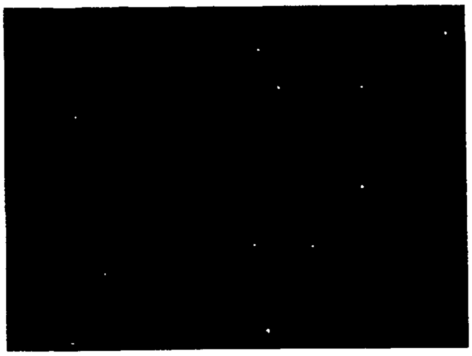

FIG.16A

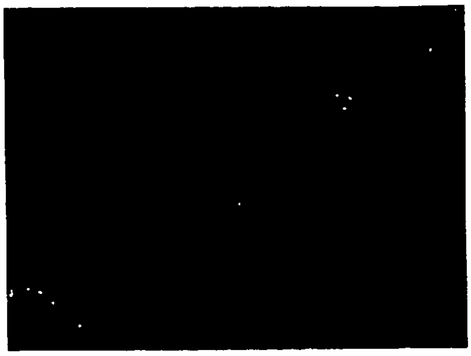

FIG.16B

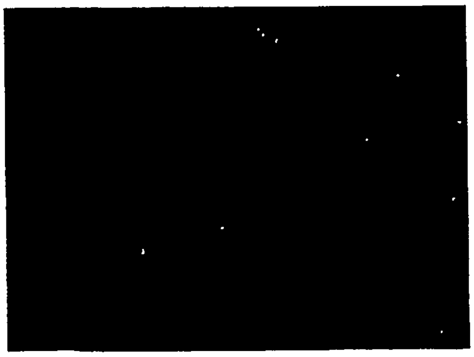

FIG.16C

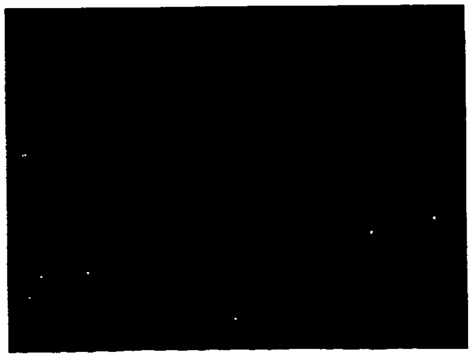

FIG.16D

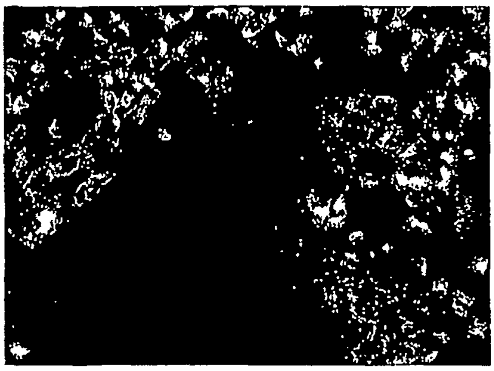

FIG.17A

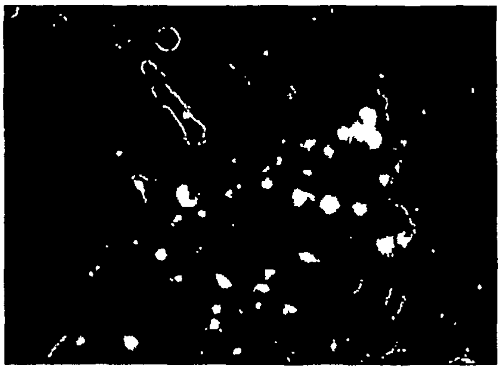

FIG.17B

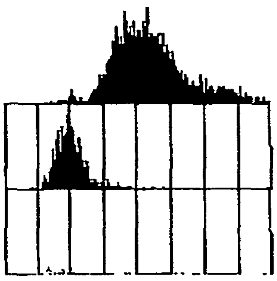

MCF-10

FIG.17C

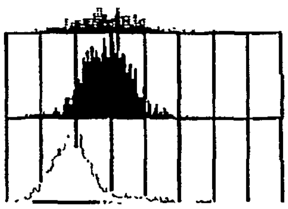

MDA-MB-231

FIG.17D

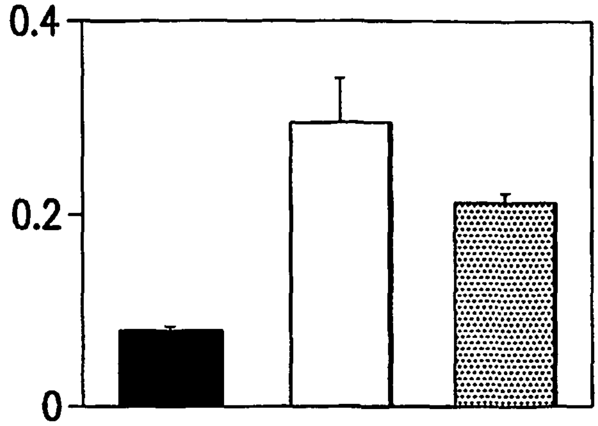


FIG.18A

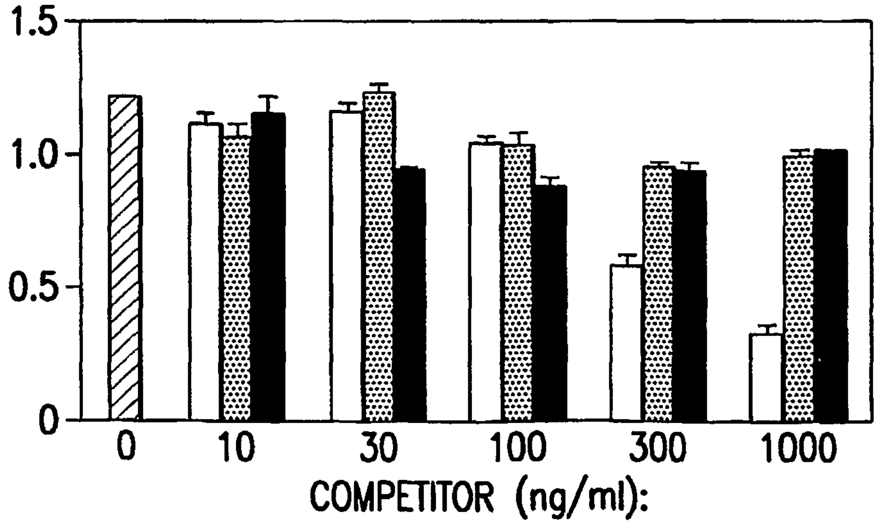


FIG.18B

208 Variable Light Chain

```
caa att gtt ctc acc cag tct cca gca ctc atg tct gca tct cca ggg      48
Gln Ile Val Leu Thr Gln Ser Pro Ala Leu Met Ser Ala Ser Pro Gly
1               5                   10                  15
                                          CDR1
gag aag gtc acc atg acc tgc agt gcc agc tca agt gta agt tac atg      96
Glu Lys Val Thr Met Thr Cys Ser Ala Ser Ser Ser Val Ser Tyr Met
            20                  25                  30

tac tgg tac cag cag aag cca aga tcc tcc ccc aaa ccc tgg att tat     144
Tyr Trp Tyr Gln Gln Lys Pro Arg Ser Ser Pro Lys Pro Trp Ile Tyr
        35                  40                  45
      CDR2
ctc aca acc aac ctg gct tct gga gtc cct gct cgc ttc agt ggc agt     192
Leu Thr Thr Asn Leu Ala Ser Gly Val Pro Ala Arg Phe Ser Gly Ser
    50                  55                  60

ggg tct ggg acc tct tac tct ctc aca atc agc agc atg gag gct gaa     240
Gly Ser Gly Thr Ser Tyr Ser Leu Thr Ile Ser Ser Met Glu Ala Glu
65                  70                  75                  80
                                       CDR3
gat gct gcc act tat tac tgc cag cag tgg agt agt aac cca ttc acg     288
Asp Ala Ala Thr Tyr Tyr Cys Gln Gln Trp Ser Ser Asn Pro Phe Thr
                85                  90                  95

ttc ggc tcg ggg aca aag ttg gaa ata aga                             318
Phe Gly Ser Gly Thr Lys Leu Glu Ile Arg
            100                 105
```

FIG.19A

208 Variable heavy Chain

```
cag gtc caa ctg cag cag cct ggg gct gag ctg gta aag cct ggg gct     48
Gln Val Gln Leu Gln Gln Pro Gly Ala Glu Leu Val Lys Pro Gly Ala
1               5                   10                  15
                                           CDR1
tca gtg aag ttg tcc tgc aag gct tct ggc tac act ttc acc agc tac     96
Ser Val Lys Leu Ser Cys Lys Ala Ser Gly Tyr Thr Phe Thr Ser Tyr
            20                  25                  30

tgg atg cac tgg gtg aaa caa agg cct gga caa ggc ctt gag tgg att    144
Trp Met His Trp Val Lys Gln Arg Pro Gly Gln Gly Leu Glu Trp Ile
        35                  40                  45
                                 CDR2
ggg atg att cat cct aat agt ggt agt act aac tac aat gag aag ttc    192
Gly Met Ile His Pro Asn Ser Gly Ser Thr Asn Tyr Asn Glu Lys Phe
    50                  55                  60

aag agc aag gcc aca ctg act gta gac aaa tcc tcc agc aca gcc tac    240
Lys Ser Lys Ala Thr Leu Thr Val Asp Lys Ser Ser Ser Thr Ala Tyr
65                  70                  75                  80

atg cga ctc agc agc ctg aca tct gag gac tct gcg gtc tat tac tgt    288
Met Arg Leu Ser Ser Leu Thr Ser Glu Asp Ser Ala Val Tyr Tyr Cys
                85                  90                  95
                       CDR3
gca aga ggg ggt aac atg gta ggg ggg ggc tac tgg ggc caa ggc acc    336
Ala Arg Gly Gly Asn Met Val Gly Gly Gly Tyr Trp Gly Gln Gly Thr
            100                 105                 110

act ctc aca gtc tcc tca                                             354
Thr Leu Thr Val Ser Ser
        115
```

FIG.19B

233 Variable Light Chain

```
gat att gtg cta act cag tct cca gcc acc ctg tct gtg act cca gga     48
Asp Ile Val Leu Thr Gln Ser Pro Ala Thr Leu Ser Val Thr Pro Gly
1           5                   10                  15
                                          CDR1
gat agc gtc aat ctt tcc tgc agg gcc agc caa agt att agc aac aac     96
Asp Ser Val Asn Leu Ser Cys Arg Ala Ser Gln Ser Ile Ser Asn Asn
            20                  25                  30

cta cac tgg tat caa caa aaa tca cat gag tct cca agg ctt ctc atc    144
Leu His Trp Tyr Gln Gln Lys Ser His Glu Ser Pro Arg Leu Leu Ile
        35                  40                  45
                  CDR2
aag tat gtt ttc cag tcc atc tct ggg atc ccc tcc agg ttc agt ggc    192
Lys Tyr Val Phe Gln Ser Ile Ser Gly Ile Pro Ser Arg Phe Ser Gly
    50                  55                  60

agt gga tca ggg aca gat ttc act ctc agt atc aac agt gtg gag act    240
Ser Gly Ser Gly Thr Asp Phe Thr Leu Ser Ile Asn Ser Val Glu Thr
65                  70                  75                  80
                                              CDR3
gaa gat ttt gga atg tat ttc tgt caa cag agt aac agc tgg ccg ctc    288
Glu Asp Phe Gly Met Tyr Phe Cys Gln Gln Ser Asn Ser Trp Pro Leu
                85                  90                  95

acg ttc ggt gct ggg acc aag ctg gag ctg aaa                        321
Thr Phe Gly Ala Gly Thr Lys Leu Glu Leu Lys
            100                 105
```

FIG.19C

233 Variable Heavy Chain

gag gtg aag ctg gtg gag tct gga gga ggc ttg gta cag cct ggg ggt 48
Glu Val Lys Leu Val Glu Ser Gly Gly Gly Leu Val Gln Pro Gly Gly
1 5 10 15

CDR1
tct ctg agt ctc tcc tgt gca gct tct **gga ttc acc ttc act gat tac** 96
Ser Leu Ser Leu Ser Cys Ala Ala Ser **Gly Phe Thr Phe Thr Asp Tyr**
20 25 30

**tcc atg aac** tgg gtc cgc cag cct cca ggg aag gca ctt gag tgg ttg 144
**Ser Met Asn** Trp Val Arg Gln Pro Pro Gly Lys Ala Leu Glu Trp Leu
35 40 45

CDR2
ggt **ttt att aga aac aaa gct aat gat tac aca aca gag tac agt gca** 192
Gly **Phe Ile Arg Asn Lys Ala Asn Asp Tyr Thr Thr Glu Tyr Ser Ala**
50 55 60

**tct gtg aag ggt** cgg ttc acc atc tcc aga gat aat tcc caa agc atc 240
**Ser Val Lys Gly** Arg Phe Thr Ile Ser Arg Asp Asn Ser Gln Ser Ile
65 70 75 80

ctc tat ctt caa atg aat gcc ctg aga gct gag gac agt gcc act tat 288
Leu Tyr Leu Gln Met Asn Ala Leu Arg Ala Glu Asp Ser Ala Thr Tyr
85 90 95

CDR3
tac tgt gta aga **tac cct agg tat cat gct atg gac tcc** tgg ggt caa 336
Tyr Cys Val Arg **Tyr Pro Arg Tyr His Ala Met Asp Ser** Trp Gly Gln
100 105 110

gga acc tca gtc acc gtc tcc tca 360
Gly Thr Ser Val Thr Val Ser Ser
115 120

FIG.19D

EA2 Variable Light Chain

```
gac atc aag atg acc cag tct cca tct tcc atg tat gca tct cta gga      48
Asp Ile Lys Met Thr Gln Ser Pro Ser Ser Met Tyr Ala Ser Leu Gly
1               5                   10                  15
                                            CDR1
gag aga gtc act atc act tgc aag gcg agt cag gac att aat aac tat      96
Glu Arg Val Thr Ile Thr Cys Lys Ala Ser Gln Asp Ile Asn Asn Tyr
            20                  25                  30

tta agc tgg ttc cag cag aaa cca ggg aaa tct cct aag acc ctg atc     144
Leu Ser Trp Phe Gln Gln Lys Pro Gly Lys Ser Pro Lys Thr Leu Ile
        35                  40                  45
            CDR2
tat cgt gca aac aga ttg gta gat ggg gtc cca tca agg ttc agt ggc     192
Tyr Arg Ala Asn Arg Leu Val Asp Gly Val Pro Ser Arg Phe Ser Gly
    50                  55                  60

agt gga tct ggg caa gat tat tct ctc acc atc agc agc ctg gag tat     240
Ser Gly Ser Gly Gln Asp Tyr Ser Leu Thr Ile Ser Ser Leu Glu Tyr
65                  70                  75                  80
                                                CDR3
gaa gat atg gga att tat tat tgt ctg aaa tat gat gag ttt ccg tac     288
Glu Asp Met Gly Ile Tyr Tyr Cys Leu Lys Tyr Asp Glu Phe Pro Tyr
                85                  90                  95

acg ttc gga ggg ggg acc aag ctg gaa ata aaa                         321
Thr Phe Gly Gly Gly Thr Lys Leu Glu Ile Lys
            100                 105
```

FIG.19E

EA2 Variable Heavy Chain

```
gac gtg aag ctg gtg gag tct ggg gga ggc tta gtg aag cct gga ggg      48
Asp Val Lys Leu Val Glu Ser Gly Gly Gly Leu Val Lys Pro Gly Gly
1               5                   10                  15
                                         _________CDR1__________
tcc ctg aaa ctc tcc tgt gca gcc tct gga ttc act ttc agt agc tat      96
Ser Leu Lys Leu Ser Cys Ala Ala Ser Gly Phe Thr Phe Ser Ser Tyr
            20                  25                  30
___________
acc atg tct tgg gtt cgc cag act ccg gag aag agg ctg gag tgg gtc     144
Thr Met Ser Trp Val Arg Gln Thr Pro Glu Lys Arg Leu Glu Trp Val
        35                  40                  45
    ________________________CDR2____________________________
gca acc att agt agt ggt ggt act tac acc tac tat cca gac agt gtg     192
Ala Thr Ile Ser Ser Gly Gly Thr Tyr Thr Tyr Tyr Pro Asp Ser Val
    50                  55                  60
_______
aag ggc cga ttc acc atc tcc aga gac aat gcc aag aac acc ctg tac     240
Lys Gly Arg Phe Thr Ile Ser Arg Asp Asn Ala Lys Asn Thr Leu Tyr
65                  70                  75                  80

ctg caa atg agc agt ctg aag tct gag gac aca gcc atg tat tac tgt     288
Leu Gln Met Ser Ser Leu Lys Ser Glu Asp Thr Ala Met Tyr Tyr Cys
                85                  90                  95
        ________CDR3_____________
aca aga gaa gct atc ttt act tac tgg ggc caa ggg act ctg gtc act     336
Thr Arg Glu Ala Ile Phe Thr Tyr Trp Gly Gln Gly Thr Leu Val Thr
            100                 105                 110

gtc tct gca                                                          345
Val Ser Ala
        115
```

FIG.19F

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 239400 A
- EP 592106 A
- EP 519596 A
- WO 9109967 A
- WO 9317105 A
- US 5225539 A
- US 5530101 A
- US 5565332 A
- US 5585089 A
- US 5766886 A
- US 6407213 B
- WO 9317715 A
- WO 9208802 A
- WO 9100360 A
- WO 9205793 A
- US 4474893 A
- US 4714681 A
- US 4925648 A
- US 5573920 A
- US 5601819 A
- WO 9404678 A
- WO 9425591 A
- US 6005079 A
- WO 9734631 A
- WO 02060919 A
- WO 9321232 A
- EP 439095 A
- US 5474981 A
- US 5336603 A
- US 5622929 A
- US 5359046 A
- US 5349053 A
- US 5447851 A
- US 5112946 A
- EP 307434 A
- EP 367166 A
- WO 9604388 A
- WO 9106570 A
- US 5605793 A
- US 5811238 A
- US 5830721 A
- US 5834252 A
- US 5837458 A
- WO 9733899 A
- WO 9734911 A
- WO 9923105 A
- US 4676980 A, Segal
- GB 9101134 W
- WO 9002809 A
- WO 9110737 A
- WO 9201047 A
- WO 9218619 A
- WO 9311236 A
- WO 9515982 A
- WO 9520401 A
- WO 9713844 A
- US 5698426 A
- US 5223409 A
- US 5443484 A
- US 5580717 A
- US 5427908 A
- US 5750753 A
- US 5521047 A
- US 5571698 A
- US 5516637 A
- US 5780225 A
- US 5655727 A
- US 5733743 A
- US 5969108 A
- WO 9222324 A
- US 4444887 A
- US 4716111 A
- WO 9846645 A
- WO 9850433 A
- WO 9824893 A
- WO 9816654 A
- WO 9634096 A
- WO 9633735 A
- WO 91110741 A
- US 5413923 A
- US 5625126 A
- US 5633425 A
- US 5569825 A
- US 5661016 A
- US 5545806 A
- US 5814318 A
- US 5939598 A
- US 6311415 B
- US 5807715 A
- US 4816567 A
- US 4816397 A
- WO 8605807 A
- WO 8901036 A
- US 5122464 A
- US 6373577 B
- US 6289286 B
- US 5322798 B
- US 5341215 B

- US 6268125 B
- US 5679377 A
- US 5916597 A
- US 5912015 A
- US 5989463 A
- US 5128326 A
- WO 9915154 A
- WO 9920253 A
- US 4526938 A
- WO 9105548 A
- WO 9620698 A
- US 60379322 B
- US 60418213 B

**Non-patent literature cited in the description**


- **Robbins ; Angell.** Basic Pathology. W.B. Saunders Co, 1976, 68-122
- **Rhim et al.** *Critical Reviews in Oncogenesis,* 1997, vol. 8, 305
- **Patarca.** *Critical Reviews in Oncogenesis,* 1996, vol. 7, 343
- **Malik et al.** *Biochimica et Biophysica Acta,* 1996, vol. 1287, 73
- **Cance et al.** *Breast Cancer Res Treat,* 1995, vol. 35, 105
- **Hunter.** *Cell,* 1997, vol. 88, 333
- **Levitzki et al.** *Science,* 1995, vol. 267, 1782
- **Kondapaka et al.** *Molecular & Cellular Endocrinology,* 1996, vol. 117, 53
- **Fry et al.** *Current Opinion in BioTechnology,* 1995, vol. 6, 662
- **Zantek et al.** *Cell Growth & Differentiation,* 1999, vol. 10, 629
- **Lindberg et al.** *Molecular & Cellular Biology,* 1990, vol. 10, 6316
- *Cell,* 1997, vol. 90, 403
- **Gale et al.** *Cell & Tissue Research,* 1997, vol. 290, 227
- **Lawrence ; Steeg.** *World J. Urol.,* 1996, vol. 14, 124-130
- Principles of Cancer Patient Management. **Stockdale.** Scientific American: Medicine. 1998, vol. 3
- **Gilman et al.** Goodman and Gilman's: The Pharmacological Basis of Therapeutics. Pergamom Press, 1990
- Principles Of Cancer Patient Management. **Stockdale.** Scientific American Medicine. 1998, vol. 3
- **Padlan.** *Molecular Immunology,* 1991, vol. 28 (4/5), 489-498
- **Studnicka et al.** *Protein Engineering,* 1994, vol. 7 (6), 805-814
- **Roguska et al.** *PNAS,* 1994, vol. 91, 969-973
- **Tan et al.** *J. Immunol.,* 2002, vol. 169, 1119-25
- **Caldas et al.** *Protein Eng.,* 2000, vol. 13, 353-60
- **Morea et al.** *Methods,* 2000, vol. 20, 267-79
- **Baca et al.** *J. Biol. Chem.,* 1997, vol. 272, 10678-84
- **Roguska et al.** *Protein Eng.,* 1996, vol. 9, 895-904
- **Couto et al.** *Cancer Res.,* 1995, vol. 55 (23), 5973s-5977s
- **Couto et al.** *Cancer Res.,* 1995, vol. 55, 1717-22
- **Sandhu.** *Gene,* 1994, vol. 150, 409-10
- **Pedersen et al.** *J. Mol. Biol.,* 1994, vol. 235, 959-73
- **Jones et al.** *Nature,* 1986, vol. 321, 522-525
- **Reichmann et al.** *Nature,* 1988, vol. 332, 323-329
- **Presta.** *Curr. Op. Struct. Biol.,* 1992, vol. 2, 593-596
- **Kabat et al.** Sequences of Proteins of Immunological Interest. National Institutes of Health, 1991
- **Chothia ; Lesk.** *J. Mol. Biol.,* 1987, vol. 196, 901-917
- Physicians' Desk Reference. 2002
- **Pluckthun.** The Pharmacology of Monoclonal Antibodies. Springer-Verlag, 1994, vol. 113, 269-315
- **Tutt et al.** *J. Immunol.,* 1991, vol. 147, 60-69
- **Kostelny et al.** *J. Immunol.,* 1992, vol. 148, 1547-1553
- **Muyldermans et al.** *Trends Biochem. Sci.,* 2001, vol. 26, 230
- **Nuttall et al.** *Cur. Pharm. Biotech.,* 2000, vol. 1, 253
- **Reichmann ; Muyldermans.** *J. Immunol. Meth.,* 1999, vol. 231, 25
- Current Protocols in Molecular Biology. Publishing Associates, Inc. and John Wiley and Sons, Inc, 1989, vol. 1, 6.3.1-6.3.62.10.3
- **Naramura et al.** *Immunol. Lett.,* vol. 39, 91-99
- **Gillies et al.** *PNAS,* 1992, vol. 89, 1.428-1432
- **Fell et al.** *J. Immunol.,* 1991, vol. 146, 2446-2452
- **Ashkenazi et al.** *PUNAS,* 1991, vol. 88, 10535-10539
- **Zheng et al.** *J. Immunol.,* 1995, vol. 154, 5590-5600
- **Vi1 et al.** *PNAS,* 1992, vol. 89, 11337-11341

- **Patten et al.** *Curr. Opinion Biotechnol.,* 1997, vol. 8, 724-33
- **Harayama.** *Trends Biotechnol.,* 1998, vol. 16, 76
- **Hansson et al.** *J. Mol. Biol.,* 1999, vol. 287, 265
- **Lorenzo ; Blasco.** *BioTechniques,* 1998, vol. 24, 308
- **Gentz et al.** *PNAS,* 1989, vol. 86, 821
- **Wilson et al.** *Cell,* 1984, vol. 37, 767
- **Takahashi et al.** *J. Iminunol.,* 1994, vol. 6, 1567
- **Denardo et al.** *Clin Cancer Res.,* 1998, vol. 4, 2483-90
- **Peterson et al.** *Bioconjug. Chem.,* 1999, vol. 10, 553
- **Zimmerman et al.** *Nucl. Med. Biol.,* 1999, vol. 26, 943-50
- **Garnett.** *Adv. Drug Deliv. Rev.,* 2002, vol. 53, 171-216
- Monoclonal Antibodies For Immunotargeting Of Drugs In Cancer Therapy. **Amon et al.** Monoclonal Antibodies And Cancer Therapy. Alan R. Liss, Inc, 1985, 243-56
- Antibodies For Drug Delivery. **Hellstrom et al.** Controlled Drug Delivery. Marcel Dekker, Inc, 1987, 623-53
- Antibody Carriers Of Cytotoxic Agents In Cancer Therapy: A Review. **Thorpe et al.** Monoclonal Antibodies '84: Biological And Clinical Applications. 1985, 475-506
- Analysis, Results, And Future Prospective Of The Therapeutic Use Of Radiolabeled Antibody In Cancer Therapy. Monoclonal Antibodies For Cancer Detection And Therapy. Academic Press, 1985, 303-16
- **Thorpe et al.** *Immunol. Rev.,* 1982, vol. 62, 119-58
- **Ashkenazi et al.** *PNAS,* 1991, vol. 88, 10535-10539
- **Vil et al.** *PNAS,* 1992, vol. 89, 11337-11341
- **Peterson et al.** *Bioconjug. Chem.,* vol. 10, 553
- **Harlow et al.** Antibodies: A Laboratory Manual. Cold Spring Harbor Laboratory Press, 1988
- **Hammerling et al.** Monoclonal Antibodies and T-Cell Hybridomas. Elsevier, 1981, 563-681
- **Brinkman et al.** *J. Immunol. Methods,* 1995, vol. 182, 41-50
- **Ames et al.** *J. Immunol. Methods,* 1995, vol. 184, 177
- **Kettleborough et al.** *Eur. J. Immunol.,* 1994, vol. 24, 952-958
- **Persic et al.** *Gene,* 1997, vol. 187, 9
- **Burton et al.** *Advances in Immunology,* 1994, vol. 57, 191-280
- **Mullinax et al.** *BioTechniques,* 1992, vol. 12, 864
- **Sawai et al.** *AJRI,* 1995, vol. 34, 26
- **Better et al.** *Science,* 1988, vol. 240, 1041
- **Lonberg ; Huszar.** *Int. Rev. Immunol.,* 1995, vol. 13, 65-93
- **Morrison.** *Science,* 1985, vol. 229, 1202
- **Oi et al.** *BioTechniques,* 1986, vol. 4, 214
- **Gillies et al.** *J. Immunol. Methods,* 1989, vol. 125, 191-202
- **Studnicka et al.** *Protein Engineering,* 1994, vol. 7, 805
- **Roguska et al.** *PNAS,* 1994, vol. 91, 969
- **Riechmann et al.** *Nature,* 1988, vol. 332, 323
- **Greenspan ; Bona.** *FASEB J.,* 1989, vol. 7, 437-444
- **Nissinoff.** *J. Immunol.,* 1991, vol. 147, 2429-2438
- **Kutmeier et al.** *BioTechniques,* 1994, vol. 17, 242
- **Sambrook et al.** Molecular Cloning, A Laboratory Manual. Cold Spring Harbor Laboratory, 1990
- Current Protocols in Molecular Biology. John Wiley & Sons, 1998
- **Chothia et al.** *J. Mol. Biol.,* 1998, vol. 278, 457-479
- **Foecking et al.** *Gene,* 1986, vol. 45, 101
- **Cockett et al.** *BioTechnology,* 1990, vol. 8, 2
- **Ruther et al.** *EMBO,* 1983, vol. 12, 1791
- **Inouye ; Inouye.** *Nucleic Acids Res.,* 1985, vol. 13, 3101-3109
- **Van Heeke ; Schuster.** *J. Biol. Chem.,* 1989, vol. 24, 5503-5509
- **Logan ; Shenk.** *PNAS,* 1984, vol. 8 (1), 355-359
- **Bittner et al.** *Methods in Enzymol.,* 1987, vol. 153, 516-544
- **Wigler et al.** *Cell,* 1977, vol. 11, 223
- **Szybalska ; Szybalski.** *Proc. Natl. Acad. Sci. USA,* 1992, vol. 48, 202
- **Lowy et al.** *Cell,* 1980, vol. 22, 8-17
- **Wigler et al.** *PNAS,* 1980, vol. 77, 357
- **O'Hare et al.** *PNAS,* 1981, vol. 78, 1527
- **Mulligan ; Berg.** *PNAS,* 1981, vol. 78, 2072
- **Wu ; Wu.** *Biotherapy,* 1991, vol. 3, 87
- **Tolstoshev.** *Ann. Rev. Pharmacol. Toxicol.,* 1993, vol. 32, 573
- **Mulligan.** *Science,* 1993, vol. 260, 926
- **Morgan ; Anderson.** *Ann. Rev. Biochem.,* 1993, vol. 62, 191
- **May.** *TIB TECH,* 1993, vol. 11, 155
- **Santerre et al.** *Gene,* 1984, vol. 30, 147
- Current Protocols in Molecular Biology. John Wiley & Sons, 1993
- **Kriegler.** Gene Transfer and Expression, A Laboratory Manual. Stockton Press, 1990
- Current Protocols in Human Genetics. John Wiley & Sons, 1994

- **Colberre-Garapin et al.** *J. Mol. Biol.,* 1981, vol. 150, 1
- DNA cloning. Academic Press, 1987, vol. 3
- **Crouse et al.** *Mol. Cell. Biol.,* 1983, vol. 3, 257
- **Proudfoot.** *Nature,* 1986, vol. 322, 52
- **Kohler.** *PNAS,* 1980, vol. 77, 2197
- Physician's Desk Reference. 2002
- **Fishman et al.** Medicine. J.B. Lippincott Co, 1985
- **Murphy et al.** Informed Decisions: The Complete Book of Cancer Diagnosis, Treatment, and Recovery. Penguin Books U.S.A., Inc, 1997
- **Hardie ; Hanks.** The Protein Kinase Facts Book, I and II. Academic Press, 1995, vol. I, II
- **Zelinski et al.** *Cancer Res.,* 2001, vol. 61, 2301-6
- **Mullet et al.** *Methods,* 2000, 77-91
- **Dong et al.** *Review in Mol. Biotech.,* 2002, vol. 82, 303-23
- **Fivash et al.** *Current Opinion in Biotechnology,* 1998, vol. 9, 97-101
- **Rich et al.** *Current Opinion in Biotechnology,* 2000, vol. 11, 54-61
- **Myszka.** *Current Opinion in Biotechnology,* 1997, vol. 8, 50-7
- **Fisher et al.** *Current Opinion in Biotechnology,* 1994, vol. 5, 389-95
- **O'Shannessy.** *Current Opinion in Biotechnology,* 1994, vol. 5, 65-71
- **Chaiken et al.** *Analytical Biochemistry,* 1992, vol. 201, 197-210
- **Morton et al.** *Analytical Biochemistry,* 1995, vol. 227, 176-85
- **O'Shannessy et al.** *Analytical Biochemistry,* 1996, vol. 236, 275-83
- Relevance of Tumor Models for Anticancer Drug Development. 1999
- **Karger.** *Contributions to Oncology,* 1999
- The Nude Mouse in Ontology Research. 1991
- Anticancer Drug Development Guide. 1997
- **Wu ; Wu.** *J. Biol. Chem.,* 1987, vol. 262, 4429-4432
- **Sefton.** *CRC Crit. Ref. Biomed. Eng.,* 1987, vol. 14, 20
- **Buchwald et al.** *Surgery,* 1980, vol. 88, 507
- **Saudek et al.** *N. Engl. J. Med.,* 1989, vol. 321, 574
- Medical Applications of Controlled Release. CRC Pres, 1974
- Controlled Drug Bioavailability, Drug Product Design and Performance. Wiley, 1984
- **Ranger ; Peppas.** *J. Macromol. Sci. Rev. Macromol. Chem.,* 1983, vol. 23, 61
- **Levy et al.** *Science,* 1985, vol. 228, 190
- **During et al.** *Ann. Neural.,* 1989, vol. 25, 351
- **Howard et al.** *J. Neurosurg.,* 1989, vol. 7 1, 105
- **Goodson.** *Medical Applications of Controlled Release,* 1984, vol. 2, 115-138
- **Langer.** *Science,* 1990, vol. 249, 1527-1533
- **Ning et al.** *Radiotherapy & Oncology,* 1996, vol. 39, 179-189
- **Song et al.** *PEA Journal of Pharmaceutical Science & Technology,* 1995, vol. 50, 372-397
- **Cleek et al.** *Pyro. Int'l. Symp. Control. Rel. Bioact. Mater.,* 1997, vol. 24, 853-854
- **Lam et al.** *Proc. Int'l. Symp. Control Rel. Bioact. Mater.,* 1997, vol. 24, 759-760
- **Zantek et al.** *Cell Growth Diff.,* 1999, vol. 10, 629-38
- **Nakshatri et al.** *Mol. Cell. Biol.,* 1997, vol. 17, 3629-39
- Neoplasms of the prostate. **Bostwick.** Urologic Surgical Pathology. 1997, 343-422
- **Gleson ; Mellinger.** *J. Urol.,* 1974, vol. 111, 58-64
- **Fleming et al.** AJCC Cancer Staging Manual. Raven and Lippincott, 1997
- **Jiang et al.** *Am. J. Pathol.,* 2002, vol. 160, 667-71
- **Cheng et al.** *Am J. Pathol.,* 1996, vol. 148, 1375-80